(19) Europäisches Patentamt
European Patent Office
Office européen des brevets

(11) Publication number: 0 228 192
A2

(12) # EUROPEAN PATENT APPLICATION

(21) Application number: 86309362.1

(22) Date of filing: 01.12.86

(51) Int. Cl.[4]: C07K 5/02 , C07K 5/06 , C07C 103/76 , A61K 37/02 , A61K 37/64 , C07D 233/64 , A61K 31/195 , A61K 31/165

Claims for the following Contracting States: AT-ES

(30) Priority: 29.11.85 JP 268415/85
26.12.85 JP 297664/85
04.09.86 JP 208621/86

(43) Date of publication of application: 08.07.87 Bulletin 87/28

(84) Designated Contracting States: AT BE CH DE ES FR GB IT LI LU NL SE

(71) Applicant: SANKYO COMPANY LIMITED
No. 1-6, 3-chome Nihonbashi Honcho
Chuo-ku
Tokyo(JP)

(72) Inventor: Morisawa, Yasuhiro
Sankyo Chemical Res. Lab. 2-58 1-chome
Hiromachi
Shinagawa-ku Tokyo(JP)
Inventor: Yabe, Yuichiro
Sankyo Chemical Res. Lab. 2-58 1-chome
Hiromachi
Shinagawa-ku Tokyo(JP)
Inventor: Kataoka, Mitsuru
Sankyo Chemical Res. Lab. 2-58, 1-chome,
Hiromachi
Shinagawa-ku Tokyo(JP)
Inventor: Iijama, Yasuteru
Sankyo Bio Res Lab 2-58, 1 chome,
Hiromachi
Shinagawa-ku Tokyo(JP)
Inventor: Koike, Hiroyuki
Sankyo Bio Res Lab 2-58, 1-chome,
Hiromachi
Shinagawa-ku Tokyo(JP)
Inventor: Takahagi. Hidekuni
Sankyo Anal Meta Res Lab 2-58 1-chome
Hiromachi
Shinagawa-ku Tokyo(JP)
Inventor: Kokubu, Tatsuo
2310-7, Tanokubo, Shigenobu-cho
Onsen-gun Ehime-prefecture(JP)
Inventor: Hiwada, Kunio
2108-6, Tanokubo, Shigenobu-cho
Onsen-gun Ehime-prefecture(JP)

(74) Representative: Gibson, Christian John Robert et al
MARKS & CLERK 57/60 Lincoln's Inn Fields
London WC2A 3LS(GB)

EP 0 228 192 A2

(54) **Renin-inhibitory oligopeptides, their preparation and use.**


(57) Oligopeptides of formula (I):

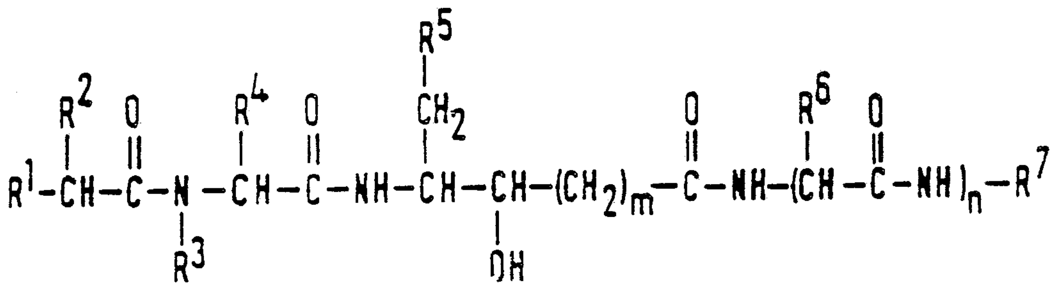

(I)

where $R^1$-$R^7$ are various organic groups and n and m are 0 or 1, have renin-inhibitory activity and are particularly suitable for oral administration. They may be prepared by condensing their component amino acids or lower oligopeptides using conventional peptide synthesis reactions.

# RENIN-INHIBITCRY OLIGOPEPTIDES, THEIR PREPARATION AND USE

The present invention relates to a series of new oligopeptides which have renin-inhibitory and, hence, hypotensive activities and thus are of particular value in the treatment of hypertension induced by failures in the renin-angiotensin system. The invention also relates to the preparation of such compounds and to their use in such treatment.

There is considerable evidence that reduction of elevated blood pressure reduces the risks of morbidity and mortality. Elevated blood pressure (hypertension) can be caused by a variety of factors and a large number of drugs is available for the treatment of hypertension, the drug of choice being dictated in large measure by the cause of the hypertension. Angiotensin I is a polypeptide formed by the action of renin upon a plasma protein and is converted to angiotensin II by the action of ACE (angiotensin converting enzyme). Angiotensin II causes constriction of the arterioles and can produce hypertension. Hypertension of this type can be reduced by reducing the plasma concentration of angiotensin which, in turn, can be achieved by inhibiting the activity of renin. The number of available drugs having this type of inhibitory activity is very limited, and, to date, no such drug is commercially available.

A variety of peptide derivatives having this type of activity is known. Those prior art compounds believed to be closest to the compounds of the present invention, in that they are based upon the rather uncommon amino acid statine and analogs thereof, are disclosed in European Patent Publication No. 156,321 and in copending European Patent Application Publication No. 0186977.

A serious disadvantage common to almost all of the known renin-inhibitory oligopeptides, including those mentioned in the previous paragraph, is that, in practice, it is necessary to administer them by parenteral routes, e.g. by injection, as suppositories or even by inhalation. This applies even in those cases where the compounds have been suggested for oral use, since it has subsequently been found that they either are insufficiently stable to enzymes, e.g. esterases, present in the digestive system or are inadequately absorbed from the stomach and/or intestines or both. Of course, the poor stability is expected with oligopeptides, as the mammalian digestive system is specifically designed to break down compounds of that type. Consequently, even if the compounds can be administered orally, such high doses are necessary in order to make up for poor absorption and/or losses caused by digestion as to make oral administration impractical.

It is, of course, well known that the oral route is the preferred route of administration, particularly where (as with the drugs with which the present invention is concerned) drugs are intended for self-administration by the patient, generally over a long period of time.

Hence, the inability of the known renin-inhibitory oligopeptides to be administered via the oral route is a serious disadvantage to their practical therapeutic use.

We have now discovered a series of peptide derivatives having a very marked ability to inhibit the activity of renin, which ability is believed to be significantly better than that of the prior art compounds. However, most significantly and surprisingly, the compounds of the invention have been found to have excellent absorptive properties (especially through the intestinal and digestive tracts) upon oral administration, quite contrary to what has been generally experienced with prior art oligopeptide compounds. Moreover, certain of the compounds of the invention have additionally and unexpectedly demonstrated very good stability on oral administration (i.e. they are stable to digestive enzymes, e.g. esterases). Moreover, and again surprisingly in view of the limited water solubility of most oligopeptides, the compounds of the present invention have good water solubility.

These unexpected properties render the compounds of the invention especially suited to oral administration as well, of course, as the more traditional parenteral routes of administration.

The compounds of the invention are peptides having the general formula (I):

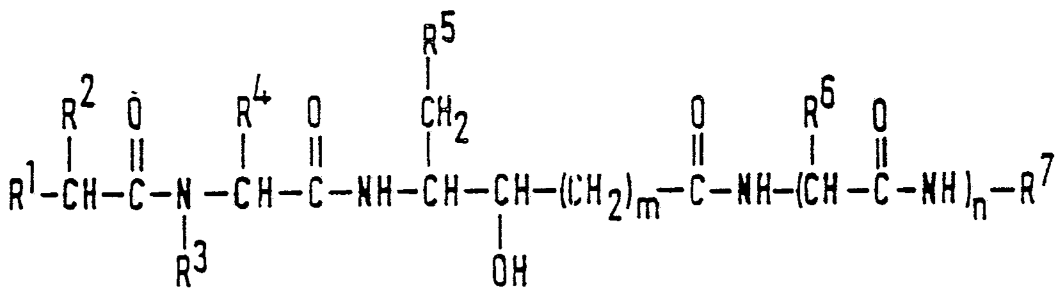

(I)

wherein:
m is the cypher 0 or the integer 1;
nis the cypher 0 or the integer 1;

$R^1$ represents a group of formula $-A-R^8$,
wherein: A represents a $C_1$-$C_8$ alkylene group; and $R^8$ represents an aryl group, a heterocyclic group, a $C_2$-$C_5$ aliphatic acyl group, a heterocyclic acyl group or an alkoxyalkoxy group in which each alkoxy part is $C_1$-$C_4$,

a group of formula $-CONR^9R^{10}$,
wherein $R^9$ and $R^{10}$ are the same or different and each represents a hydrogen atom, a $C_1$-$C_4$ alkyl group or a $C_1$-$C_4$ alkyl groups having at least one of substituents (a), defined below,

or a group of formula $-NHCOR^{11}$,
wherein $R^{11}$ represents a $C_1$-$C_{10}$ alkyl group, a $C_1$-$C_{10}$ alkyl group having at least one of substituents (b), defined below, an aryl group, a heterocyclic group, a $C_1$-$C_4$ alkoxy group, a $C_1$-$C_4$ alkoxy group having at least one of substituents (c), defined below, an aralkyloxy group or an aryloxy group,
provided that, when both m = 1 and n = 1, $R^1$ represents said group of formula $-A-R^8$ or said group of formula $-CONR^9R^{10}$;

$R^2$ represents a group of formula $-B-R^{12}$,
wherein B represents a $C_1$-$C_{10}$ alkylene group and
$R^{12}$ represents an aryl group or a heterocyclic group;
$R^3$ represents a hydrogen atom or a $C_1$-$C_4$ alkyl group;

$R^4$ represents a $C_1$-$C_{10}$ alkyl group, a $C_1$-$C_{10}$ alkyl group having at least one of substituents (d), defined below, a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

$R^5$ represents an isopropyl group, a $C_3$-$C_8$ cycloalkyl group or a phenyl group;

$R^6$ represents a $C_1$-$C_6$ alkyl group;

$R^7$ represents a $C_1$-$C_{10}$ alkyl group having at least one of substituents (e), defined below, or a $C_1$-$C_{10}$ alkyl group having at least one of substituents (e) and at least one of substituents (f), defined below;

said aryl groups and the aryl parts of said aryloxy, aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one of substituents (d) and/or (g) and/or (h), defined below;
the alkyl parts of said aralkyloxy, aralkylthio, aralkyloxycarbonylamino and aralkyloxycarbonyl groups have from 1 to 4 carbon atoms;
said heterocyclic groups have from 5 to 14 ring atoms, of which from 1 to 5 are nitrogen, oxygen or sulphur hetero-atoms, and are unsubstituted or have at least one of substituents (d) and/or (g) and/or (h) and/or (i), defined below;

substituents (a): carboxy groups, $C_2$-$C_5$ alkoxycarbonyl groups, carbamoyl groups, alkylcarbamoyl groups where the alkyl part is $C_1$-$C_4$ and dialkylcarbamoyl groups where each alkyl part is $C_1$-$C_4$;

substituents (b):$C_1$-$C_4$ alkoxy groups, alkoxyalkoxy groups in which each alkoxy part is $C_1$-$C_4$ alkoxy, halogen atoms, $C_2$-$C_5$ alkoxycarbonyl groups, aralkyloxycarbonyl groups, aryl groups, heterocyclic groups, $C_1$-$C_7$ aliphatic carboxylic acyl groups, aromatic carboxylic acyl groups, heterocyclic carboxylic acyl groups and aryloxy groups;

substituents (c):$C_1$-$C_4$ alkoxy groups and alkoxyalkoxy groups where each alkoxy part is $C_1$-$C_4$;

substituents (d):hydroxy groups, $C_1$-$C_4$ alkoxy groups, aryloxy groups, aralkyloxy groups where the alkyl part is $C_1$-$C_4$, $C_1$-$C_7$ aliphatic carboxylic acyloxy groups, $C_1$-$C_4$ alkylthio groups, arylthio groups, aralkylthio groups where the alkyl part is $C_1$-$C_4$, $C_1$-$C_4$ alkylsulphinyl groups, $C_1$-$C_4$ alkylsulphonyl groups, arylsulphinyl groups, arylsulphonyl groups, $C_1$-$C_7$ aliphatic carboxylic acylamino groups, aromatic carboxylic acylamino groups, $C_2$-$C_7$ alkoxycarbonylamino groups, aralkyloxycarbonylamino groups where the alkyl part is $C_1$-$C_4$, $C_2$-$C_7$ alkoxycarbonyl groups, aryloxycarbonyl groups, aralkyloxycarbonyl groups where the alkyl part is $C_1$-$C_4$, $C_1$-$C_7$ aliphatic carboxylic acyl groups, aromatic carboxylic acyl groups, heterocyclic carboxylic acyl groups, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di- and tri-halomethyl groups, mercapto groups, amino groups, $C_1$-$C_4$ alkylamino groups, dialkylamino groups where each alkyl part is $C_1$-$C_4$, carboxy groups, guanidino groups and guanidino groups having at least one $C_1$-$C_4$ alkyl substituent;

substituents (e): amino groups, protected amino groups, aminoalkylamino groups where the alkyl part is $C_1$-$C_4$ and one or both amino parts may be unsubstituted or have a $C_1$-$C_4$ alkyl substituent, aminoalkylaminoalkylamino groups where each alkyl part is $C_1$-$C_4$ and any amino part may be unsubstituted or have a $C_1$-$C_4$ alkyl substituent, guanidino groups, guanidino group having at least one $C_1$-$C_4$ alkyl substituent, phenyl groups substituted by at least one $C_1$-$C_4$ aminoalkyl substituent, phenyl groups substituted by at least one substituted $C_1$-$C_4$ hydroxyalkoxy group said hydroxyalkoxy group having at least one of substituents (j), defined below, and sulpho groups:

substituents (f):hydroxy groups, carboxy groups, $C_2$-$C_5$ alkoxycarbonyl groups, carbamoyl groups, alkylcarbamoyl groups where the alkyl part is $C_1$-$C_4$ and dialkylcarbamoyl groups where each alkyl part is $C_1$-$C_4$;

substituents (g):hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

substituents (h): $C_1$-$C_4$ alkyl groups, nitro groups, cyano groups, $C_3$-$C_5$ alkenoyl groups, $C_3$-$C_5$ alkenoyl groups having at least one of substituents (d) and $C_1$-$C_4$ alkyl groups having at least one of substituents (g);

substituents (i):oxygen atoms; and

substituents (j):amino groups, $C_1$-$C_4$ alkylamino groups, dialkylamino groups where each alkyl part is $C_1$-$C_4$, heterocyclic groups and heterocyclic-substituted $C_1$-$C_4$ alkylamino groups;

and pharmaceutically acceptable salts, e.g. acid addition salts, thereof.

The invention also provides the use for the manufacture of a medicament for the treatment or prophylaxis of angiotensin-induced hypertension in a mammal, which may be human or non-human, of a compound of formula (I) or a pharmaceutically acceptable salt thereof.

The invention also provides a composition for the treatment of angiotensin-induced hypertension in a mammal, which may be human or non-human, which comprises a compound of formula (I) or a pharmaceutically acceptable salt thereof in admixture with a pharmaceutically acceptable carrier or diluent.

The compounds of the invention may be prepared by reacting together two compounds, one having a terminal carboxy group or reactive derivative thereof and the other having a terminal amino group or reactive derivative thereof, under conditions conventional for peptide synthesis, said two compounds corresponding to the fragments derivable by cleavage of any one of the peptide bonds A, B, C and D:

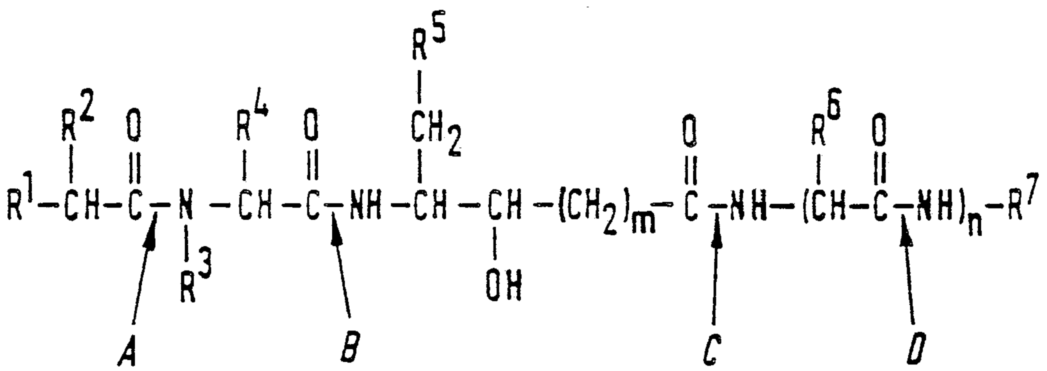 (I)

(in which $R^1$-$R^7$, m and n are as defined above).

Where $R^1$ represents a group of formula -A-$R^8$, A may represent a $C_1$-$C_8$, preferably a $C_1$-$C_4$, alkylene group, which may be a straight or branched chain group. This group is a bivalent hydrocarbon group and the two "free" valencies may be on different carbon atoms or on the same carbon atom. An alkylene group in which the two "free" valencies are on the same carbon atom is sometimes referred to as an "alkylidene" group. Examples of such groups include the methylene, ethylene, ethylidene, trimethylene, propylene, tetramethylene, 2-ethylethylene, 3-methyltrimethylene, 2-methyltrimethylene, pentamethylene, hexamethylene, heptamethylene, octamethylene, propylidene and butylidene groups.

When $R^2$ represents a group of formula -B-$R^{12}$, B likewise represents an alkylene group, which may be a straight or branched chain alkylene group and which may have its two "free" valencies on the same carbon atom or on different carbon atoms. B has from 1 to 10, preferably from 1 to 4, carbon atoms. Examples of alkylene groups which may be represented by B include the $C_1$-$C_8$ alkylene groups exemplified above in relation to A, as well as the nonamethylene and decamethylene groups.

Where $R^8$, $R^{11}$, $R^{12}$ or substituent (b), (d) or (g) represents an aryl group or where $R^{11}$ or substituent (d) represents an aryloxy group or where substituent (d) represents an arylthio group, the aryl group is a carbocyclic aryl group having from 6 to 14, preferably from 6 to 10, ring carbon atoms and which may be unsubstituted or have at least one of the substituents (d), (g) and (h) defined above. Examples of the unsubstituted aryl groups are the phenyl, indenyl (which may be 1-, 2-, 3-, 4-, 5-, 6-or 7-indenyl) and naphthyl (1-or 2-naphthyl) groups. Where such aryl groups are substituted, they may be any of the groups,

e.g. phenyl, indenyl and naphthyl groups, exemplified as unsubstituted aryl groups, and have at least one of the substituents (d), (g) and (h) as exemplified herein. Preferred substituents for aryl groups are: $C_1$-$C_4$ alkyl groups, e.g. the methyl, ethyl, propyl, isopropyl, butyl, isobutyl, sec-butyl and t-butyl groups; halogen atoms, e.g. the fluorine, chlorine, bromine and iodine atoms; $C_1$-$C_4$ alkoxy groups, e.g. the methoxy, ethoxy, propoxy, isopropoxy, butoxy, isobutoxy, sec-butoxy and t-butoxy groups; $C_2$-$C_5$ alkoxycarbonyl groups, i.e. groups containing a $C_1$-$C_4$ alkoxy group, such as those alkoxy groups exemplified above; the trifluoromethyl group; the amino group; the cyano group; or the nitro group. In general, there is no particular limitation on the maximum number of substituents possible except that dictated by the number of substitutable positions (e.g. 5 for phenyl, 7 for indenyl, 7 for naphthyl); however, in particular circumstances (as described more fully below in relation to substituents generally) steric constraints may dictate that less than this maximum number of substituents may be present. In practice, most substituted aryl groups employed in compounds of this type will normally have from 1 to 5, more commonly 1 to 3, substituents.

$R^8$, $R^{11}$, $R^{12}$, substituent (b), substituent (d), substituent (g) and substituent (j) may represent heterocyclic groups. Such groups may be aromatic or non-aromatic in character.

Where any of these groups represents an aromatic heterocyclic group, this is a heterocyclic group (as defined above) having aromatic character, i.e. a group having from 5 to 14 ring atoms of which from 1 to 5, preferably from 1 to 4 more preferably from 1 to 3, are nitrogen, oxygen or sulphur hetero-atoms, and it may be monocyclic or polycyclic (e.g. bicyclic). Preferably, it has from 5 to 10, more preferably 5 or 6, ring atoms, of which from 1 to 3 are such hetero-atoms. Included amongst the preferred aromatic heterocyclic groups are bicyclic groups comprising a benzene ring fused to a heterocyclic ring (the heterocyclic ring preferably having 5, 6 or 7 ring atoms of which from 1 to 3 are said hetero-atoms). Examples of such aromatic heterocyclic groups include the furyl, thienyl, oxazolyl, isoxazolyl, thiazolyl, imidazolyl, pyridyl, pyrazinyl, benzofuryl, benzothienyl, indolyl, benzothiazolyl, benzimidazolyl, quinolyl and isoquinolyl groups. Such groups may be unsubstituted or may have at least one of the substituents (d), (g), (h) and (i) defined above. Preferred such substituents are those specified above in relation to aryl groups, particularly the alkyl, halogen and alkoxy substituents. The maximum number of such substituents is generally also as described in relation to the aryl groups.

Where $R^8$ represents a heterocyclic group, this has from 5 to 14 ring atoms, of which from 1 to 5, more preferably from 1 to 3 and most preferably 1 or 2, are hetero-atoms selected from the group consisting of nitrogen, oxygen and sulphur atoms. The most preferred heterocyclic groups contain 5 or 6 ring atoms and most preferably 1 or 2 of these atoms are nitrogen atoms and 0 or 1 of these atoms are oxygen atoms or sulphur atoms. The heterocyclic group represented by $R^8$ may be aromatic in character (in which case it may be any one of those groups exemplified above) or it may be non-aromatic, in which case the ring may be a fully saturated ring or it may be an unsaturated ring, provided that the unsaturation is not aromatic in character.

Specific examples of preferred non-aromatic heterocyclic groups which may be represented by $R^8$, $R^{11}$, $R^{12}$, substituent (b), substituent (d), substituent (g) and substituent (j) include the piperidyl (including the 1-piperidyl group known as "piperidino"), pyrrolidinyl (e.g. l-pyrrolidinyl), morpholinyl (including the morpholino group), thiomorpholinyl (including the thiomorpholino group), oxazolidinyl (including 1-oxazolidinyl), thiazolidinyl (including 1-thiazolidinyl), imidazolidinyl (including 1-imidazolidinyl) and piperazinyl (including 1-piperazinyl). Such a ring may be unsubstituted or may have at least one of substituents (d) and/or (g) and/or (h) and/or (i) defined above. In the case of the heterocyclic groups represented by, <u>inter alia</u>, $R^8$, the preferred substituents are: $C_1$-$C_4$ alkyl groups, such as those exemplified above in relation to substituents on aryl groups; $C_1$-$C_4$ hydroxyalkyl groups, such as the hydroxymethyl, 1-hydroxyethyl, 2-hydroxyethyl, 2-hydroxypropyl, 3-hydroxypropyl and 4-hydroxybutyl groups; $C_1$-$C_4$ alkoxy groups, such as those exemplified below in relation to $R^{11}$; phenyl groups, which may be unsubstituted or may themselves have one or more of substituents (d), (g) and (h), but most preferably the substituents exemplified above in relation to aryl groups; aromatic heterocyclic groups, such as those exemplified above in relation to, <u>inter alia</u>, $R^8$ and which, of course, may be unsubstituted or substituted as defined above; aralkyloxycarbonyl groups, in which the aralkyloxy part may be any one of those aralkyloxy groups exemplified below in relation to $R^{11}$; $C_2$-$C_5$ alkoxycarbonyl groups, in which the alkoxy part may be any one of those exemplified below in relation to $R^{11}$; arylalkenoyl groups, in which the aryl part may be any one of those exemplified above and the alkenoyl part is a $C_3$-$C_5$ alkenoyl group, preferably a propenoyl group, for example the cinnamoyl group, which may itself be substituted; aliphatic acyl groups, such as those exemplified below in relation to $R^8$ - (especially as substituents on nitrogen hetero-atoms): and (also especially as substituents on nitrogen hetero-atoms) heterocyclic-carbonyl groups, including those exemplified below in relation to $R^8$ and others, such as the nicotinoyl and isonicotinoyl groups. The maximum number of substituents is as described generally below and as described specifically above in relation to aryl groups.

Where $R^8$ represents a $C_2$-$C_5$ aliphatic acyl group, this may be a straight or branched chain group, whose carbon chain may be saturated or unsaturated. It is preferably a $C_2$-$C_5$ alkanoyl group or a $C_3$-$C_5$ alkenoyl or alkynoyl group. Examples of such groups include the acetyl, propionyl, butyryl, isobutyryl, valeryl, isovaleryl, pivaloyl, acryloyl, propioloyl, methacryloyl, crotonoyl and isocrotonoyl groups.

Where $R^8$ represents a heterocyclic acyl group, this is preferably a heterocyclic-carbonyl group where the heterocyclic part is as defined above. More preferably, the heterocyclic part has 5 or 6 ring atoms, of which one or two are nitrogen and optionally another one is oxygen or sulphur. Preferred such groups include the piperidylcarbonyl (e.g. piperidinocarbonyl), pyrrolidinylcarbonyl (e.g. 1-and 2-pyrrolidinylcarbonyl, especially the prolyl and N-substituted prolyl groups, e.g. N-t-butoxycarbonyl-prolyl and N-benzyloxycarbonyl-prolyl groups), morpholinylcarbonyl (e.g. morpholinocarbonyl), oxazolidinylcarbonyl (e.g. 1-oxazolidinylcarbonyl), thiazolidinylcarbonyl (e.g. 1-thiazolidinylcarbonyl), imidazolidinylcarbonyl (e.g. 1-imidazolidinylcarbonyl), piperazinylcarbonyl (e.g. 1-piperazinylcarbonyl) and perhydro-1,4-thiazinylcarbonyl - (more commonly known as thiomorpholinylcarbonyl, e.g. thiomorpholinocarbonyl) groups.

Where $R^8$ represents an alkoxyalkoxy group, each alkoxy part is $C_1$-$C_4$, preferably $C_1$ or $C_2$, and may be a straight or branched chain group. Examples include the methoxymethoxy, ethoxymethoxy, propoxymethoxy, isopropoxymethoxy, butoxymethoxy, isobutoxymethoxy, 2-methoxyethoxy, 1-methoxyethoxy, 2-ethoxyethoxy, 2-propoxyethoxy, 2-butoxyethoxy, 3-methoxypropoxy, 3-ethoxypropoxy, 4-methoxybutoxy, 2-methoxybutoxy and 4-ethoxybutoxy groups.

Where $R^1$ represents a group of formula $-CONR^9R^{10}$, $R^9$ and $R^{10}$ are the same or different and each represents a hydrogen atom or an optionally substituted $C_1$-$C_4$ alkyl group, i.e. $R^1$ is a carbamoyl, alkylcarbamoyl or dialkylcarbamoyl group. More preferably, at least one of $R^9$ and $R^{10}$ is a hydrogen atom. Examples of such groups include the carbamoyl, methylcarbamoyl, ethylcarbamoyl, propylcarbamoyl, isopropylcarbamoyl, butylcarbamoyl, isobutylcarbamoyl, sec-butylcarbamoyl, t-butylcarbamoyl, dimethylcarbamoyl, diethylcarbamoyl, dipropylcarbamoyl, dibutylcarbamoyl, methylethylcarbamoyl, methylpropylcarbamoyl, ethylbutylcarbamoyl and methyl-t-butylcarbamoyl groups. Such alkylcarbamoyl and dialkylcarbamoyl groups may be unsubstituted or may be substituted on their alkyl parts by one or more of substituents (a), e.g. the following groups:

carboxy groups;

$C_2$-$C_5$ alkoxycarbonyl groups, e.g. the methoxycarbonyl, ethoxycarbonyl, propoxycarbonyl, isopropoxycarbonyl, butoxycarbonyl, isobutoxycarbonyl, sec-butoxycarbonyl or t-butoxycarbonyl groups; and

carbamoyl, alkylcarbamoyl and dialkylcarbamoyl groups, e.g. the unsubstituted such groups exemplified in relation to $R^1$.

Where the alkylcarbamoyl or dialkylcarbamoyl group is substituted, the maximum number of substituents may be as explained hereafter, but we prefer that the or each alkyl group should have only one such substituent. Preferred examples of such substituted carbamoyl groups which may be represented by $R^1$ include the carboxymethylcarbamoyl, 2-carboxyethylcarbamoyl, 2-carboxypropylcarbamoyl, 3-carboxypropylcarbamoyl, methoxycarbonylmethylcarbamoyl, ethoxycarbonylmethylcarbamoyl, t-butoxycarbonylmethylcarbamoyl, 2-methoxycarbonylethylcarbamoyl, 2-ethoxycarbonylethylcarbamoyl, carbamoylmethylcarbamoyl, methylcarbamoylmethylcarbamoyl and 2-ethylcarbamoylethylcarbamoyl groups.

Where $R^1$ represents a group of formula $-NHCOR^{11}$ and $R^{11}$ represents a $C_1$-$C_{10}$ alkyl group, this may be a straight or branched chain group and examples include the methyl, ethyl, propyl, isopropyl, butyl, isobutyl, sec-butyl, t-butyl, pentyl, isopentyl, neopentyl, t-pentyl, 1-methylbutyl, 2-methylbutyl, hexyl, isohexyl, sec-hexyl, 1,3-dimethylbutyl, 3,3-dimethylbutyl, 2-methylpentyl, heptyl, octyl, 1,5-dimethylhexyl, 2-ethylhexyl, nonyl and decyl groups, of which the $C_1$-$C_4$ alkyl groups are preferred, particularly, the methyl, ethyl, propyl, isopropyl, butyl and isobutyl groups.

Such alkyl groups represented by $R^{11}$ may be unsubstituted or they may have one or more of substituents (b), e.g. the following substituents:

$C_1$-$C_4$ alkoxy groups, e.g. the methoxy, ethoxy, propoxy, isopropoxy, butoxy, isobutoxy and t-butoxy groups;

alkoxyalkoxy groups, e.g. as exemplified above in relation to $R^8$;

halogen atoms, e.g. the chlorine, bromine, iodine and fluorine atoms;

$C_2$-$C_5$ alkoxycarbonyl groups, e.g. those groups exemplified above as substituents on alkylcarbamoyl groups represented by $R^1$;

aralkyloxycarbonyl groups, in which the aryl part is as defined above, but is preferably phenyl (which may be substituted or unsubstituted) and the alkyl part is $C_1$-$C_4$ alkyl (preferably methyl, ethyl or propyl), for example, the benzyloxycarbonyl, p-methylbenzyloxycarbonyl, p-chlorobenzyloxycarbonyl and p-methoxybenzyloxycarbonyl groups;

aryl groups, e.g. as exemplified above in relation, inter alia, to $R^8$, preferably a phenyl group (which may be substituted or unsubstituted); where the substituent is an aryl group, the alkyl group represented by $R^{11}$ which it substitutes is preferably a $C_1$-$C_4$ alkyl group, and examples of the resulting aralkyl groups include the benzyl, phenethyl, 3-phenylpropyl and 2-phenylpropyl groups;

heterocyclic groups, e.g. as exemplified above in relation to, inter alia, $R^8$, especially the pyridyl (which may be 2-, 3-or 4-pyridyl, preferably 3-pyridyl), piperazinyl (e.g. 1-piperazinyl), pyrrolidinyl (preferably 2-pyrrolidinyl) and morpholino groups, any of which may be unsubstituted or substituted as defined above; in this case, the alkyl group represented by $R^{11}$ on which this heterocyclic group is a substituent is preferably a $C_1$-$C_4$ alkyl group;

aliphatic, aromatic and heterocyclic acyl groups, e.g. as exemplified elsewhere herein, inter alia in relation to $R^8$; and

aryloxy groups, e.g. where the aryl part is as exemplified above in relation to, inter alia, $R^8$, preferably a phenoxy group (which may be unsubstituted or substituted as defined above).

Where $R^{11}$ represents an aryl or heterocyclic group, these may be as exemplified above. In the case of the heterocyclic groups, where these contain a ring nitrogen atom, that atom may be protected, e.g. with the aliphatic acyl, heterocyclic acyl, aromatic acyl, araliphatic acyl, alkoxycarbonyl, aryloxycarbonyl and aralkyloxycarbonyl groups exemplified herein, preferably with a heterocyclic acyl group and more preferably with a nicotinoyl group.

Where $R^{11}$ represents a $C_1$-$C_4$ alkoxy group, these may be as exemplified above in relation to substituents on an alkyl group represented by $R^{11}$. Such an alkoxy group may be unsubstituted or it may be substituted. Where it is substituted, it may have at least one (and preferably only one) alkoxy or alkoxyalkoxy substituent, the alkoxy substituent and the alkoxy parts of the alkoxyalkoxy substituent also preferably being thus exemplified. Examples of such substituted alkoxy groups include the methoxymethoxy, ethoxymethoxy, propoxymethoxy, isopropoxymethoxy, butoxymethoxy, t-butoxymethoxy, 2-methoxyethoxy, 2-ethoxyethoxy, 2-t-butoxyethoxy, methoxymethoxymethoxy, ethoxymethoxymethoxy, 2-(methoxymethoxy)ethoxy, 2-(2-methoxyethoxy)ethoxy, 2-(2-ethoxyethoxy)ethoxy, (2-methoxyethoxy)-methoxy, 3-methoxymethoxypropoxy and 4-(3-methoxypropoxy)butoxy groups.

Where $R^{11}$ represents an aralkyloxy group, the aryl group is as defined above and may be any one of those aryl groups exemplified above in relation to, inter alia, $R^8$, but is preferably a phenyl or naphthyl (1-or 2-naphthyl) group, more preferably a phenyl group. The alkyl part of this group is a $C_1$-$C_4$, preferably $C_1$-$C_3$, group, which may be a straight or branched chain group, e.g. the methyl, ethyl, propyl or isopropyl group. The aralkyloxy group is most preferably a benzyloxy or phenethyloxy group, and these groups may be unsubstituted or may have one or more of the above-defined substituents (d), (g) and (h). Most preferably, the substituents are as exemplified above in relation to aryl groups.

Where $R^{11}$ represents an aryloxy group, it may be any one of those aryloxy groups exemplified above as substituents on the alkyl group represented by $R^{11}$.

Where $R^3$ represents a $C_1$-$C_4$ alkyl group, this may be a straight or branched chain group, e.g. the methyl, ethyl, propyl, isopropyl, butyl, isobutyl, sec-butyl or t-butyl group. $R^3$ preferably represents a hydrogen atom or a methyl group and more preferably a hydrogen atom.

Where $R^4$ represents a $C_1$-$C_{10}$ alkyl group, it may be any one of those groups defined above in relation to $R^{11}$ and may be unsubstituted or may have at least one of the substituents defined above as substituents (d).

Examples of such substituents include:

(i) the hydroxy group;

(ii) $C_1$-$C_4$ alkoxy groups, such as those defined above in relation to $R^{11}$;

(iii) aryloxy groups, where the aryl part may be any one of those aryl groups described above, and may be unsubstituted or substituted.

(iv) aralkyloxy groups, such as those defined above in relation to $R^{11}$;

(v) aliphatic carboxylic acyloxy groups having from 1 to 7 carbon atoms, for example the formyloxy, acetoxy, propionyloxy, butyryloxy, isobutyryloxy, valeryloxy, isovaleryloxy, pivaloyloxy, hexanoyloxy and heptanoyloxy groups;

(vi) $C_1$-$C_4$ alkylthio groups, in which the alkyl part is a $C_1$-$C_4$ alkyl group chosen from any one of the $C_1$-$C_4$ groups exemplified above in relation to $R^3$;

(vii) arylthio groups, in which the aryl part may be any one of those aryl groups described above, and may be unsubstituted or substituted.

(viii) aralkylthio groups, in which the aralkyl part may be any one of those exemplified above;

(ix) $C_1$-$C_4$ alkylsulphinyl and alkylsulphonyl groups, in which the alkyl parts may be any one of those $C_1$-$C_4$ alkyl groups exemplified above in relation to $R^3$;

(x) arylsulphinyl and arylsulphonyl groups, in which the aryl part may be any of those aryl groups defined above in relation to,inter alia, $R^8$;

(xi) $C_1$-$C_7$ aliphatic acylamino groups, such as the formamido, acetamido, propionamido, butyramido, isobutyramido, valeramido, isovaleramido, pivaloylamino, hexanoylamino and heptanoylamino groups;

(xii) aromatic carboxylic acylamino groups, in which the aromatic part may be any one of those aryl groups exemplified above but is preferably a phenyl group which may be unsubstituted or have one or more substituents, for example the benzoylamino or p-nitrobenzoylamino groups;

(xiii) alkoxycarbonylamino groups having a total of from 2 to 5 carbon atoms, in which the alkoxycarbonyl part may be any one of those alkoxycarbonyl groups exemplified above in relation to substituents on aryl groups;

(xiv) aralkyloxycarbonylamino groups, in which the aralkyloxy part may be any one of those aralkyloxy groups exemplified above in relation to $R^{11}$;

(xv) $C_2$-$C_7$ alkoxycarbonyl groups, which may be chosen from those $C_2$-$C_5$ groups exemplified above in relation to substituents on alkyl groups represented by $R^1$, as well as, for example, the pentyloxycarbonyl, isopentyloxycarbonyl, hexyloxycarbonyl and isohexyloxycarbonyl groups;

(xvi) aryloxycarbonyl groups, in which the aryl part may be any one of those aryl groups exemplified above in relation to, inter alia, $R^8$;

(xvii) aralkyloxycarbonyl groups, in which the aralkyloxy part may be any one of those aralkyloxy groups exemplified above in relation to substituents on $R^{11}$;

(xviii) carbamoyl and thiocarbamoyl groups;

(xix) mono-and di-alkylcarbamoyl and mono-and di-alkyl(thiocarbamoyl) groups, in which the or each alkyl part is a $C_1$-$C_4$ alkyl group and may be chosen from those $C_1$-$C_4$ alkyl groups exemplified above in relation to $R^3$;

(xx) ureido and thioureido groups;

(xxi) mono-and di-alkylureido and mono-and di-alkyl(thioureido) groups, in which the or each alkyl part is a $C_1$-$C_4$ alkyl group, which may be chosen from any one of those $C_1$-$C_4$ alkyl groups exemplified above in relation to $R^3$;

(xxii) $C_3$-$C_8$ cycloalkyl groups, for example the cyclopropyl, cyclobutyl, cyclopentyl, cyclohexyl, cycloheptyl or cyclooctyl groups;

(xxiii) $C_5$-$C_8$ cycloalkenyl groups, for example the 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 4-cycloheptenyl, 1-cyclooctenyl, 2-cyclooctenyl, 3-cyclooctenyl or 4-cyclooctenyl groups;

(xxiv) aryl groups, which may be substituted or unsubstituted, for example any one of those aryl groups defined above in relation to $R^8$, preferably a phenyl group or a phenyl group having at least one substituent selected from hydroxy groups, halogen atoms, $C_1$-$C_4$ alkyl groups and $C_1$-$C_4$ alkoxy groups;

(xxv) heterocyclic groups, preferably aromatic heterocyclic groups, which may be unsubstituted or substituted, for example any one of those aromatic heterocyclic groups defined above in relation to $R^8$, preferably a furyl, thienyl, oxazolyl, isoxazolyl, thiazolyl, imidazolyl or pyridyl group, more preferably an isoxazolyl, thiazolyl or imidazolyl group and most preferably a thiazolyl or imidazolyl group, or, less preferably, any one of the non-aromatic groups described in relation to $R^{11}$, any of which may be unsubstituted or may have at least one of the previously defined substituents;

(xxvi) halogen atoms, for example the chlorine, fluorine, bromine or iodine atoms;

(xxvii) halomethyl groups, for example the chloromethyl, bromomethyl, iodomethyl, fluoromethyl, dichloromethyl, dibromomethyl, difluoromethyl and trifluoromethyl groups;

(xxviii) mercapto groups;

(xxix) amino groups;

(xxx) mono-and di-alkylamino groups in which the or each alkyl part is a $C_1$-$C_4$ alkyl group, for example the methylamino, ethylamino, propylamino, isopropylamino, butylamino, isobutylamino, sec-butylamino, dimethylamino, methyl(ethyl)amino, diethylamino, dipropylamino, diisopropylamino, dibutylamino, methyl(butyl)amino, ethyl(butyl)amino and diisobutylamino groups;

(xxxi) carboxy groups;

(xxxii) guanidino groups; and

(xxxiii) alkyl-substituted guanidino groups, especially mono-and di-substituted groups, for example the 1-methylguanidino, 2-methylguanidino, 3-methylguanidino, 1-ethylguanidino, 2-ethylguanidino, 3-ethylguanidino, 2-propylguanidino, 3-butylguanidino, 1,2-dimethylguanidino, 1,3-dimethylguanidino, 3,3-dimethylguanidino, 1-methyl-3-ethylguanidino and 2-ethyl-3-propylguanidino groups.

Where $R^4$ represents a $C_2$-$C_5$ alkenyl group or a $C_2$-$C_5$ alkynyl group, these may be straight or branched chain groups and, in the case of the alkenyl groups, they may be unsubstituted or may have at least one halogen subtituent. Examples of such groups include the vinyl, propenyl (1-propenyl or allyl), isopropenyl, butenyl (1-, 2-or 3-butenyl), pentenyl (1-, 2-, 3-or 4-pentenyl), 3-methyl-2-butenyl, 2-methyl-3-butenyl, 1-chlorovinyl, 2-chlorovinyl, 2,2-dichlorovinyl, 2-bromovinyl, 2-chloroallyl, 2,3-dichloroallyl, 3-chloro-1-propenyl, 4-bromo-2-butenyl, ethynyl, propynyl (2-propynyl and 3-propynyl), butynyl (1-, 2-and 3-butynyl) and pentynyl (1-, 2-, 3-and 4-pentynyl) groups, more preferably a $C_3$ or $C_4$ alkenyl or alkynyl group.

Where $R^4$ represents a $C_3$-$C_8$ cycloalkyl group, this may be any one of those described above in relation to substituents (d)-(xxii).

$R^5$ may represent an isopropyl group, a phenyl group or a $C_3$-$C_8$ cycloalkyl group. Where $R^5$ represents a $C_3$-$C_8$ cycloalkyl group, this may be a cyclopropyl, cyclobutyl, cyclopentyl, cyclohexyl, cycloheptyl or cyclooctyl group. It is preferably an isopropyl or cyclohexyl group.

$R^6$ represents a $C_1$-$C_6$ alkyl group, which may be a straight or branched chain group, and examples include the methyl, ethyl, propyl, isopropyl, butyl, isobutyl, sec-butyl, t-butyl, pentyl, isopentyl, neopentyl, t-pentyl, hexyl, isohexyl, sec-hexyl, 1-methylbutyl, 1,3-dimethylbutyl, 3,3-dimethylbutyl and 2-methylpentyl groups. It is preferably a $C_3$ or $C_4$ alkyl group.

$R^7$ represents a substituted $C_1$-$C_{10}$ alkyl group, which may be a straight or branched chain group, and examples of such alkyl groups are given in relation to the $C_1$-$C_{10}$ alkyl groups which may be represented by $R^4$ and $R^{11}$. Such alkyl groups represented by $R^7$ must contain at least one of substituents (e), i.e. the following substituents:

amino groups and protected amino groups; in the case of the protected amino groups, these may be protected by any of the acyl groups herein referred to and specific examples of such protected amino groups include the acetamido, propionamido, t-butyramido, t-butoxycarbonylamino, benzyloxycarbonylamino and benzamido groups;

aminoalkylamino groups, in which the alkyl part is a $C_1$-$C_4$ alkyl group and any of the amino parts may be unsubstituted or may have a $C_1$-$C_4$ alkyl substituent; examples of the alkyl part and of the alkyl substituents are included amongst the $C_1$-$C_4$ alkyl groups exemplified in relation to $R^3$; specific examples of such aminoalkylamino groups include the N-(3-aminopropyl)-N-methylamino, 3-aminopropylamino, aminomethylamino, 2-aminoethylamino and 4-aminobutylamino groups;

aminoalkylaminoalkylamino groups, in which each alkyl part is $C_1$-$C_4$ and may be as exemplified above in relation to $R^3$ and in which any of the amino groups may, if desired, have a $C_1$-$C_4$ alkyl substituent; examples of such aminoalkylaminoalkylamino groups include the aminomethylaminomethylamino, 2-aminoethylaminomethylamino, 2-(2-aminoethylamino)ethylamino, 3-(2-aminoethylamino)propylamino and 3-(3-aminopropylamino)propylamino groups;

guanidino and alkyl-substituted guanidino groups, examples of which have been given above in relation to substituents (d) on the alkyl groups represented by $R^4$;

substituted phenyl groups having a $C_1$-$C_4$ aminoalkyl substituent (e.g. an aminomethyl, 2-aminoethyl, 3-aminopropyl, 2-aminopropyl or 4-aminobutyl group) or a di-substituted $C_1$-$C_4$ alkoxy group, wherein the alkoxy part is as exemplified above in relation to $R^{11}$ and in which one of the substituents is a hydroxy group and the other is an amino group, a mono-or dialkylamino group, a heterocyclic group or an alkylamino group having a heterocyclic substituent on the alkyl part; and

sulpho groups.

If desired, the substituted $C_1$-$C_{10}$ alkyl group represented by $R^7$ may have, in addition to at least one of the above substituents, one or more of substituents (f), i.e. the following substituents:

hydroxy groups;

carboxy groups;

$C_2$-$C_5$ alkoxycarbonyl groups, e.g. as exemplified above in relation to substituents (a) on the alkylcarbamoyl groups represented by $R^1$;

carbamoyl groups; and

mono-and di-alkylcarbamoyl groups in which the or each alkyl part is $C_1$-$C_4$, e.g. as exemplified above in relation to $R^1$.

The alkyl group represented by $R^7$ is preferably substituted either by a m-aminomethylphenyl group or by two groups. In the latter case, one of the groups is an amino or protected amino group and the other is a hydroxy, carboxy, $C_2$-$C_5$ alkoxycarbonyl or carbamoyl group. More preferably, the alkyl group represented by $R^7$ is substituted by the said two groups.

Aromatic acyl groups referred to herein are preferably arylcarbonyl groups in which the aryl part is as defined above. Preferred such groups are the benzoyl and naphthoyl groups, which may be unsubstituted or have one or more of substituents (d), (g) and (h), defined above. Examples include the benzoyl, p-nitrobenzoyl, 1-naphthoyl and 2-naphthoyl groups.

Specific examples of groups which may be represented by $R^1$ include the benzyl, o -chlorobenzyl, m-chlorobenzyl, p-chlorobenzyl, o-methylbenzyl, m-methylbenzyl, p-methylbenzyl, 3,4-dimethoxybenzyl, phenethyl, o-methoxybenzyl, m-methoxybenzyl, p-methoxybenzyl, o-methoxyphenethyl, m-methoxyphenethyl, p -methoxyphenethyl, 3-phenylpropyl, 4-phenylbutyl, 1-phenylbutyl, 3-phenylbutyl, 2-phenylbutyl, 2-indenylmethyl, 3-methyl-2-indenylmethyl, 1-naphthylmethyl, 2-naphthylmethyl, furfuryl, 5-methyl-2-furylmethyl, 2-methyl-3-furylmethyl, 2-thenyl, 5-methyl-2-thenyl, 3-methyl-2-thenyl, 2-(3-thienyl)ethyl, 2-(2-thienyl)ethyl, 4-(2-thienyl)butyl, 2-(4-methyl-5-thiazolyl)ethyl, 4-pyridylmethyl, 3-pyridylmethyl, 2-pyridylmethyl, 2-(2-pyridyl)ethyl, 3-(3-pyridyl)propyl, 5-methoxy-2-indolylmethyl, 3-indolylmethyl, 1-methyl-2-indolylmethyl, 2-methyl-3-indolylmethyl, 2-benzimidazolylmethyl, 4-quinolylmethyl, 3-quinolylmethyl, 2-quinolylmethyl, 2-(2-methoxyethoxy)ethyl, 2-(2-ethoxyethoxy)ethyl, 1-pyrrolidinylmethyl, 2-(1-pyrrolidinyl)-ethyl, piperidinomethyl, 2-piperidinoethyl, 3-piperidinopropyl, 2-(1-methyl-2-pyrrolidinyl)ethyl, morpholinomethyl, 2-morpholinoethyl, 3-morpholinopropyl, 4-ethyl-1-piperazinylmethyl, 4-phenyl-1-piperazinylmethyl, 4-(p-ethoxycarbonylphenyl)-1-piperazinylmethyl, 2-(4-methyl-1-piperazinyl)ethyl, 2-[4-(p-chlorophenyl)-1-piperazinyl]ethyl, morpholinocarbonylmethyl, 1-pyrrolidinylcarbonylmethyl, piperidinocarbonylmethyl, 4-methyl-1-piperazinylcarbonylmethyl, thiomorpholinocarbonylmethyl, 3-(morpholinocarbonyl)-propyl, 2-pyrrolidinylcarbonylmethyl, 1-acetyl-2-pyrrolidinylcarbonylmethyl, 1-pivaloyloxy-2-pyrrolidinylcarbonylmethyl, 1-nicotinoyl-2-pyrrolidinylcarbonylmethyl, acetamido, propionamido, butyramido, isobutyramido, valeramido, hexanamido, heptanamido, octanamido, nonanamido, decanamido, undecanamido, α-methoxyacetamido, α-ethoxyacetamido, 3-ethoxypropionamido, 3-methoxypropionamido, α-phenylacetamido 4-phenylbutyramido, 3-phenylbutyramido, 2-phenylbutyramido, α-(4-benzyl-1-piperazinyl)-acetamido, α-(4-methyl-1-piperazinyl)acetamido, α-(4-phenyl-1-piperazinyl)acetamido, α-[4-(p -methoxyphenyl)-1-piperazinyl]acetamido, α-[4-(2-pyridyl)-1-piperazinyl]acetamido, morpholinoacetamido, prolylamino, N-t-butoxycarbonyl-prolylamino, N-benzyloxycarbonyl-prolylamino, benzamido, p-toluamido, p-methoxybenzamido, p-chlorobenzamido, p-hydroxybenzamido, 2-pyridinecarboxamido, nicotinamido, isonicotinamido, 2-quinolinecarboxamido, 2-furoylamino, 2-thenoylamino, 2-pyrazinecarboxamido, methoxycarbonylamino, ethoxycarbonylamino, propoxycarbonylamino, butoxycarbonylamino, t-butoxycarbonylamino, benzyloxycarbonylamino, p -methoxybenzyloxycarbonylamino, phenoxycarbonylamino, α-(2-pyridyl)acetamido, α-(3-pyridyl)acetamido, 2-(2-methoxyethoxy)ethoxycarbonylamino, o-hydroxybenzamido, o-acetoxybenzamido, 2,3-dihydroxybenzamido, 2,3-diacetoxybenzamido, 5-amino-2-nitrobenzamido, 4-t-butoxycarbonyl-1-pyrrolidinecarboxamido, pivaloylmethyl, carbamoyl, methylcarbamoyl, ethylcarbamoyl, t-butylcarbamoyl, carboxymethylcarbamoyl and ethoxycarbonylmethylcarbamoyl groups.

Specific examples of groups which may be represented by $R^2$ include the benzyl, o-chlorobenzyl, m-chlorobenzyl, p-chlorobenzyl, o-methylbenzyl, m-methylbenzyl, p-methylbenzyl, 3,4-dimethoxybenzyl, phenethyl, o-methoxybenzyl, m-methoxybenzyl, p-methoxybenzyl, 2,3,4,5,6-pentamethylbenzyl, o-aminobenzyl, m-aminobenzyl, p-aminobenzyl, o-nitrobenzyl, m-nitrobenzyl, p-nitrobenzyl, o -methoxyphenethyl, m-methoxyphenethyl, p-methoxyphenethyl, 3-phenylpropyl, 4-phenylbutyl, 1-phenylbutyl, 3-phenylbutyl, 2-phenylbutyl, 1-indenylmethyl, 2-indenylmethyl, 3-methyl-2-indenylmethyl, 1-naphthylmethyl, 2-naphthylmethyl, furfuryl, 5-methyl-2-furylmethyl, 2-methyl-3-furylmethyl, 2-thenyl, 5-methyl-2-thenyl, 3-methyl-2-thenyl, 2-(3-thienyl)ethyl, 2-(2-thienyl)ethyl, 4-(2-thienyl)butyl, 2-(4-methyl-5-thiazolyl)-ethyl, 4-pyridylmethyl, 3-pyridylmethyl, 2-pyridylmethyl, 2-(2-pyridyl)ethyl, 3-(3-pyridyl)propyl, 5-methoxy-2-indolylmethyl, 2-indolylmethyl, 3-indolylmethyl, 1-methyl-2-indolylmethyl, 2-methyl-3-indolylmethyl, 2-benzimidazolylmethyl, 4-quinolylmethyl, 3-quinolylmethyl, 2-quinolylmethyl, 3-benzothienylmethyl, 1,7-diazaindenyl, 1-pyrrolidinylmethyl, 2-(1-pyrrolidinyl)ethyl, piperidinomethyl, 2-piperidinoethyl, 3-piperidinopropyl, 2-(1-methyl-2-pyrrolidinyl)ethyl, morpholinomethyl, 2-morpholinoethyl, 3-morpholinopropyl, 4-ethyl-1-piperazinylmethyl, 4-phenyl-1-piperazinylmethyl, 4-(p-ethoxycarbonylphenyl)-1-piperazinylmethyl, 2-(4-methyl-1-piperazinyl)ethyl and 2-[4-(p-chlorophenyl)-1-piperazinyl]ethyl groups.

$R^3$ preferably represents a hydrogen atom or a methyl group.

Preferred examples of groups which may be represented by $R^4$ include the hydrogen atom and the methyl, ethyl, propyl, isopropyl, butyl, isobutyl, sec-butyl, pentyl, isopentyl, hexyl, heptyl, octyl, nonyl, decyl, fluoromethyl, chloromethyl, 2,2-dichloroethyl, 2,2,2-trichloroethyl, 3,3,3-trifluoro-2-(trifluoromethyl)-propyl, hydroxymethyl, 2-hydroxyethyl, 1-hydroxyethyl, 2-hydroxy-1-methylethyl, methoxymethyl, ethoxymethyl, t-butoxymethyl, 2-methoxyethyl, 2-ethoxyethyl, phenoxymethyl, benzyloxymethyl, 1-benzyloxyethyl, acetoxymethyl, mercaptomethyl, 2-mercaptoethyl, 2-methylthioethyl, 2-ethylthioethyl, 3-methylthiopropyl. 4-methylthiobutyl, phenylthiomethyl, benzylthiomethyl, p-methoxybenzylthiomethyl, 2-methanesulphinylethyl, 2-methanesulphonylethyl, 2-benzenesulphonylethyl, aminomethyl, 2-aminoethyl, 3-aminopropyl, 4-aminobutyl, 2-(diethylamino)ethyl, (dimethylamino)methyl, acetamidomethyl, 4-propionamidobutyl, 4-t-butoxycarbonylaminobutyl, 3-benzyloxycarbonylaminopropyl, carboxymethyl, 2-carboxyethyl, methoxycarbonylmethyl, t-butoxycarbonylmethyl, 2-t-butoxycarbonylethyl, phenoxycarbonylmethyl, benzyloxycarbonylmethyl, carbamoylmethyl, 2-carbamoylethyl, methylcarbamoylmethyl, (diethylcarbamoyl)-methyl, ureidomethyl, 3-ureidopropyl, thioureidomethyl, 2-guanidinoethyl, 3-guanidinopropyl, 4-guanidinobutyl, cyclopropylmethyl, cyclopentylmethyl, cyclohexylmethyl, 2-cyclohexylethyl, 2-cyclopentenylmethyl, 2-(2-cyclopentenyl)ethyl, 1-cyclohexenylmethyl, 3-cyclohexenylmethyl, 1-cycloheptenylmethyl, benzyl, phenethyl, o-fluorobenzyl, m-fluorobenzyl, p-fluorobenzyl, o-methylbenzyl, m-methylbenzyl, p-methylbenzyl, o-chlorobenzyl, m-chlorobenzyl, p-chlorobenzyl, o-hydroxybenzyl, m-hydroxybenzyl, p-hydroxybenzyl, o-methoxybenzyl, m-methoxybenzyl, p-methoxybenzyl, o-nitrobenzyl, m-nitrobenzyl, p-nitrobenzyl, o-aminobenzyl, m-aminobenzyl, p-aminobenzyl, o-bromobenzyl, m-bromobenzyl, p-bromobenzyl, 2,3,4,5,6-pentamethylbenzyl, furfuryl, thenyl, 2-pyridylmethyl, 3-pyridylmethyl, 4-pyridylmethyl, 4-thiazolylmethyl, 4-methyl-5-thiazolylmethyl, 2-methyl-4-thiazolylmethyl, 5-isoxazolylmethyl, 5-imidazolylmethyl, 3-indolylmethyl, 3-benzothienylmethyl, cyclopropyl, cyclopentyl, cyclohexyl, allyl, methallyl, 2-butenyl, 3,3-dichloroallyl and 2-propynyl groups.

Preferred examples of groups which may be represented by $R^5$ include the isopropyl, cyclohexyl and phenyl groups.

Preferred examples of groups which may be represented by $R^6$ include the propyl, isopropyl, butyl, isobutyl, sec-butyl, pentyl and isopentyl groups.

Preferred examples of groups which may be represented by $R^7$ include the 2-aminoethyl, 3-aminopropyl, 4-aminobutyl, 5-aminopentyl, 6-aminohexyl, 8-aminooctyl, 9-aminononyl, 2-(2-aminoethylamino)ethyl, 2-[N-methyl-N -(2-aminoethyl)amino]ethyl, 2-[2-(2-aminoethylamino)ethylamino]ethyl, m-aminomethylbenzyl, m-(2-hydroxy-3-isopropylaminopropoxy)benzyl, p-(2-hydroxy-3-isopropylaminopropoxy)benzyl, o-(2-hydroxy-3-isopropylaminopropoxy)benzyl, m-(3-amino-2-hydroxypropoxy)benzyl, p-(3-amino-2-hydroxypropoxy)benzyl, o-(3-amino-2-hydroxypropoxy)benzyl, p-(2-hydroxy-3-propylaminopropoxy)benzyl, m-(2-hydroxy-3-propylaminopropoxy)benzyl, m-(3-t-butylamino-2-hydroxypropoxy)benzyl, p-(3-t-butylamino-2-hydroxypropoxy)benzyl, o-(3-t-butylamino-2-hydroxypropoxy)benzyl, p-[2-hydroxy-3-(4-methyl-1-piperazinyl)propoxy]benzyl, m-[2-hydroxy-3-(4-methyl-1-piperazinyl)propoxy]-benzyl, p-[2-hydroxy-3-(2-morpholinoethylamino)propoxy]benzyl, m-[2-hydroxy-3-(2-morpholinoethylamino)-propoxy]benzyl, m-(2-hydroxy-3-morpholinopropoxy)benzyl, p-(2-hydroxy-3-morpholinopropoxy)benzyl, o-(2-hydroxy-3-morpholinopropoxy)benzyl, o-[2-hydroxy-3-(4-methyl-1-piperazinyl)propoxy]benzyl, 2-sulphoethyl, 2-amino-1-carboxyethyl, 2-amino-2-carbamoylethyl, 2-amino-1-carbamoylethyl, 2-amino-3-hydroxypropyl, 3-amino-1-carboxypropyl, 3-amino-1-ethoxycarbonylpropyl, 3-amino-1-carbamoylpropyl, 3-amino-1-hydroxymethylpropyl, 4-amino-1-carboxybutyl, 4-amino-1-carbamoylbutyl, 4-amino-1-ethoxycarbonylbutyl, 4-amino-1-hydroxymethylbutyl, 5-amino-1-carboxypentyl, 5-amino-1-ethoxycarbonylpentyl, 5-amino-1-carbamoylpentyl, 5-amino-1-methylcarbamoylpentyl, 5-amino-1-dimethylcarbamoylpentyl 5-amino-1-hydrox-

ymethylpentyl. 5-amino-5-ethoxycarbonylpentyl. 5-amino-5-carbamoylpentyl, 5-amino-6-hydroxyhexyl, 1-carboxy-4-guanidinobutyl, 1-carbamoyl-5-guanidinopentyl, 5-guanidino-1-hydroxymethylpentyl 5-amino-1,5-dicarboxypentyl, 5-amino-1,5-dicarbamoylpentyl, 5-amino-6-hydroxy-1-hydroxymethylhexyl and 3-amino-2-hydroxypropyl groups.

Of the compounds listed above, one preferred class of compounds of the invention are those compounds in which:

m is the cypher 0 or the integer 1;

nis the cypher 0 or the integer 1;

$R^1$ represents a group of formula $-A-R^8$
wherein A represents a $C_1$-$C_4$ alkylene group and $R^8$ represents an aryl group, an aromatic heterocyclic group, a $C_2$-$C_5$ aliphatic carboxylic acyl group or a heterocyclic carboxylic acyl group,

an alkylcarbamoyl group in which the alkyl part is $C_1$-$C_4$ alkyl, a dialkylcarbamoyl group in which each alkyl part is $C_1$-$C_4$ alkyl, a substituted alkylcarbamoyl group in which the alkyl part is $C_1$-$C_4$ alkyl and has at least one carboxy substituent, a substituted dialkylcarbamoyl group in which each alkyl part is $C_1$-$C_4$ alkyl and in which at least one of said alkyl parts has at least one carboxy substituent, or a group of formula $-NHCOR^{11}$ wherein $R^{11}$ represents a $C_1$-$C_4$ alkyl group, a $C_7$-$C_9$ aralkyl group, a $C_1$-$C_4$ alkyl group having a heterocyclic substituent, an aryl group, a heterocyclic group, a $C_1$-$C_4$ alkoxy group, an alkoxyalkoxyalkoxy group in which each alkoxy part is $C_1$-$C_4$ or a $C_7$-$C_9$ aralkyloxy group,
provided that, when both m = 1 and n = 1, $R^1$ represents said group of formula $-A-R^8$ or said alkylcarbamoyl, dialkylcarbamoyl, substituted alkylcarbamoyl or substituted dialkylcarbamoyl group;

$R^2$ represents a group of formula $-B-R^{12}$, in which B represents a $C_1$-$C_4$ alkylene group and $R^{12}$ represents an aryl group or an aromatic heterocyclic group;

$R^3$ represents a hydrogen atom or a methyl group;

$R^4$ represents a hydrogen atom, a $C_1$-$C_6$ alkyl group, a $C_1$-$C_6$ alkyl group having at least one of substituents -(k), a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^6$ represents a $C_1$-$C_6$ alkyl group;

$R^7$ represents a substituted $C_2$-$C_6$ alkyl group having at least one of substituents (m) or a substituted $C_2$-$C_6$ alkyl group having at least one of substituents (m) and at least one of substituents (f), defined above;

said heterocyclic groups have 5 or 6 ring atoms, of which from 1 to 3 are nitrogen, oxygen or sulphur hetero-atoms, and are unsubstituted or have at least one of substituents (d) and/or (g) and/or (h) and/or (i), defined above;

substituents (k):hydroxy groups, $C_1$-$C_4$ alkoxy groups, $C_1$-$C_4$ alkylthio groups, $C_1$-$C_7$ aliphatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, $C_3$-$C_6$ cycloalkyl groups, aryl groups, aromatic heterocyclic groups, mercapto groups, amino groups, alkylamino groups in which the alkyl part is $C_1$-$C_4$ alkyl, dialkylamino groups in which each alkyl part is $C_1$-$C_4$ alkyl, carboxy groups, guanidino groups, alkylguanidino groups in which the alkyl part is $C_1$-$C_4$ and dialkylguanidino groups in which each alkyl part is $C_1$-$C_4$;

substituents (m):amino groups, protected amino groups, $C_1$-$C_4$ aminoalkylamino groups, aminoalkylaminoalkylamino groups in which each alkyl part is $C_1$-$C_4$, guanidino groups, alkylguanidino groups in which the alkyl part is $C_1$-$C_4$, dialkylguanidino groups in which each alkyl part is $C_1$-$C_4$, substituted phenyl groups having at least one of substituents (n) and sulpho groups; and

substituents (n):$C_1$-$C_4$ aminoalkyl groups and di-substituted $C_1$-$C_4$ alkoxy groups, in which one substituent is the hydroxy group and the other is selected from amino groups, $C_1$-$C_4$ alkylamino groups, dialkylamino groups in which each alkyl part is $C_1$-$C_4$ and heterocyclic groups.

Of these, a more preferred class of compounds of the invention are those compounds in which:

m is 1;

n is 0;

$R^1$ represents a group of formula $-A-R^8$ in which A represents a $C_1$ or $C_2$ alkylene group and $R^8$ represents a phenyl group, a naphthyl group or a nitrogen-containing aromatic heterocyclic group,

an alkylcarbamoyl group in which the alkyl part is $C_1$-$C_4$ alkyl, a dialkylcarbamoyl group in which each alkyl part is $C_1$-$C_4$ alkyl, a carboxyalkylcarbamoyl group in which the alkyl part is $C_1$-$C_4$ alkyl, or a group of formula $-NHCOR^{11}$ in which $R^{11}$ represents a $C_1$-$C_4$ alkyl group, a $C_1$-$C_4$ alkyl group having a heterocyclic substituent, a phenyl group, a phenyl group having at least one of substituents (d) and/or (g) and/or (h), a nitrogen-containing heterocyclic group or a $C_7$-$C_9$ aralkyloxy group;

$R^2$ represents a group of formula $-B-R^{12}$, in which B represents a $C_1$ or $C_2$ alkylene group, and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ alkyl and the phenyl group is unsubstituted or has at least one of substituents (d) and/or (g) and/or (h), a $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^7$ represents a substituted $C_2$-$C_6$ alkyl group having at least one substituent of substituents (o) or a substituted $C_2$-$C_6$ alkyl group having at least one of substituents (o) and at least one of substituents (p);

said heterocyclic groups have 5 or 6 ring atoms of which 1 or 2 are nitrogen, oxygen or sulphur hetero-atoms, and said heterocyclic groups being unsubstituted or having at least one of substituents (d) and/or - (g) and/or (h) and/or (i), defined above;

substituents (o):amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

An alternative more preferred class of compounds of the present invention are those compounds in which:

m is 1;

n is 1;

$R^1$ represents a group of formula -A-$R^8$
in which A represents a $C_1$ or $C_2$ alkylene group and $R^8$ represents a phenyl group, a naphthyl group or a nitrogen-containing aromatic heterocyclic group,
an alkylcarbamoyl group in which the alkyl part is $C_1$-$C_4$, a dialkylcarbamoyl group in which each alkyl part is $C_1$-$C_4$ or a carboxyalkylcarbamoyl group in which the alkyl part is $C_1$-$C_4$;

$R^2$ represents a group of formula -B-$R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h), a $C_1$-$C_6$ alkyl group having an aromatic heterocyclic substituent, a $C_3$ or $C_4$ alkenyl group or a $C_3$-$C_4$ alkynyl group;

$R^5$ represents an isopropyl group, a cyclopentyl group, a cyclohexyl group or a phenyl group;

$R^6$ represents a $C_3$ or $C_4$ alkyl group;
$R^7$ represents a substituted $C_2$-$C_6$ alkyl group having at least one of substituents (o) or a substituted $C_2$-$C_6$ alkyl group having at least one of substituents (o) and at least one of substituents (p);
and

said heterocyclic groups have 5 or 6 ring atoms, of which 1 or 2 are nitrogen, oxygen or sulphur hetero-atoms, said heterocyclic group being unsubstituted or having at least one of substituents (d) and/or (g) and/or (h).

Another more preferred class of compounds of the present invention are those compounds in which:

m and n are both 0;

$R^1$ represents a group of formula -A-$R^8$,
in which A represents a $C_1$ or $C_2$ alkylene group, and $R^8$ represents a phenyl group, a naphthyl group or a nitrogen-containing aromatic heterocyclic group,
an alkylcarbamoyl group in which the alkyl part is $C_1$-$C_4$, a dialkylcarbamoyl group in which each alkyl part is $C_1$-$C_4$, a carboxyalkylcarbamoyl group in which the alkyl part is $C_1$-$C_4$ or a group of formula -NHCOR$^{11}$,
in which $R^{11}$ represents a $C_1$-$C_4$ alkyl group, a $C_1$-$C_4$ alkyl group having a heterocyclic substituent, a phenyl group, a phenyl group having at least one of substituents (d) and/or (g) and/or (h), a nitrogen-containing heterocyclic group or a $C_7$-$C_9$ aralkyloxy group;

$R^2$ represents a group of formula -B-$R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h), a $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a cyclopentyl group, a cyclohexyl group or a phenyl group;

$R^7$ represents a substituted $C_2$-$C_6$ alkyl group having at least one of substituents (o) or a substituted $C_2$-$C_6$ alkyl group having at least one of substituents (o) and at least one of substituents (p); and

said heterocyclic groups and said heterocyclic substituents have 5 or 6 ring atoms of which 1 or 2 are nitrogen, oxygen or sulphur hetero-atoms, said groups and substituents being unsubstituted or having at least one of substituents (d) and/or (g) and/or (h), defined above.

The most preferred compounds of the invention are those in which:

m is 1;

n is 0;

$R^1$ represents a group of formula -A-$R^8$
in which A represents a $C_1$ or $C_2$ alkylene group and $R^8$ represents a phenyl group, a naphthyl group or a nitrogen-containing aromatic heterocyclic group,
an alkylcarbamoyl group in which the alkyl part is $C_1$-$C_4$ alkyl, a dialkylcarbamoyl group in which each alkyl part is $C_1$-$C_4$ alkyl or a group of formula -NHCOR$^{11}$,
in which $R^{11}$ represents a $C_1$-$C_4$ alkyl group, a $C_1$-$C_4$ alkyl group having a heterocyclic substituent or a nitrogen-containing heterocyclic group;

$R^2$ represents a group of formula -B-$R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_3$ or $C_4$ alkyl group, a phenylalkyl group in which the alkyl part is $C_1$ or $C_2$ alkyl and the phenyl part is unsubstituted or has at least one of substituents (d) and/or (g) and/or (h), a $C_1$ or $C_2$ alkyl group having an aromatic heterocyclic substituent, a $C_3$ or $C_4$ alkenyl group or a $C_3$ or $C_4$ alkynyl group;

$R^5$ represents an isopropyl group or a cyclohexyl group; and

$R^7$ represents a substituted $C_2$-$C_6$ alkyl group having at least one amino and/or protected amino substituent or a substituted $C_2$-$C_6$ alkyl group having at least one amino and/or protected amino substituent and at least one of substituents (p), defined above.

An alternative preferred group of compounds of the present invention are those compounds in which:

m is 1;

n is the cypher 0 or the integer 1;

$R^1$ represents a group of formula -A-$R^8$,
wherein A represents a $C_1$-$C_4$ alkylene group and $R^8$ represents an aryl group or an aromatic heterocyclic group having 5 or 6 ring atoms,
or a group of formula -NHCOR$^{11}$,
wherein $R^{11}$ represents a $C_1$-$C_4$ alkyl group, a $C_7$-$C_9$ aralkyl group, a $C_1$-$C_4$ alkyl group having a heterocyclic substituent wherein the heterocyclic substituent has 5 or 6 ring atoms, an aryl group, an aromatic heterocyclic group having from 5 to l0 ring atoms, a $C_1$-$C_4$ alkoxy group or a $C_7$-$C_9$ aralkyloxy group,
provided that, when both m = 1 and n = 1, $R^1$ represents said group of formula -A-$R^8$;

$R^2$ represents a group of formula -B-$R^{12}$, in which B represents a $C_1$-$C_4$ alkylene group, and $R^{12}$ represents an aryl group or an aromatic heterocyclic group having 5 or 6 ring atoms;

$R^3$ represents a hydrogen atom;

$R^4$ represents a hydrogen atom, a $C_1$-$C_6$ alkyl group, a $C_1$-$C_6$ alkyl group having at least one of substituents (q), a $C_2$-$C_5$ alkenyl group, a $C_2$-$C_5$ alkenyl group having at least one halogen substituent or a $C_2$-$C_5$ alkynyl group;

$R^5$ represents an isopropyl group, a cyclopentyl group, a cyclohexyl group or a phenyl group;

$R^6$ represents a $C_1$-$C_5$ alkyl group;

$R^7$ represents a substituted $C_2$-$C_6$ alkyl group having at least one of substituents (r) or a substituted $C_2$-$C_6$ alkyl group having at least one of substituents (r) and at least one of substituents (f), defined above;

said heterocyclic groups and heterocyclic substituents have from 1 to 3 ring hetero-atoms selected from nitrogen, oxygen and sulphur hetero-atoms and are unsubstituted or have at least one of substituents (d) and/or (g) and/or (h) and/or (i) defined above;

<u>substituents (q)</u>:hydroxy groups, $C_1$-$C_4$ alkoxy groups, $C_7$-$C_9$ aralkyloxy groups, $C_1$-$C_7$ aliphatic carboxylic acyloxy groups, $C_1$-$C_4$ alkylthio groups, $C_7$-$C_9$ aralkylthio groups, $C_1$-$C_4$ alkylsulphinyl groups, $C_1$-$C_4$ alkylsulphonyl groups, $C_1$-$C_7$ aliphatic carboxylic acylamino groups, aromatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, aryloxycarbonyl groups, $C_8$-$C_{10}$ aralkyloxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, thiocarbamoyl groups, alkyl(thiocarbamoyl) groups in which the alkyl part is $C_1$-$C_4$ alkyl, dialkyl(thiocarbamoyl) groups in which each alkyl part is $C_1$-$C_4$, ureido groups, thioureido groups, $C_3$-$C_8$ cycloalkyl groups, $C_5$-$C_8$ cycloalkenyl groups, aryl groups, aromatic heterocyclic groups, halogen atoms, halomethyl groups, mercapto groups, amino groups, $C_1$-$C_4$ alkylamino groups, dialkylamino groups in which each alkyl part is $C_1$-$C_4$ alkyl, carboxy groups and guanidino groups;

<u>substituents (r)</u>:amino groups, protected amino groups, $C_1$-$C_4$ aminoalkylamino groups, aminoalkylaminoalkylamino groups in which each alkyl part is $C_1$-$C_4$, guanidino groups, substituted phenyl groups having at least one of substituents (n), defined above and sulpho groups.

In general, where reference is made herein to "substituted" groups, there is no specific maximum limit to the number of possible substituents, although, in specific cases, steric constraints may impose a practical maximum limit. In principle, the maximum number of substituents is determined by the number of substitutable atoms and, where the substituent is relatively small, complete substitution may be possible. For example where the substituent is relatively small such as a halogen (e.g. fluorine or chlorine) atom, the substituted group may range from the monohalo to perhalo groups, e.g. from monohaloalkyl to perhaloalkyl. Where the substituent is "bulkier", e.g. a t-butyl group, steric effects may prevent substitution of all substitutable positions. However, these effects are well known to those skilled in the art and require no further discussion here.

The compounds of the invention contain at least one asymmetric carbon atom, that is to say the carbon atom to which the group represented by $R^5CH_2$ is attached, and can, depending upon the values of the various substituent groups $R^1$-$R^7$ defined above, also contain other asymmetric carbon atoms. Accordingly, a variety of optical isomers are possible. The present invention envisages both the individual isolated isomers as well as mixtures (e.g. racemates) thereof. However, we particularly prefer those isomers in which the carbon atom to which the group represented by $R^5CH_2$ is attached has the <u>S</u>-configuration and, where $R^4$ represents a group other than hydrogen, we prefer those isomers in which the carbon atom to which the group represented by $R^4$ is attached also has the <u>S</u>-configuration. Where optically active starting materials are employed to produce the compounds of the invention and/or stereo-specific routes are employed, it may be possible to produce individual isomers of the compounds of the invention. In other cases, mixtures of various isomers may be produced and, in such a case, these mixtures may be used as such or the individual isomers may be isolated by well-known techniques.

The compounds of the invention contain basic nitrogen atoms and, depending upon the value of certain of the substituents represented by $R^1$, $R^2$, $R^4$ and $R^7$, may also contain free carboxy groups. Accordingly, the compounds of the invention will normally form acid addition salts and may, where they contain at least one carboxy group, also form salts with cations. The nature of the acid or cation employed to form such a salt is not critical to the invention, except where the compounds of the invention are intended for therapeutic use,

in which case the resulting salt must be pharmaceutically acceptable which, as is well-known to those skilled in the art, means that the salt must not have an increased toxicity or substantially increased toxicity or a reduced activity or substantially reduced activity, as compared with the free compound of formula (I). However, where the compounds of the invention are intended for non-therapeutic use, e.g. as intermediates, even this limitation need not apply.

Examples of acids which can be employed to form acid addition salts include: mineral acids, such as hydrochloric acid, sulphuric acid and phosphoric acid; organic carboxylic acids, such as oxalic acid, maleic acid, succinic acid and citric acid; and organic sulphonic acids, such as methanesulphonic acid, benzenesulphonic acid and p-toluenesulphonic acid. The compounds may also form salts with: alkali and alkaline earth metals, such as sodium, potassium, calcium and magnesium; and organic bases, such as dicyclohexylamine.

The compounds of the invention having a carboxy group may likewise form esters and those esters which are particularly preferred in the present invention are defined as the various substituted oxycarbonyl groups. However, it will be appreciated that other esters may likewise be employed.

Specific examples of compounds of the invention are given by the following formulae (I-1) to (I-14) in which the substituent groups are as defined in subsequent Tables 1-14, respectively. In these Tables, the following abbreviations are used:

Ac acetyl
tBoc t-butoxycarbonyl
Boz benzoyl
Bu butyl
iBu isobutyl
sBu sec-butyl
tBu t-butyl
Bz benzyl
Byr butyryl
Car carbamoyl
Et ethyl
Etc ethoxycarbonyl
Fur furyl
Guan guanidino
Hp heptyl
cHp cycloheptyl
Hx hexyl
cHx cyclohexyl
Imid imidazolyl
Ind indolyl
Isox isoxazolyl
Me methyl
Mor morpholino
Nic nicotinoyl
Np naphthyl
Ph phenyl
Pip piperidyl
Piz piperazinyl
Pn pentyl
c Pn cyclopentyl
Pr propyl
iPr isopropyl
Pyr pyridyl
Pyrd pyrrolidinyl
Pyz pyrazinyl
Thi thienyl
Thiz 1,3-thiazolyl

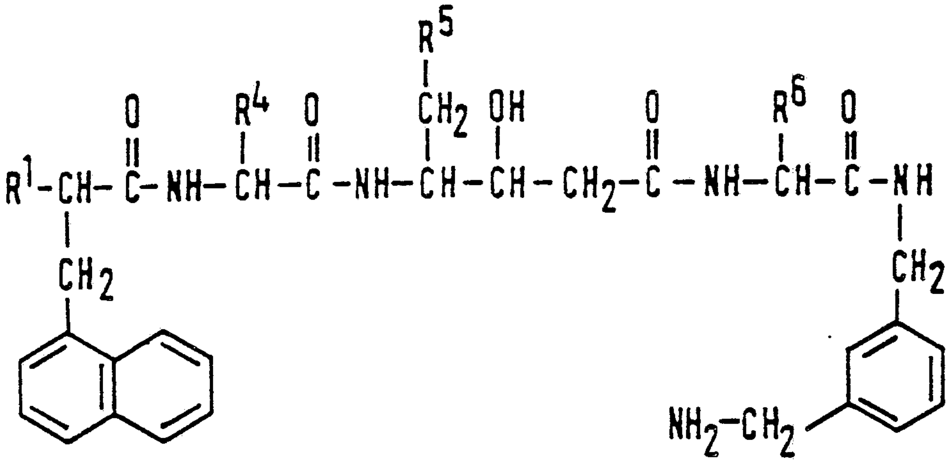

(I-1)

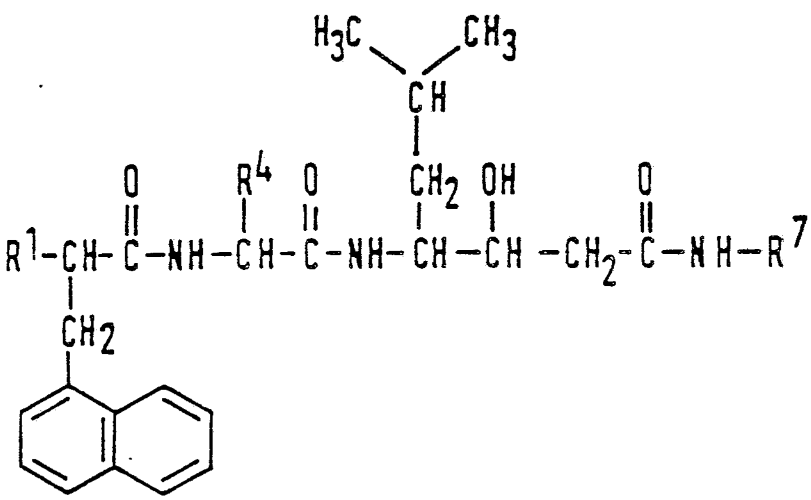

(I-2)

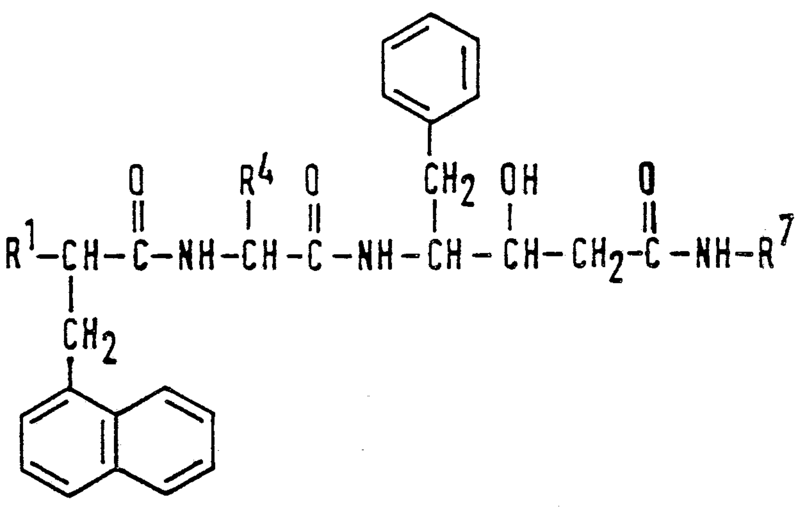

(I-3)

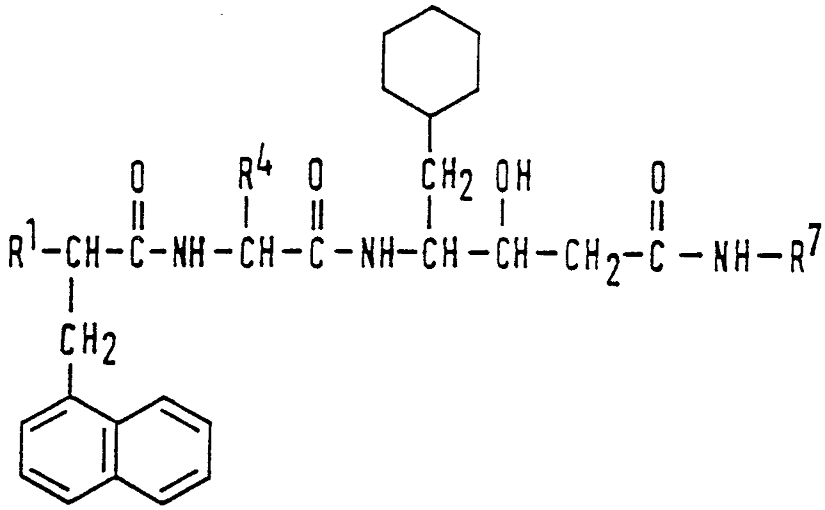

(I-4)

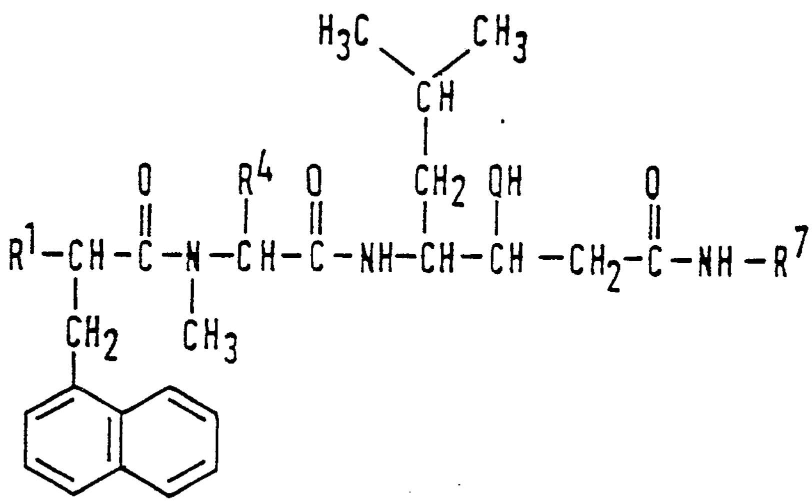

(I-5)

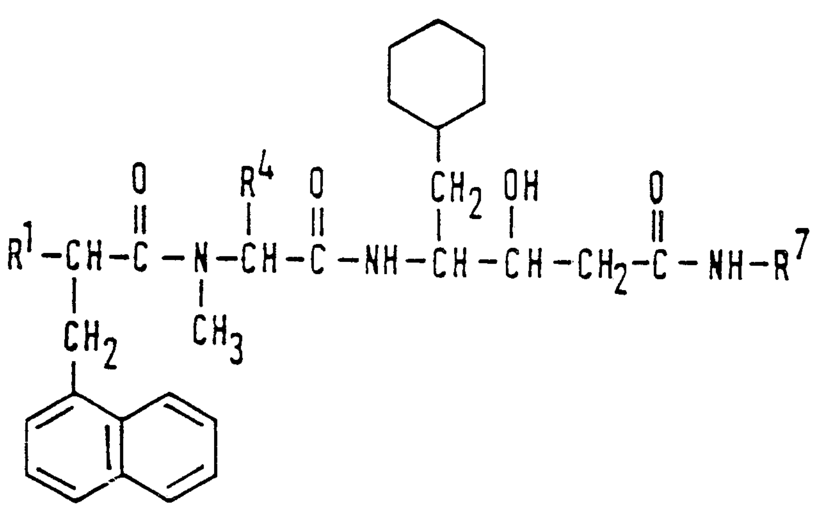

(I-6)

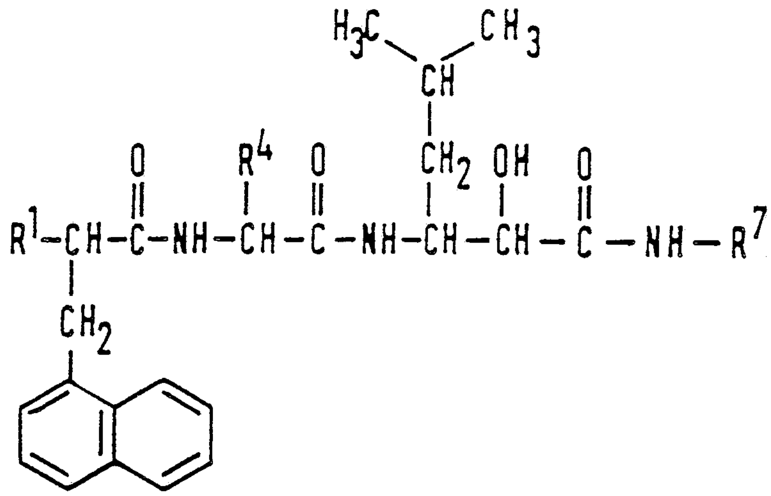

(I-7)

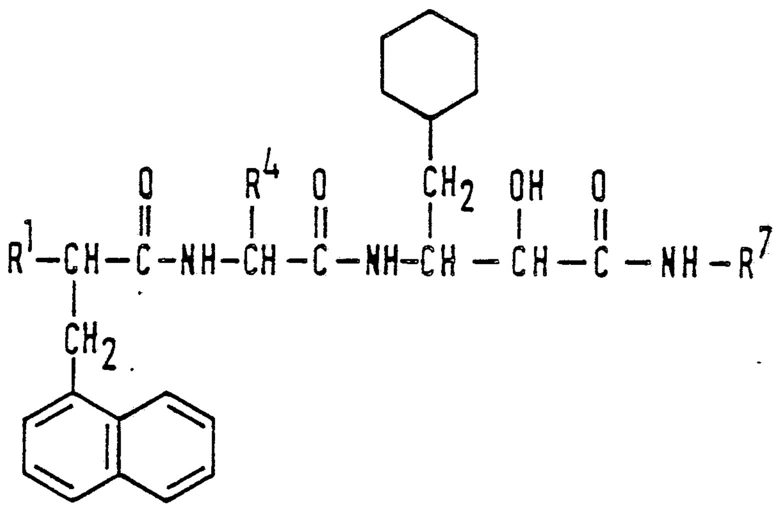

(I-8)

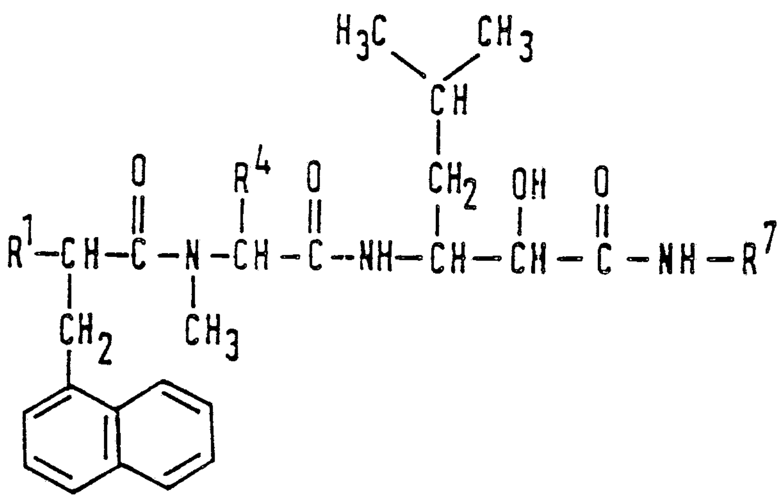

(I-9)

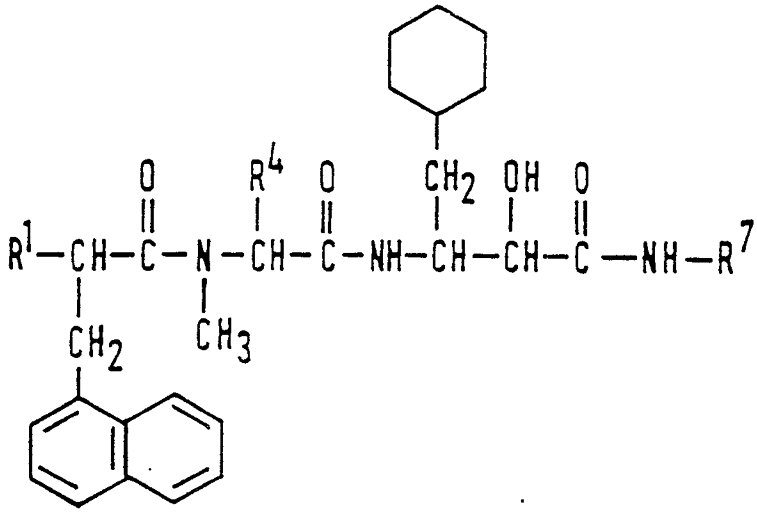

(I-10)

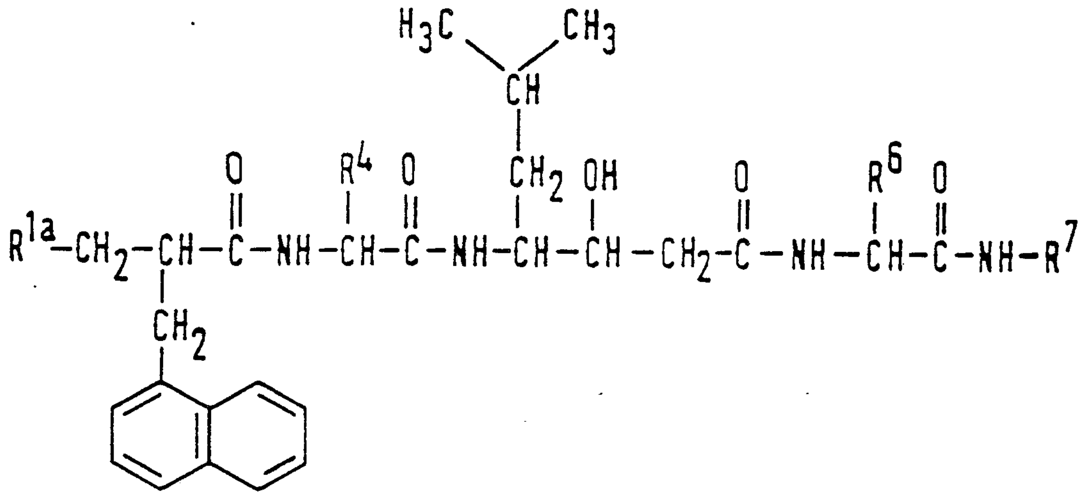

(I-11)

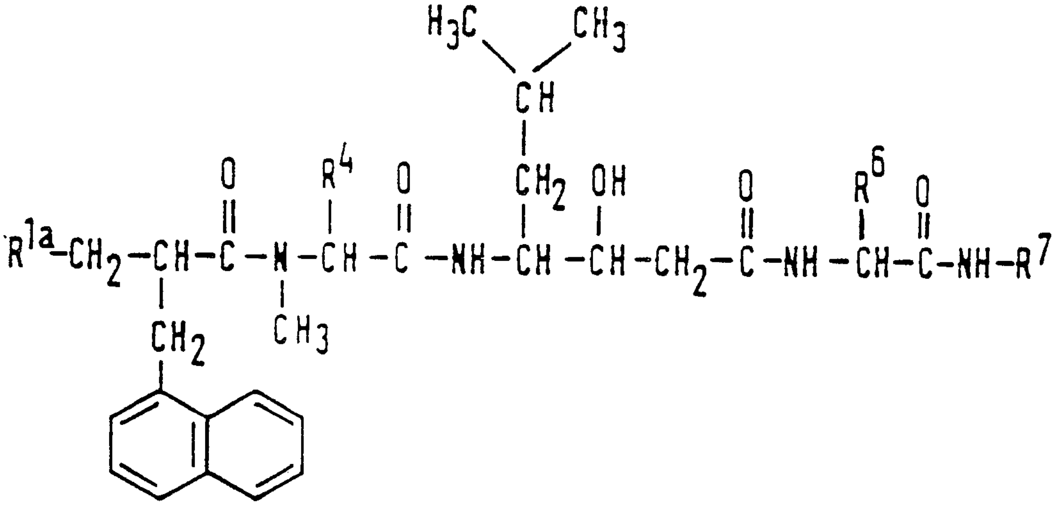

(I-12)

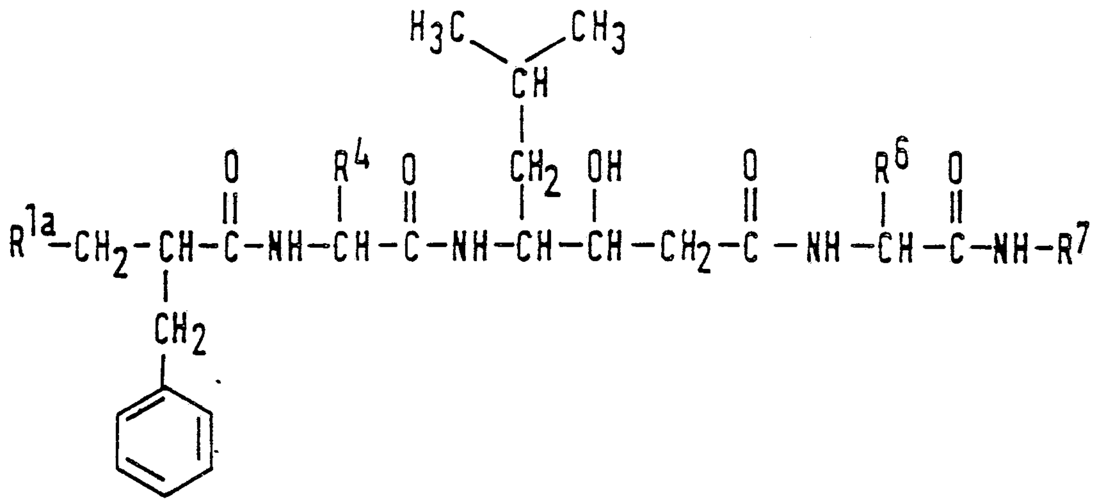

(I-13)

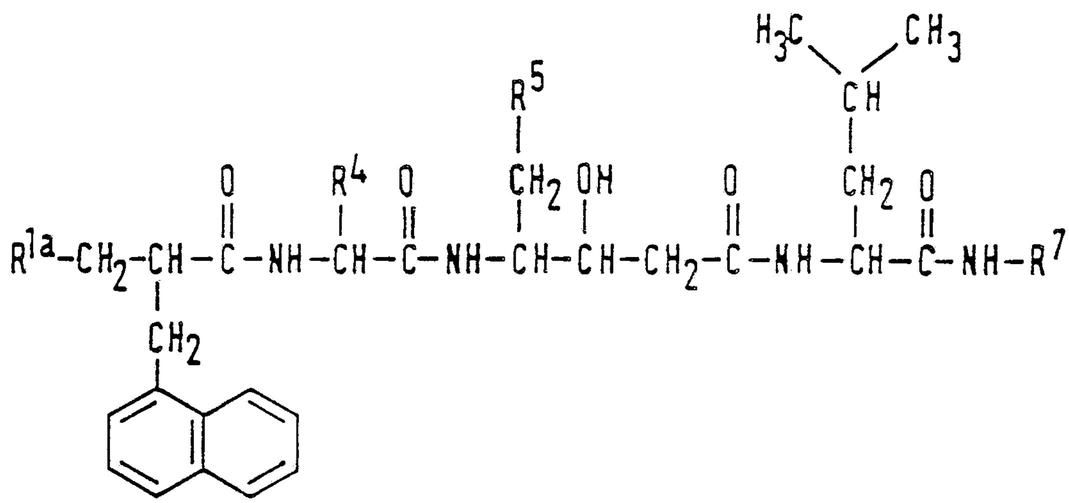

(I-14)

TABLE 1

| Cpd No. | $R^1$ | $R^4$ | $R^5$ | $R^6$ |
|---|---|---|---|---|
| 1-1 | Bz | iBu | iPr | iBu |
| 1-2 | p-MeBz | iBu | iPr | iBu |
| 1-3 | p-CℓBz | iBu | iPr | iBu |
| 1-4 | p-MeOBz | iBu | iPr | iBu |
| 1-5 | 1-NpMe | iBu | iPr | iBu |
| 1-6 | 1-NpMe | iBu | iPr | sBu |
| 1-7 | 1-NpMe | 5-ImidMe | iPr | sBu |
| 1-8 | 1-NpMe | 5-ImidMe | iPr | iBu |
| 1-9 | 1-NpMe | 5-ImidMe | iPr | iPr |
| 1-10 | 1-NpMe | 4-ThizMe | iPr | iPr |
| 1-11 | 1-NpMe | 4-ThizMe | iPr | iBu |
| 1-12 | 1-NpMe | 4-ThizMe | iPr | sBu |
| 1-13 | 1-NpMe | 2-PyrMe | iPr | sBu |
| 1-14 | 2-ThiMe | 5-ImidMe | iPr | iBu |
| 1-15 | 2-(2-MeOEtO)Et | 5-ImidMe | iPr | iBu |
| 1-16 | 2-(2-EtOEtO)Et | 5-ImidMe | iPr | iBu |
| 1-17 | 1-NpMe | iBu | Ph | iBu |
| 1-18 | 1-NpMe | 5-ImidMe | Ph | iBu |
| 1-19 | 1-NpMe | 4-ThizMe | Ph | iBu |
| 1-20 | 1-NpMe | iBu | cHx | iBu |
| 1-21 | 1-NpMe | 5-ImidMe | cHx | iBu |
| 1-22 | 1-NpMe | 4-ThizMe | cHx | iBu |

TABLE 2

| Cpd No. | $R^1$ | $R^4$ | $R^7$ |
|---|---|---|---|
| 2-1 | 3-PyrCONH- | Me | 1-Car-5-$NH_2$Pn |
| 2-2 | 3-PyrCONH- | Pr | 1-Car-5-$NH_2$Pn |
| 2-3 | 3-PyrCONH- | iBu | 1-Car-5-$NH_2$Pn |
| 2-4 | 3-PyrCONH- | iBu | 1-HOMe-5-$NH_2$Pn |
| 2-5 | 3-PyrCONH- | iBu | 1-HOMe-5-GuanPn |
| 2-6 | 3-PyrCONH- | iBu | 1-Etc-5-$NH_2$Pn |
| 2-7 | 3-PyrCONH- | iBu | 1-Car-5-GuanPn |
| 2-8 | 3-PyrCONH- | iBu | 5-HOOC-5-$NH_2$Pn |
| 2-9 | 3-PyrCONH- | iBu | 5-Car-5-$NH_2$Pn |
| 2-10 | 3-PyrCONH- | iBu | 5-Car-5-GuanPn |
| 2-11 | 3-PyrCONH- | iBu | 6-HO-5-$NH_2$Hx |
| 2-12 | 3-PyrCONH- | iBu | 5-Etc-5-$NH_2$Pn |
| 2-13 | 3-PyrCONH- | iBu | 1-Car-4-$NH_2$Bu |
| 2-14 | 3-PyrCONH- | iBu | 1-HOMe-4-$NH_2$Bu |
| 2-15 | 3-PyrCONH- | iBu | 4-Etc-4-$NH_2$Bu |
| 2-16 | 3-PyrCONH- | iBu | 4-Car-4-$NH_2$Bu |
| 2-17 | 3-PyrCONH- | iBu | 1-HOMe-4-GuanBu |
| 2-18 | 3-PyrCONH- | iBu | 1-HOOC-5-GuanPn |
| 2-19 | 3-PyrCONH- | iBu | 1-HOOC-2-$NH_2$Et |
| 2-20 | 3-PyrCONH- | iBu | 1-Car-2-$NH_2$Et |
| 2-21 | 3-PyrCONH- | iBu | m-$NH_2CH_2$Bz |
| 2-22 | 3-PyrCONH- | iPr | 1-Car-5-$NH_2$Pn |
| 2-23 | 3-PyrCONH- | iPr | 5-Car-5-$NH_2$Pn |

TABLE 2 (continued)

| Cpd No. | $R^1$ | $R^4$ | $R^7$ |
|---|---|---|---|
| 2-24 | 3-PyrCONH- | _i_Pr | 1-Car-5-GuanPn |
| 2-25 | 3-PyrCONH- | Bu | 1-Car-5-$NH_2$Pn |
| 2-26 | 3-PyrCONH- | Bu | 5-Car-5-$NH_2$Pn |
| 2-27 | 3-PyrCONH- | Bu | 2-$HSO_3$Et |
| 2-28 | 3-PyrCONH- | Bu | _m_-$NH_2CH_2$Bz |
| 2-29 | 3-PyrCONH- | 5-ImidMe | 6-HO-5-$NH_2$Hx |
| 2-30 | 3-PyrCONH- | 5-ImidMe | 5-Car-5-$NH_2$Pn |
| 2-31 | 3-PyrCONH- | 5-ImidMe | 1-Car-5-$NH_2$Pn |
| 2-32 | 3-PyrCONH- | 5-ImidMe | 1-HOMe-5-$NH_2$Pn |
| 2-33 | 3-PyrCONH- | 5-ImidMe | $H(NHCH_2CH_2)_3$- |
| 2-34 | 3-PyrCONH- | 5-ImidMe | 3-[3-$NH_2$PrN(Me)]Pr |
| 2-35 | 3-PyrCONH- | 4-ThizMe | 1-HOMe-5-$NH_2$Pn |
| 2-36 | 3-PyrCONH- | 4-ThizMe | 1-Car-5-$NH_2$Pn |
| 2-37 | 3-PyrCONH- | 4-ThizMe | 5-Car-5-$NH_2$Pn |
| 2-38 | 3-PyrCONH- | 4-ThizMe | 6-HO-5-$NH_2$Hx |
| 2-39 | 3-PyrCONH- | 4-ThizMe | $H(NHCH_2CH_2)_3$- |
| 2-40 | 3-PyrCONH- | 4-ThizMe | 3-(3-$NH_2$PrNH)Pr |
| 2-41 | 2-PyrCONH- | 4-ThizMe | 3-(3-$NH_2$PrNH)Pr |
| 2-42 | 2-PyrCONH- | 4-ThizMe | 1-Car-5-$NH_2$Pn |
| 2-43 | 2-PyrCONH- | 5-ImidMe | 1-Car-5-$NH_2$Pn |
| 2-44 | 2-PyrCONH- | 5-ImidMe | 1-HOMe-5-$NH_2$Pn |
| 2-45 | 2-PyrCONH- | 5-ImidMe | 6-HO-5-$NH_2$Hx |
| 2-46 | 2-PyrCONH- | Bu | 1-HOMe-5-$NH_2$Pn |

TABLE 2 (continued)

| Cpd No. | $R^1$ | $R^4$ | $R^7$ |
|---|---|---|---|
| 2-47 | 2-PyrCONH- | iBu | 1-Car-5-$NH_2$Pn |
| 2-48 | 2-PyrCONH- | iBu | 1-Etc-5-$NH_2$Pn |
| 2-49 | 2-PyrCONH- | iBu | 1-HOMe-5-$NH_2$Pn |
| 2-50 | 2-PyrCONH- | iBu | 1-Car-5-GuanPn |
| 2-51 | 2-PyrCONH- | iBu | 5-Car-5-$NH_2$Pn |
| 2-52 | 2-PyrCONH- | iBu | 6-HO-5-$NH_2$Hx |
| 2-53 | 2-PyrCONH- | iBu | 5-HOOC-5-$NH_2$Pn |
| 2-54 | 4-PyrCONH- | iBu | 1-Car-5-$NH_2$Pn |
| 2-55 | 4-PyrCONH- | iBu | 6-HO-5-$NH_2$Hx |
| 2-56 | 4-PyrCONH- | iBu | 5-Car-5-$NH_2$Pn |
| 2-57 | 4-PyrCONH- | 5-ImidMe | 1-HOMe-5-$NH_2$Pn |
| 2-58 | 4-PyrCONH- | 5-ImidMe | 6-HO-5-$NH_2$Hx |
| 2-59 | 4-PyrCONH- | 5-ImidMe | 3-(3-$NH_2$PrNH)Pr |
| 2-60 | 4-PyrCONH- | 4-ThizMe | 1-HOMe-5-$NH_2$Pn |
| 2-61 | 4-PyrCONH- | 4-ThizMe | 6-HO-5-$NH_2$Hx |
| 2-62 | 4-PyrCONH- | 4-ThizMe | 5-Car-5-$NH_2$Pn |
| 2-63 | 4-PyrCONH- | 4-ThizMe | m-$NH_2CH_2$Bz |
| 2-64 | 3-Pyr$CH_2$CONH- | 4-ThizMe | m-$NH_2CH_2$Bz |
| 2-65 | 3-Pyr$CH_2$CONH- | 4-ThizMe | 1-HOMe-5-$NH_2$Pn |
| 2-66 | 3-Pyr$CH_2$CONH- | 4-ThizMe | 6-HO-5-$NH_2$Hx |
| 2-67 | 3-Pyr$CH_2$CONH- | 5-ImidMe | 6-HO-5-$NH_2$Hx |
| 2-68 | 2-FurCONH- | 5-ImidMe | $H(NHCH_2CH_2)_3$- |
| 2-69 | 2-FurCONH- | 5-ImidMe | 6-HO-5-$NH_2$Hx |

TABLE 2 (continued)

| Cpd No. | $R^1$ | $R^4$ | $R^7$ |
|---|---|---|---|
| 2-70 | 2-FurCONH- | 4-ThizMe | 6-HO-5-$NH_2$Hx |
| 2-71 | 2-FurCONH- | 4-ThizMe | 3-(3-$NH_2$PrNH)Pr |
| 2-72 | 2-ThiCONH- | 4-ThizMe | 3-(3-$NH_2$PrNH)Pr |
| 2-73 | 2-ThiCONH- | 4-ThizMe | $H(NHCH_2CH_2)_3$- |
| 2-74 | 2-ThiCONH- | 4-ThizMe | m-$NH_2CH_2$Bz |
| 2-75 | 2-ThiCONH- | 4-ThizMe | 1-HOMe-5-$NH_2$Pn |
| 2-76 | 2-ThiCONH- | 5-ImidMe | 1-HOMe-5-$NH_2$Pn |
| 2-77 | 2-ThiCONH- | 5-ImidMe | 3-(3-$NH_2$PrNH)Pr |
| 2-78 | 2-ThiCONH- | 5-ImidMe | m-$NH_2CH_2$Bz |
| 2-79 | 2-PyzCONH- | iBu | m-$NH_2CH_2$Bz |
| 2-80 | 2-PyzCONH- | 5-ImidMe | 1-HOMe-5-$NH_2$Pn |
| 2-81 | 2-PyzCONH- | 5-ImidMe | 6-HO-5-$NH_2$Hx |
| 2-82 | 2-PyzCONH- | 5-ImidMe | m-$NH_2CH_2$Bz |
| 2-83 | 2-PyzCONH- | 5-ImidMe | 3-(3-$NH_2$PrNH)Pr |
| 2-84 | 2-PyzCONH- | 4-ThizMe | 1-HOMe-5-$NH_2$Pn |
| 2-85 | 2-PyzCONH- | 4-ThizMe | 6-HO-5-$NH_2$Hx |
| 2-86 | BozNH | 5-ImidMe | 1-HOMe-5-$NH_2$Pn |
| 2-87 | BozNH | 5-ImidMe | 3-(3-$NH_2$PrNH)Pr |
| 2-88 | p-MeOBozNH- | 5-ImidMe | 3-(3-$NH_2$PrNH)Pr |
| 2-89 | p-MeOBozNH- | 5-ImidMe | m-$NH_2CH_2$Bz |
| 2-90 | p-ClBozNH- | 5-ImidMe | m-$NH_2CH_2$Bz |
| 2-91 | p-ClBozNH- | 4-ThizMe | m-$NH_2CH_2$Bz |
| 2-92 | 4-PhByrNH- | 4-ThizMe | $H(NHCH_2CH_2)_3$- |

Table 2 (continued)

| Cpd No. | $R^1$ | $R^4$ | $R^7$ |
|---|---|---|---|
| 2-93 | 4-PhByrNH- | 5-ImidMe | $H(NHCH_2CH_2)_3$- |
| 2-94 | 4-PhByrNH- | 5-ImidMe | m-$NH_2CH_2$Bz |
| 2-95 | 4-PhByrNH- | 5-ImidMe | 1-HOMe-5-$NH_2$Pn |
| 2-96 | AcNH- | 5-ImidMe | 1-HOMe-5-$NH_2$Pn |
| 2-97 | AcNH- | 5-ImidMe | 5-HOOC-5-$NH_2$Pn |
| 2-98 | AcNH- | 5-ImidMe | 6-HO-5-$NH_2$Hx |
| 2-99 | AcNH- | 5-ImidMe | 3-(3-$NH_2$PrNH)Pr |
| 2-100 | AcNH- | 4-ThizMe | 6-HO-5-$NH_2$Hx |
| 2-101 | EtCONH- | 5-ImidMe | 1-HOMe-5-$NH_2$Pn |
| 2-102 | EtCONH- | 5-ImidMe | 6-HO-5-$NH_2$Hx |
| 2-103 | EtCONH- | 5-ImidMe | $H(NHCH_2CH_2)_3$- |
| 2-104 | 2-EtOEtCONH- | 5-ImidMe | $H(NHCH_2CH_2)_3$- |
| 2-105 | 2-EtOEtCONH- | 5-ImidMe | 5-Car-5-$NH_2$Pn |
| 2-106 | 2-EtOEtCONH- | 5-ImidMe | 3-(3-$NH_2$PrNH)Pr |
| 2-107 | 2-EtOEtCONH- | iBu | 6-HO-5-$NH_2$Hx |
| 2-108 | HpCONH- | 5-ImidMe | 1-HOMe-5-$NH_2$Pn |
| 2-109 | HpCONH- | 5-ImidMe | $H(NHCH_2CH_2)_3$- |
| 2-110 | HpCONH- | 5-ImidMe | 3-(3-$NH_2$PrNH)Pr |
| 2-111 | HpCONH- | 5-ImidMe | m-$NH_2CH_2$Bz |
| 2-112 | 4-Ph-1-Piz$CH_2$CONH- | 5-ImidMe | m-$NH_2CH_2$Bz |
| 2-113 | 4-Ph-1-Piz$CH_2$CONH- | 5-ImidMe | 6-HO-5-$NH_2$Hx |
| 2-114 | 1-NpMe | iBu | 1,5-diHOOC-5-$NH_2$Pn |

Table 2 (continued)

| Cpd No. | $R^1$ | $R^4$ | $R^7$ |
|---|---|---|---|
| 2-115 | 1-NpMe | iBu | 1-HOMe-5-$NH_2$Pn |
| 2-116 | 1-NpMe | iBu | 1-Car-5-$NH_2$Pn |
| 2-117 | 1-NpMe | 5-ImidMe | 1-HOMe-5-$NH_2$Pn |
| 2-118 | 1-NpMe | 5-ImidMe | 1-Car-5-$NH_2$Pn |
| 2-119 | 1-NpMe | 5-ImidMe | 6-HO-5-$NH_2$Hx |
| 2-120 | 1-NpMe | 5-ImidMe | 5-HOOC-5-$NH_2$Pn |
| 2-121 | 1-NpMe | 5-ImidMe | 5-Car-5-$NH_2$Pn |
| 2-122 | 1-NpMe | 5-ImidMe | m-$NH_2CH_2$Bz |
| 2-123 | 1-NpMe | 5-ImidMe | 2-$HSO_3$Et |
| 2-124 | 1-NpMe | 4-ThizMe | 5-HOOC-5-$NH_2$Pn |
| 2-125 | 4-PhBu | 5-ImidMe | 3-(3-$NH_2$PrNH)Pr |
| 2-126 | MeOCONH- | 5-ImidMe | 6-HO-5-$NH_2$Hx |
| 2-127 | EtcNH- | 5-ImidMe | 6-HO-5-$NH_2$Hx |
| 2-128 | EtcNH- | 5-ImidMe | 5-HOOC-5-$NH_2$Pn |
| 2-129 | EtcNH- | 5-ImidMe | 5-Car-5-$NH_2$Pn |
| 2-130 | EtcNH- | 5-ImidMe | $H(NHCH_2CH_2)_3$- |
| 2-131 | EtcNH- | 5-ImidMe | m-$NH_2CH_2$Bz |
| 2-132 | EtcNH- | 5-ImidMe | 3-(3-$NH_2$PrNH)Pr |
| 2-133 | EtcNH- | 5-ImidMe | 1-Car-5-$NH_2$Pn |
| 2-134 | EtcNH- | 5-ImidMe | 1-HOMe-5-$NH_2$Pn |
| 2-135 | PrOCONH- | 5-ImidMe | 5-Car-5-$NH_2$Pn |
| 2-136 | PrOCONH- | 5-ImidMe | 1-Car-5-$NH_2$Pn |
| 2-137 | tBocNH- | 5-ImidMe | 6-HO-5-$NH_2$Hx |

Table 2 (continued)

| Cpd No. | $R^1$ | $R^4$ | $R^7$ |
|---|---|---|---|
| 2-138 | PhOCONH- | 5-ImidMe | 6-HO-5-$NH_2$Hx |
| 2-139 | BzOCONH- | 5-ImidMe | 6-HO-5-$NH_2$Hx |
| 2-140 | BzOCONH- | 5-ImidMe | 5-HOOC-5-$NH_2$Pn |
| 2-141 | BzOCONH- | 5-ImidMe | 5-Car-5-$NH_2$Pn |
| 2-142 | BzOCONH- | 5-ImidMe | 5-Etc-5-$NH_2$Pn |
| 2-143 | BzOCONH- | 5-ImidMe | 1-HOMe-5-$NH_2$Pn |
| 2-144 | BzOCONH- | 5-ImidMe | $H(NHCH_2CH_2)_3$- |
| 2-145 | BzOCONH- | 5-ImidMe | 3-(3-$NH_2$PrNH)Pr |
| 2-146 | 3-PyrCONH- | $BzOCH_2$- | 5-HOOC-5-$NH_2$Pn |
| 2-147 | 3-PyrCONH- | 2-MeSEt | 5-HOOC-5-$NH_2$Pn |
| 2-148 | 3-PyrCONH- | 2-MeSOEt | 5-HOOC-5-$NH_2$Pn |
| 2-149 | 1-NpMe | CarMe | 3-(3-$NH_2$PrNH)Pr |
| 2-150 | 1-NpMe | 2-CarEt | 3-(3-$NH_2$PrNH)Pr |
| 2-151 | 3-PyrCONH- | Bz | 5-Etc-5-$NH_2$Pn |
| 2-152 | 3-PyrCONH- | <u>c</u>$PnCH_2$- | 5-Car-5-$NH_2$Pn |
| 2-153 | 3-PyrCONH- | <u>i</u>Bu | <u>m</u>-(2-HO-3-$NH_2$PrO)Bz |
| 2-154 | 3-PyrCONH- | <u>i</u>Bu | <u>m</u>-(2-HO-3-PrNHPrO)Bz |
| 2-155 | 3-PyrCONH- | <u>i</u>Bu | <u>m</u>-(2-HO-3-<u>i</u>PrNHPrO)Bz |
| 2-156 | 3-PyrCONH- | <u>i</u>Bu | <u>m</u>-(2-HO-3-<u>t</u>BuNHPrO)Bz |
| 2-157 | 3-PyrCONH- | <u>i</u>Bu | <u>p</u>-(2-HO-3-$NH_2$PrO)Bz |
| 2-158 | 3-PyrCONH- | <u>i</u>Bu | <u>m</u>-[2-HO-3-(4-MePiz)PrO]Bz |
| 2-159 | 3-PyrCONH- | <u>i</u>Bu | <u>p</u>-[2-HO-3-(2-MorEtNH)PrO]Bz |

Table 2 (continued)

| Cpd No. | $R^1$ | $R^4$ | $R^7$ |
|---|---|---|---|
| 2-160 | 3-PyrCONH- | 5-ImidMe | m-(2-HO-3-$NH_2$PrO)Bz |
| 2-161 | 3-PyrCONH- | 5-ImidMe | p-(2-HO-3-$NH_2$PrO)Bz |
| 2-162 | 3-PyrCONH- | 5-ImidMe | m-(2-HO-3-PrNHPrO)Bz |
| 2-163 | 3-PyrCONH- | 5-ImidMe | m-(2-HO-3-tBuNHPrO)Bz |
| 2-164 | 3-PyrCONH- | 5-ImidMe | m-(2-HO-3-MorPrO)Bz |
| 2-165 | 3-PyrCONH- | 5-ImidMe | p-[2-HO-3-(4-MePiz)PrO]Bz |
| 2-166 | 3-PyrCONH- | 4-ThizMe | m-(2-HO-3-$NH_2$PrO)Bz |
| 2-167 | 3-PyrCONH- | 4-ThizMe | p-(2-HO-3-$NH_2$PrO)Bz |
| 2-168 | 3-PyrCONH- | 4-ThizMe | m-(2-HO-3-iPrNHPrO)Bz |
| 2-169 | 3-PyrCONH- | 4-ThizMe | m-(2-HO-3-tBuNHPrO)Bz |
| 2-170 | 3-PyrCONH- | 4-ThizMe | p-(2-HO-3-tBuNHPrO)Bz |
| 2-171 | 3-PyrCONH- | 4-ThizMe | m-[2-HO-3-(4-MePiz)PrO]Bz |
| 2-172 | EtCONH- | 4-ThizMe | 2-HO-3-$NH_2$Pr |
| 2-173 | EtCONH- | 5-ImidMe | 2-HO-3-$NH_2$Pr |
| 2-174 | 3-PyrCONH- | iBu | 2-HO-3-$NH_2$Pr |
| 2-175 | 3-PyrCONH- | 5-ImidMe | 2-HO-3-$NH_2$Pr |
| 2-176 | tBocNH- | 5-ImidMe | 1-Car-5-$NH_2$Pn |
| 2-177 | tBocNH- | 5-ImidMe | 5-Car-5-$NH_2$Pn |
| 2-178 | tBocNH- | 5-ImidMe | 5-HOOC-5-$NH_2$Pn |
| 2-179 | tBocNH- | 5-ImidMe | 2-HO-3-$NH_2$Pr |
| 2-180 | tBocNH- | 4-ThizMe | 5-Car-5-$NH_2$Pn |
| 2-181 | tBocNH- | 4-ThizMe | 6-HO-5-$NH_2$Hx |
| 2-182 | tBocNH- | 4-ThizMe | 2-HO-3-$NH_2$Pr |

Table 2 (continued)

| Cpd No. | $R^1$ | $R^4$ | $R^7$ |
|---|---|---|---|
| 2-183 | tBocNH- | iBu | 2-HO-3-$NH_2$Pr |
| 2-184 | tBocNH- | iBu | 6-HO-5-$NH_2$-Hx |
| 2-185 | tBocNH- | iBu | 5-Car-5-$NH_2$Pn |
| 2-186 | 3-PyrCONH- | 2-CarEt | 1-Car-5-$NH_2$Pn |
| 2-187 | 3-PyrCONH- | 2-CarEt | 1-HOMe-5-$NH_2$Pn |
| 2-188 | 3-PyrCONH- | 5-ImidMe | 5-MeOCO-5-$NH_2$Pn |
| 2-189 | 3-PyrCONH- | 4-ThizMe | 6-HO-5-$NH_2$Hx |
| 2-190 | 3-PyrCONH- | 4-ThizMe | 5-HOOC-5-$NH_2$Pn |
| 2-191 | 3-PyrCONH- | 4-ThizMe | 1-HOOC-5-$NH_2$Pn |
| 2-192 | 3-PyrCONH- | 4-ThizMe | 1-Car-5-GuanPn |
| 2-193 | 3-PyrCONH- | 5-IsoxMe | 5-HOOC-5-$NH_2$Pn |
| 2-194 | 3-PyrCONH- | 5-IsoxMe | 5-Car-5-$NH_2$Pn |
| 2-195 | 3-PyrCONH- | 5-IsoxMe | 5-Etc-5-$NH_2$Pn |
| 2-196 | 3-PyrCONH- | 5-IsoxMe | 1-Car-5-$NH_2$Pn |
| 2-197 | 3-PyrCONH- | 5-IsoxMe | 1-HOMe-5-$NH_2$Pn |
| 2-198 | 2-PyrCONH- | 4-ThizMe | 5-Car-5-$NH_2$Pn |
| 2-199 | 2-PyrCONH- | 4-ThizMe | 5-HOOC-5-$NH_2$Pn |
| 2-200 | 4-PyrCONH- | 5-ImidMe | 5-HOOC-5-$NH_2$Pn |
| 2-201 | 4-PyrCONH- | 5-ImidMe | 5-Car-5-$NH_2$Pn |
| 2-202 | AcNH- | 4-ThizMe | 5-HOOC-5-$NH_2$Pn |
| 2-203 | AcNH- | 4-ThizMe | 5-Car-5-$NH_2$Pn |
| 2-204 | AcNH- | 4-ThizMe | 1-Car-5-$NH_2$Pn |
| 2-205 | AcNH- | 5-ImidMe | 1-Car-5-$NH_2$Pn |

Table 2 (continued)

| Cpd No. | $R^1$ | $R^4$ | $R^7$ |
|---|---|---|---|
| 2-206 | AcNH- | 5-ImidMe | 5-Car-5-$NH_2$Pn |
| 2-207 | AcNH- | 5-IsoxMe | 5-Car-5-$NH_2$Pn |
| 2-208 | AcNH- | 5-IsoxMe | 1-Car-5-$NH_2$Pn |
| 2-209 | BzOCONH- | 5-IsoxMe | 1-Car-5-$NH_2$Pn |
| 2-210 | BzOCONH- | 5-IsoxMe | 5-Car-5-$NH_2$Pn |
| 2-211 | BzOCONH- | 5-ImidMe | 5-Etc-5-tBocNHPn |
| 2-212 | BzOCONH- | 5-ImidMe | 1-Car-5-$NH_2$Pn |
| 2-213 | BzOCONH- | 4-ThizMe | 1-Car-5-$NH_2$Pn |
| 2-214 | BzOCONH- | 4-ThizMe | 5-Car-5-$NH_2$Pn |
| 2-215 | BzOCONH- | 4-ThizMe | 5-HOOC-5-$NH_2$Pn |
| 2-216 | BzOCONH- | iBu | 5-Car-5-$NH_2$Pn |
| 2-217 | BzOCONH- | sBu | 5-HOOC-5-$NH_2$Pn |
| 2-218 | 2-(2-MeOEtO)-EtcNH- | 5-ImidMe | 5-Car-5-$NH_2$Pn |
| 2-219 | 2-(2-MeOEtO)-EtcNH- | 4-ThizMe | 5-Car-5-$NH_2$Pn |
| 2-220 | 2-(2-MeOEtO)-EtcNH- | iBu | 1-HOMe-5-$NH_2$Pn |
| 2-221 | 2-(2-MeOEtO)-EtcNH- | iBu | 1-Car-5-$NH_2$Pn |
| 2-222 | o-HOBozNH- | 5-ImidMe | 1-Car-5-$NH_2$Pn |
| 2-223 | o-HOBozNH- | 4-ThizMe | 1-Car-5-$NH_2$Pn |
| 2-224 | o-AcOBozNH- | 4-ThizMe | 1-Car-5-$NH_2$Pn |

Table 2 (continued)

| Cpd No. | $R^1$ | $R^4$ | $R^7$ |
|---|---|---|---|
| 2-225 | o-AcOBozNH- | 4-ThizMe | 5-Car-5-$NH_2$Pn |
| 2-226 | o,m,diHOBozNH- | 4-ThizMe | 5-Car-5-$NH_2$Pn |
| 2-227 | o,m,diAcOBozNH- | 4-ThizMe | 5-Car-5-$NH_2$Pn |
| 2-228 | 5-$NH_2$-2-$NO_2$BozNH- | 5-ImidMe | 5-MeOCO-5-$NH_2$Pn |
| 2-229 | 5-$NH_2$-2-$NO_2$BozNH- | 5-ImidMe | 5-HOOC-5-$NH_2$Pn |
| 2-230 | MorAcNH- | iBu | 1-HOMe-5-$NH_2$Pn |
| 2-231 | MorAcNH- | 2-CarEt | 1-Car-5-$NH_2$Pn |
| 2-232 | MorAcNH- | 2-CarEt | 1-HOMe-5-$NH_2$Pn |
| 2-233 | MorAcNH- | 4-ThizMe | 6-HO-5-$NH_2$Hx |
| 2-234 | 1-NpMe | iBu | 1-HOOC-5-$NH_2$Pn |
| 2-235 | 1-NpMe | iBu | 5-HOOC-5-$NH_2$Pn |
| 2-236 | 1-NpMe | sBu | 5-HOOC-5-$NH_2$Pn |
| 2-237 | 1-NpMe | sBu | 1-HOOC-5-$NH_2$Pn |
| 2-238 | 1-NpMe | Bu | $H(NHCH_2CH_2)_3$- |
| 2-239 | 1-NpMe | 2-CarEt | 1-HOMe-5-$NH_2$Pn |
| 2-240 | 1-NpMe | 5-ImidMe | 2-(2-$NH_2$EtNH)Et |
| 2-241 | 1-NpMe | 4-ThizMe | 6-HO-5-$NH_2$Hx |
| 2-242 | 4-PhBu | iBu | 5-HOOC-5-$NH_2$Pn |
| 2-243 | 1-tBoc-2-PyrdCONH- | 4-ThizMe | 1-HOOC-5-$NH_2$Pn |

Table 2 (continued)

| Cpd No. | $R^1$ | $R^4$ | $R^7$ |
|---|---|---|---|
| 2-244 | 1-tBoc-2-PyrdCONH- | 4-ThizMe | 1-HOMe-5-$NH_2$Pn |
| 2-245 | 1-tBoc-2-PyrdCONH- | 4-ThizMe | 1-Car-5-$NH_2$Pn |
| 2-246 | 1-tBoc-2-PyrdCONH- | 4-ThizMe | 5-HOOC-5-$NH_2$Pn |
| 2-247 | 1-tBoc-2-PyrdCONH- | 4-ThizMe | 5-Etc-5-$NH_2$Pn |
| 2-248 | 1-tBoc-2-PyrdCONH- | 4-ThizMe | 5-Car-5-$NH_2$Pn |
| 2-249 | 1-tBoc-2-PyrdCONH- | 4-ThizMe | 6-HO-5-$NH_2$Hx |
| 2-250 | 1-tBoc-2-PyrdCONH- | 5-ImidMe | 1-Car-5-$NH_2$Pn |
| 2-251 | 1-tBoc-2-PyrdCONH- | 5-ImidMe | 1-HOMe-5-$NH_2$Pn |
| 2-252 | 1-tBoc-2-PyrdCONH- | 5-ImidMe | 5-Car-5-$NH_2$Pn |
| 2-253 | 1-tBoc-2-PyrdCONH- | 5-ImidMe | 5-HOOC-5-$NH_2$Pn |
| 2-254 | 1-tBoc-2-PyrdCONH- | 5-ImidMe | 5-Etc-5-$NH_2$Pn |

Table 2 (continued)

| Cpd No. | $R^1$ | $R^4$ | $R^7$ |
|---|---|---|---|
| 2-255 | 1-tBoc-2-PyrdCONH- | 5-IsoxMe | 5-Etc-5-$NH_2$Pn |
| 2-256 | 1-tBoc-2-PyrdCONH- | 5-IsoxMe | 5-Car-5-$NH_2$Pn |
| 2-257 | 1-tBoc-2-PyrdCONH- | 5-IsoxMe | 1-Car-5-$NH_2$Pn |
| 2-258 | 1-tBoc-2-PyrdCONH- | 3-IndMe | 1-Car-5-$NH_2$Pn |
| 2-259 | 1-tBoc-2-PyrdCONH- | 3-IndMe | 5-Car-5-$NH_2$Pn |
| 2-260 | 1-BzOCO-2-PyrdCONH- | 4-ThizMe | 1-Car-5-$NH_2$Pn |
| 2-261 | 1-BzOCO-2-PyrdCONH- | 4-ThizMe | 5-HOOC-5-$NH_2$Pn |
| 2-262 | 1-BzOCO-2-PyrdCONH- | 5-ImidMe | 5-HOOC-5-$NH_2$Pn |
| 2-263 | 1-BzOCO-2-PyrdCONH- | 5-ImidMe | 5-Car-5-$NH_2$Pn |
| 2-264 | 1-BzOCO-2-PyrdCONH- | 5-IsoxMe | 5-Car-5-$NH_2$Pn |
| 2-265 | tBuCOMe | 4-ThizMe | 5-Car-5-$NH_2$Pn |
| 2-266 | tBuCOMe | 4-ThizMe | 5-HOOC-5-$NH_2$Pn |
| 2-267 | tBuCOMe | 4-ThizMe | 1-Car-5-$NH_2$Pn |

Table 2 (continued)

| Cpd No. | $R^1$ | $R^4$ | $R^7$ |
|---|---|---|---|
| 2-268 | tBuCOMe | 5-ImidMe | 1-Car-5-$NH_2$Pn |
| 2-269 | tBuCOMe | 5-ImidMe | 5-Car-5-$NH_2$Pn |
| 2-270 | tBuCOMe | 5-ImidMe | 5-HOOC-5-$NH_2$Pn |
| 2-271 | tBuCOMe | 5-IsoxMe | 5-HOOC-5-$NH_2$Pn |
| 2-272 | tBuCOMe | 5-IsoxMe | 5-Car-5-$NH_2$Pn |
| 2-273 | tBuCOMe | 5-IsoxMe | 1-Car-5-$NH_2$Pn |
| 2-274 | EtNHCO- | 5-IsoxMe | 1-Car-5-$NH_2$Pn |
| 2-275 | EtNHCO- | 4-ThizMe | 1-Car-5-$NH_2$Pn |
| 2-276 | EtNHCO- | 4-ThizMe | 5-Car-5-$NH_2$Pn |
| 2-277 | EtNHCO- | 4-ThizMe | 5-HOOC-5-$NH_2$Pn |
| 2-278 | EtNHCO- | 5-ImidMe | 5-HOOC-5-$NH_2$Pn |
| 2-279 | EtNHCO- | 5-ImidMe | 5-Car-5-$NH_2$Pn |
| 2-280 | EtNHCO- | 5-ImidMe | 1-Car-5-$NH_2$Pn |
| 2-281 | tBuNHCO- | 5-ImidMe | 1-Car-5-$NH_2$Pn |
| 2-282 | tBuNHCO- | 4-ThizMe | 1-Car-5-$NH_2$Pn |
| 2-283 | tBuNHCO- | 4-ThizMe | 5-Car-5-$NH_2$Pn |
| 2-284 | $HOOCCH_2NHCO$- | 5-ImidMe | 5-Car-5-$NH_2$Pn |
| 2-285 | $HOOCCH_2NHCO$- | 4-ThizMe | 5-Car-5-$NH_2$Pn |
| 2-286 | $HOOCCH_2NHCO$- | 4-ThizMe | 1-Car-5-$NH_2$Pn |
| 2-287 | $HOOCCH_2NHCO$- | iBu | 1-Car-5-$NH_2$Pn |
| 2-288 | MorCOMe | 5-ImidMe | 1-Car-5-$NH_2$Pn |
| 2-289 | MorCOMe | 5-ImidMe | 5-Car-5-$NH_2$Pn |
| 2-290 | MorCOMe | 4-ThizMe | 5-Car-5-$NH_2$Pn |

Table 2 (continued)

| Cpd No. | $R^1$ | $R^4$ | $R^7$ |
|---|---|---|---|
| 2-291 | MorCOMe | 4-ThizMe | 1-Car-5-$NH_2$Pn |
| 2-292 | 3-PyrMe | 4-ThizMe | 1-Car-5-$NH_2$Pn |
| 2-293 | 3-PyrMe | 4-ThizMe | 5-Car-5-$NH_2$Pn |
| 2-294 | 3-PyrMe | 4-ThizMe | 5-HOOC-5-$NH_2$Pn |
| 2-295 | 3-PyrMe | 5-ImidMe | 5-HOOC-5-$NH_2$Pn |
| 2-296 | 3-PyrMe | 5-ImidMe | 5-Car-5-$NH_2$Pn |
| 2-297 | t-BocNH- | 4-ThizMe | 5-HOOC-5-$NH_2$Pn |
| 2-298 | t-BocNH- | 4-ThizMe | m-$NH_2CH_2$Bz |
| 2-299 | t-BocNH- | 5-ImidMe | m-$NH_2CH_2$Bz |
| 2-300 | 3-PyrCONH- | 5-ImidMe | m-$NH_2CH_2$Bz |
| 2-301 | 1-tBoc-2-PyrdCONH- | 4-ThizMe | m-$NH_2CH_2$Bz |
| 2-302 | 1-tBoc-2-PyrdCONH- | 5-ImidMe | m-$NH_2CH_2$Bz |
| 2-303 | AcNH- | 5-ImidMe | m-$NH_2CH_2$Bz |
| 2-304 | AcNH- | 4-ThizMe | m-$NH_2CH_2$Bz |
| 2-305 | 3-PyrCONH- | 5-ImidMe | 5-HOOC-5-$NH_2$Pn |
| 2-306 | 3-PyrCONH- | 4-ThizMe | 1-Car-4-$NH_2$Bu |
| 2-307 | 4-PyrCONH- | 5-ImidMe | 1-Car-4-$NH_2$Bu |
| 2-308 | tBocNH- | 5-IsoxMe | 5-Car-5-$NH_2$Pn |
| 2-309 | tBocNH- | 5-IsoxMe | 5-HOOC-5-$NH_2$Pn |
| 2-310 | tBocNH- | 5-IsoxMe | 1-Car-4-$NH_2$Bu |
| 2-311 | 3-PyrCONH- | 4-ThizMe | m-$NH_2CH_2$Bz |
| 3-312 | 1-NpMe | 5-IsoxMe | m-$NH_2CH_2$Bz |

Table 3

| Cpd No. | $R^1$ | $R^4$ | $R^7$ |
| --- | --- | --- | --- |
| 3-1 | AcNH- | 5-ImidMe | 1-HOMe-5-$NH_2$Pn |
| 3-2 | EtCONH- | 5-ImidMe | 5-Car-5-$NH_2$Pn |
| 3-3 | EtCONH- | 5-ImidMe | 6-HO-5-$NH_2$Hx |
| 3-4 | EtCONH- | 5-ImidMe | 2-HO-3-$NH_2$Pr |
| 3-5 | EtCONH- | 4-ThizMe | 2-HO-3-$NH_2$Pr |
| 3-6 | EtCONH- | 4-ThizMe | 6-HO-5-$NH_2$Hx |
| 3-7 | EtCONH- | 4-ThizMe | 5-Car-5-$NH_2$Pn |
| 3-8 | EtCONH- | 4-ThizMe | 5-HOOC-5-$NH_2$Pn |

Table 4

| Cpd No. | $R^1$ | $R^4$ | $R^7$ |
| --- | --- | --- | --- |
| 4-1 | 3-PyrCONH | iBu | 1-Car-5-$NH_2$Pn |
| 4-2 | AcNH- | 5-ImidMe | 1-HOMe-5-$NH_2$Pn |
| 4-3 | 3-PyrCONH- | 5-ImidMe | 6-HO-5-$NH_2$Hx |
| 4-4 | 3-PyrCONH- | 5-ImidMe | 5-Car-5-$NH_2$Pn |
| 4-5 | 3-PyrCONH- | i-Bu | 5-Car-5-$NH_2$Pn |
| 4-6 | 3-PyrCONH- | i-Bu | 2-HO-3-$NH_2$Pr |
| 4-7 | tBocNH- | i-Bu | 6-HO-5-$NH_2$Hx |
| 4-8 | tBocNH- | i-Bu | 5-Car-5-$NH_2$Pn |

Table 4 (continued)

| Cpd No. | $R^1$ | $R^4$ | $R^7$ |
|---|---|---|---|
| 4-9 | 3-PyrCONH- | 5-ImidMe | 1-Car-5-$NH_2$Pn |
| 4-10 | 3-PyrCONH- | 5-ImidMe | 5-HOOC-5-$NH_2$Pn |
| 4-11 | 3-PyrCONH- | 4-ThizMe | 5-HOOC-5-$NH_2$Pn |
| 4-12 | 3-PyrCONH- | 4-ThizMe | 5-Car-5-$NH_2$Pn |
| 4-13 | 3-PyrCONH- | 4-ThizMe | 5-Etc-5-$NH_2$Pn |
| 4-14 | 3-PyrCONH- | 4-ThizMe | 1-Car-5-$NH_2$Pn |
| 4-15 | 3-PyrCONH- | 5-IsoxMe | 1-Car-5-$NH_2$Pn |
| 4-16 | 3-PyrCONH- | 5-IsoxMe | 5-Car-5-$NH_2$Pn |
| 4-17 | 3-PyrCONH- | 5-IsoxMe | 5-HOOC-5-$NH_2$Pn |
| 4-18 | tBocNH- | 5-IsoxMe | 5-HOOC-5-$NH_2$Pn |
| 4-19 | tBocNH- | 5-IsoxMe | 5-Car-5-$NH_2$Pn |
| 4-20 | tBocNH- | 5-ImidMe | 5-Car-5-$NH_2$Pn |
| 4-21 | tBocNH- | 5-ImidMe | 5-Etc-5-$NH_2$Pn |
| 4-22 | tBocNH- | 5-IsoxMe | 5-Etc-5-$NH_2$Pn |
| 4-23 | tBocNH- | 5-IsoxMe | 5-Car-5-$NH_2$Pn |
| 4-24 | BzOCONH- | 4-ThizMe | 5-Car-5-$NH_2$Pn |
| 4-25 | BzOCONH- | 5-ImidMe | 5-Car-5-$NH_2$Pn |
| 4-26 | AcNH- | 5-ImidMe | 1-Car-5-$NH_2$Pn |
| 4-27 | AcNH- | 5-ImidMe | 5-Car-5-$NH_2$Pn |
| 4-28 | AcNH- | 5-ImidMe | 5-Etc-5-$NH_2$Pn |
| 4-29 | AcNH- | 4-ThizMe | 5-Etc-5-$NH_2$Pn |
| 4-30 | AcNH- | 4-ThizMe | 5-HOOC-5-$NH_2$Pn |
| 4-31 | AcNH- | 4-ThizMe | 5-Car-5-$NH_2$Pn |

Table 4 (continued)

| Cpd No. | $R^1$ | $R^4$ | $R^7$ |
|---|---|---|---|
| 4-32 | AcNH- | 4-ThizMe | 1-Car-5-$NH_2$Pn |
| 4-33 | 1-tBoc-2-PyrdCONH- | 4-ThizMe | 1-Car-5-$NH_2$Pn |
| 4-34 | 1-tBoc-2-PyrdCONH- | 4-ThizMe | 5-HOOC-5-$NH_2$Pn |
| 4-35 | 1-tBoc-2-PyrdCONH- | 4-ThizMe | 5-Car-5-$NH_2$Pn |
| 4-36 | 1-tBoc-2-PyrdCONH- | 4-ThizMe | 5-Etc-5-$NH_2$Pn |
| 4-37 | 1-tBoc-2-PyrdCONH- | 5-ImidMe | 5-Etc-5-$NH_2$Pn |
| 4-38 | 1-tBoc-2-PyrdCONH- | 5-ImidMe | 5-Car-5-$NH_2$Pn |
| 4-39 | 1-tBoc-2-PyrdCONH- | 5-ImidMe | 5-HOOC-5-$NH_2$Pn |
| 4-40 | 1-tBoc-2-PyrdCONH- | 5-ImidMe | 1-Car-5-$NH_2$Pn |
| 4-41 | 2-PyrdCONH- | 4-ThizMe | 5-HOOC-5-$NH_2$Pn |
| 4-42 | 2-PyrdCONH- | 4-ThizMe | 5-Car-5-$NH_2$Pn |
| 4-43 | tBuCOMe | 4-ThizMe | 5-HOOC-5-$NH_2$Pn |
| 4-44 | tBuCOMe | 4-ThizMe | 5-Car-5-$NH_2$Pn |
| 4-45 | tBuCOMe | 4-ThizMe | 1-Car-5-$NH_2$Pn |
| 4-46 | tBuCOMe | 5-ImidMe | 1-Car-5-$NH_2$Pn |

Table 4 (continued)

| Cpd No. | $R^1$ | $R^4$ | $R^7$ |
|---|---|---|---|
| 4-47 | tBuCOMe | 5-ImidMe | 5-Car-5-$NH_2$Pn |
| 4-48 | tBuCOMe | 5-ImidMe | 5-HOOC-5-$NH_2$Pn |
| 4-49 | EtNHCO- | 5-ImidMe | 5-HOOC-5-$NH_2$Pn |
| 4-50 | EtNHCO- | 5-ImidMe | 5-Car-5-$NH_2$Pn |
| 4-51 | EtNHCO- | 5-ImidMe | 1-Car-5-$NH_2$Pn |
| 4-52 | tBuNHCO- | 5-ImidMe | 1-Car-5-$NH_2$Pn |
| 4-53 | tBuNHCO- | 5-ImidMe | 5-Car-5-$NH_2$Pn |
| 4-54 | tBuNHCO- | 4-ThizMe | 5-Car-5-$NH_2$Pn |
| 4-55 | tBuNHCO- | 4-ThizMe | 1-Car-5-$NH_2$Pn |
| 4-56 | tBuNHCO- | 4-ThizMe | m-$NH_2CH_2$Bz |
| 4-57 | tBuNHCO- | 5-ImidMe | m-$NH_2CH_2$Bz |
| 4-58 | 3-PyrCONH- | 5-ImidMe | m-$NH_2CH_2$Bz |
| 4-59 | 3-PyrCONH- | 4-ThizMe | m-$NH_2CH_2$Bz |
| 4-60 | 3-PyrCONH- | 4-ThizMe | 6-HO-5-$NH_2$Hx |

Table 5

| Cpd No. | $R^1$ | $R^4$ | $R^7$ |
|---|---|---|---|
| 5-1 | 3-PyrCONH- | 5-ImidMe | 1-Car-5-$NH_2$Pn |
| 5-2 | 3-PyrCONH- | 5-ImidMe | 5-Etc-5-$NH_2$Pn |
| 5-3 | 3-PyrCONH- | 5-ImidMe | 5-Car-5-$NH_2$Pn |
| 5-4 | 3-PyrCONH- | 5-ImidMe | 5-HOOC-5-$NH_2$Pn |

Table 5 (continued)

| Cpd No. | $R^1$ | $R^4$ | $R^7$ |
|---|---|---|---|
| 5-5 | 3-PyrCONH- | 4-ThizMe | 5-HOOC-5-$NH_2$Pn |
| 5-6 | 3-PyrCONH- | 4-ThizMe | 5-Car-5-$NH_2$Pn |
| 5-7 | 3-PyrCONH- | 4-ThizMe | 1-Car-5-$NH_2$Pn |
| 5-8 | AcNH- | 4-ThizMe | 1-Car-5-$NH_2$Pn |
| 5-9 | AcNH- | 4-ThizMe | 5-Car-5-$NH_2$Pn |
| 5-10 | AcNH- | 4-ThizMe | 5-HOOC-5-$NH_2$Pn |
| 5-11 | 1-tBoc-2-PyrdCONH- | 4-ThizMe | 5-HOOC-5-$NH_2$Pn |
| 5-12 | 1-tBoc-2-PyrdCONH- | 4-ThizMe | 5-Car-5-$NH_2$Pn |
| 5-13 | 1-tBoc-2-PyrdCONH- | 5-ImidMe | 1-Car-5-$NH_2$Pn |
| 5-14 | tBocNH- | 5-ImidMe | 5-Car-5-$NH_2$Pn |
| 5-15 | tBocNH- | 5-ImidMe | 5-HOOC-5-$NH_2$Pn |
| 5-16 | tBocNH- | 4-ThizMe | 5-HOOC-5-$NH_2$Pn |
| 5-17 | tBocNH- | 4-ThizMe | 5-Car-5-$NH_2$Pn |
| 5-18 | tBocNH- | 4-ThizMe | m-$NH_2CH_2$Bz |
| 5-19 | 3-PyrCONH | 4-ThizMe | m-$NH_2CH_2$Bz |
| 5-20 | 3-PyrCONH | iBu | 6-HO-5-$NH_2$Hx |
| 5-21 | tBocNH- | iBu | 1-HOMe-5-$NH_2$Pn |
| 5-22 | 1-tBoc-2-PyrdCONH- | 4-ThizMe | 1-Car-5-$NH_2$Pn |
| 5-23 | 3-PyrCONH- | 4-ThizMe | 1-Car-5-NH(tBoc)-Pn |
| 5-24 | 3-PyrCONH- | 4-ThizMe | 5-Car-5-NH(COOBz)-Pn |

Table 6

| Cpd No. | $R^1$ | $R^4$ | $R^7$ |
|---|---|---|---|
| 6-1 | 3-PyrCONH- | 5-ImidMe | 1-Car-5-$NH_2$Pn |
| 6-2 | 3-PyrCONH- | 5-ImidMe | 5-Car-5-$NH_2$Pn |
| 6-3 | 3-PyrCONH- | 5-ImidMe | 5-HOOC-5-$NH_2$Pn |
| 6-4 | 3-PyrCONH- | 4-ThizMe | 5-HOOC-5-$NH_2$Pn |
| 6-5 | 3-PyrCONH- | 4-ThizMe | 5-Car-5-$NH_2$Pn |
| 6-6 | 3-PyrCONH- | 4-ThizMe | 1-Car-5-$NH_2$Pn |
| 6-7 | AcNH- | 4-ThizMe | 1-Car-5-$NH_2$Pn |
| 6-8 | AcNH- | 4-ThizMe | 5-Car-5-$NH_2$Pn |
| 6-9 | _t_BocNH- | 4-ThizMe | 5-Car-5-$NH_2$Pn |
| 6-10 | _t_BocNH- | 4-ThizMe | 5-HOOC-5-$NH_2$Pn |
| 6-11 | _t_BocNH- | 4-ThizMe | 1-Car-5-$NH_2$Pn |
| 6-12 | _t_BocNH- | 5-ImidMe | 5-Car-5-$NH_2$Pn |
| 6-13 | _t_BocNH- | 5-ImidMe | 5-Etc-5-$NH_2$Pn |
| 6-14 | _t_BocNH- | 5-ImidMe | 5-HOOC-5-$NH_2$Pn |
| 6-15 | 1-_t_Boc-2-PyrCONH- | 5-ImidMe | 5-Car-5-$NH_2$Pn |
| 6-16 | 1-_t_Boc-2-PyrCONH- | 5-ImidMe | 5-HOOC-5-$NH_2$Pn |
| 6-17 | 1-_t_Boc-2-PyrCONH- | 4-ThizMe | 5-Car-5-$NH_2$Pn |
| 6-18 | _t_BuCOMe | 4-ThizMe | 5-Car-5-$NH_2$Pn |
| 6-19 | _t_BuCOMe | 5-ImidMe | 5-Car-5-$NH_2$Pn |
| 6-20 | EtNHCO- | 5-ImidMe | 5-Car-5-$NH_2$Pn |

Table 6 (continued)

| Cpd No. | $R^1$ | $R^4$ | $R^7$ |
|---|---|---|---|
| 6-21 | EtNHCO- | 4-ThizMe | 5-Car-5-$NH_2$Pn |
| 6-22 | EtNHCO- | 4-ThizMe | 5-HOOC-5-$NH_2$Pn |
| 6-23 | EtNHCO- | 4-ThizMe | 1-Car-5-$NH_2$Pn |
| 6-24 | tBuNHCO- | 4-ThizMe | 5-Car-5-$NH_2$Pn |
| 6-25 | tBuNHCO- | 4-ThizMe | 5-HOOC-5-$NH_2$Pn |
| 6-26 | tBocNH- | 4-ThizMe | m-$NH_2CH_2$Bz |
| 6-27 | tBocNH- | 5-ImidMe | m-$NH_2CH_2$Bz |
| 6-28 | 3-PyrCONH- | 5-ImidMe | m-$NH_2CH_2$Bz |
| 6-29 | 3-PyrCONH- | 4-ThizMe | m-$NH_2CH_2$Bz |
| 6-30 | 1-tBoc-2-PyrdCONH- | 4-ThizMe | m-$NH_2CH_2$Bz |
| 6-31 | 1-tBoc-2-PyrdCONH- | 5-ImidMe | m-$NH_2CH_2$Bz |

Table 7

| Cpd No. | $R^1$ | $R^4$ | $R^7$ |
|---|---|---|---|
| 7-1 | 3-PyrCONH- | 5-ImidMe | 5-Car-5-$NH_2$Pn |
| 7-2 | 3-PyrCONH- | 5-ImidMe | 5-HOOC-5-$NH_2$Pn |
| 7-3 | 3-PyrCONH- | 4-ThizMe | 5-HOOC-5-$NH_2$Pn |

Table 7 (continued)

| Cpd No. | $R^1$ | $R^4$ | $R^7$ |
|---|---|---|---|
| 7-4 | 3-PyrCONH- | 4-ThizMe | 5-Car-5-$NH_2$Pn |
| 7-5 | 3-PyrCONH- | 4-ThizMe | 1-Car-5-$NH_2$Pn |
| 7-6 | tBocNH- | 5-ImidMe | 1-Car-5-$NH_2$Pn |
| 7-7 | tBocNH- | 5-ImidMe | 5-Car-5-$NH_2$Pn |
| 7-8 | tBocNH- | 5-ImidMe | 5-Etc-5-$NH_2$Pn |
| 7-9 | tBocNH- | 5-ImidMe | 5-HOOC-5-$NH_2$Pn |
| 7-10 | tBocNH- | 4-ThizMe | 5-HOOC-5-$NH_2$Pn |
| 7-11 | tBocNH- | 4-ThizMe | 5-Car-5-$NH_2$Pn |
| 7-12 | tBocNH- | 4-ThizMe | 1-Car-5-$NH_2$Pn |
| 7-13 | AcNH- | 4-ThizMe | 1-Car-5-$NH_2$Pn |
| 7-14 | AcNH- | 4-ThizMe | 5-Car-5-$NH_2$Pn |
| 7-15 | AcNH- | 5-ImidMe | 1-Car-5-$NH_2$Pn |
| 7-16 | BzOCONH- | 5-ImidMe | 1-Car-5-$NH_2$Pn |
| 7-17 | BzOCONH- | 5-ImidMe | 5-HOOC-5-$NH_2$Pn |
| 7-18 | 1-tBoc-2-PyrdCONH- | 5-ImidMe | 5-Car-5-$NH_2$Pn |
| 7-19 | 1-tBoc-2-PyrdCONH- | 5-ImidMe | 5-Etc-5-$NH_2$Pn |
| 7-20 | 1-tBoc-2-PyrdCONH- | 5-ImidMe | 5-HOOC-5-$NH_2$Pn |
| 7-21 | 1-tBoc-2-PyrdCONH- | 5-ImidMe | 1-Car-5-$NH_2$Pn |
| 7-22 | 1-tBoc-2-PyrdCONH- | 4-ThizMe | 1-Car-5-$NH_2$Pn |

Table 7 (continued)

| Cpd No. | $R^1$ | $R^4$ | $R^7$ |
|---|---|---|---|
| 7-23 | 1-tBoc-2-PyrdCONH- | 4-ThizMe | 5-Car-5-$NH_2$Pn |
| 7-24 | 1-tBoc-2-PyrdCONH- | 4-ThizMe | m-$NH_2CH_2$Bz |
| 7-25 | tBocNH- | 4-ThizMe | m-$NH_2CH_2$Bz |
| 7-26 | tBocNH- | 5-ImidMe | m-$NH_2CH_2$Bz |

Table 8

| Cpd No. | $R^1$ | $R^4$ | $R^7$ |
|---|---|---|---|
| 8-1 | 3-PyrCONH- | 5-ImidMe | 5-Car-5-$NH_2$Pn |
| 8-2 | 3-PyrCONH- | 4-ThizMe | 5-Car-5-$NH_2$Pn |
| 8-3 | tBocNH- | 4-ThizMe | 5-Car-5-$NH_2$Pn |
| 8-4 | tBocNH- | 5-ImidMe | 5-Car-5-$NH_2$Pn |
| 8-5 | tBocNH- | 5-ImidMe | 1-Car-5-$NH_2$Pn |
| 8-6 | tBocNH- | 4-ThizMe | 1-Car-5-$NH_2$Pn |
| 8-7 | tBocNH- | 4-ThizMe | 5-Etc-5-$NH_2$Pn |
| 8-8 | tBocNH- | 4-ThizMe | 5-HOOC-5-$NH_2$Pn |
| 8-9 | AcNH- | 4-ThizMe | 5-Car-5-$NH_2$Pn |
| 8-10 | 1-tBoc-2-PyrdCONH- | 4-ThizMe | 5-Car-5-$NH_2$Pn |

Table 8 (continued)

| Cpd No. | $R^1$ | $R^4$ | $R^7$ |
|---|---|---|---|
| 8-11 | 1-tBoc-2-PyrdCONH- | 4-ThizMe | 1-Car-5-$NH_2$Pn |
| 8-12 | 1-tBoc-2-PyrdCONH- | 5-ImidMe | 1-Car-5-$NH_2$Pn |
| 8-13 | 1-tBoc-2-PyrdCONH- | 5-ImidMe | 5-Car-5-$NH_2$Pn |
| 8-14 | 1-tBoc-2-PyrdCONH- | 5-ImidMe | m-$NH_2CH_2$Bz |
| 8-15 | 1-tBoc-2-PyrdCONH- | 4-ThizMe | m-$NH_2CH_2$Bz |
| 8-16 | tBocNH- | 4-ThizMe | m-$NH_2CH_2$Bz |
| 8-17 | 3-PyrCONH- | 4-ThizMe | m-$NH_2CH_2$Bz |

Table 9

| Cpd No. | $R^1$ | $R^4$ | $R^7$ |
|---|---|---|---|
| 9-1 | 3-PyrCONH- | 5-ImidMe | 5-Car-5-$NH_2$Pn |
| 9-2 | 3-PyrCONH- | 5-ImidMe | 5-MeOCO-5-$NH_2$Pn |
| 9-3 | 3-PyrCONH- | 5-ImidMe | 5-HOOC-5-$NH_2$Pn |
| 9-4 | 3-PyrCONH- | 5-ImidMe | 1-Car-5-$NH_2$Pn |

Table 9

| Cpd No. | $R^1$ | $R^4$ | $R^7$ |
|---|---|---|---|
| 9-5 | 3-PyrCONH- | 4-ThizMe | 1-Car-5-$NH_2$Pn |
| 9-6 | *t*BocNH- | 4-ThizMe | 1-Car-5-$NH_2$Pn |
| 9-7 | *t*BocNH- | 5-ImidMe | 1-Car-5-$NH_2$Pn |
| 9-8 | *t*BocNH- | 5-ImidMe | 5-Car-5-$NH_2$Pn |
| 9-9 | *t*BocNH- | 5-ImidMe | 5-HOOC-5-$NH_2$Pn |
| 9-10 | AcNH- | 5-ImidMe | 5-Car-5-$NH_2$Pn |
| 9-11 | 1-*t*Boc-2-PyrdCONH- | 5-ImidMe | 5-Car-5-$NH_2$Pn |
| 9-12 | 1-*t*Boc-2-PyrdCONH- | 5-ImidMe | 5-HOOC-5-$NH_2$Pn |
| 9-13 | 1-*t*Boc-2-PyrdCONH- | 5-ImidMe | 1-Car-5-$NH_2$Pn |
| 9-14 | 1-*t*Boc-2-PyrdCONH- | 4-ThizMe | *m*-$NH_2CH_2$Bz |
| 9-15 | *t*BocNH- | 4-ThizMe | *m*-$NH_2CH_2$Bz |
| 9-16 | 3-PyrCONH- | 4-ThizMe | *m*-$NH_2CH_2$Bz |

Table 10

| Cpd No. | $R^1$ | $R^4$ | $R^7$ |
|---|---|---|---|
| 10-1 | 3-PyrCONH | 5-ImidMe | 5-Car-5-$NH_2$Pn |
| 10-2 | 3-PyrCONH | 5-ImidMe | 1-Car-5-$NH_2$Pn |
| 10-3 | 3-PyrCONH | 4-ThizMe | 1-Car-5-$NH_2$Pn |
| 10-4 | tBocNH- | 4-ThizMe | 1-Car-5-$NH_2$Pn |
| 10-5 | tBocNH- | 5-ImidMe | 5-Car-5-$NH_2$Pn |
| 10-6 | tBocNH- | 5-ImidMe | 5-Etc-5-$NH_2$Pn |
| 10-7 | tBocNH- | 5-ImidMe | 5-HOOC-5-$NH_2$Pn |
| 10-8 | 1-tBoc-2-PyrdCONH- | 5-ImidMe | 5-HOOC-5-$NH_2$Pn |
| 10-9 | 1-tBoc-2-PyrdCONH- | 5-ImidMe | 5-Car-5-$NH_2$Pn |
| 10-10 | 1-tBoc-2-PyrdCONH- | 5-ImidMe | 1-Car-5-$NH_2$Pn |
| 10-11 | 1-tBoc-2-PyrdCONH- | 4-ThizMe | 1-Car-5-$NH_2$Pn |
| 10-12 | 1-tBoc-2-PyrdCONH- | 4-ThizMe | 5-Car-5-$NH_2$Pn |
| 10-13 | 1-tBoc-2-PyrdCONH- | 4-ThizMe | m-$NH_2CH_2$Bz |
| 10-14 | tBocNH- | 4-ThizMe | m-$NH_2CH_2$Bz |
| 10-15 | 3-PyrCONH- | 4-ThizMe | m-$NH_2CH_2$Bz |
| 10-16 | 3-PyrCONH- | 4-ThizMe | 5-HOOC-5-$NH_2$Pn |

Table 11

| Cpd No. | $R^{1a}$ | $R^4$ | $R^6$ | $R^7$ |
|---|---|---|---|---|
| 11-1 | 1-Np | iBu | iBu | 5-Car-5-$NH_2$Pn |
| 11-2 | 1-Np | 5-ImidMe | iBu | 5-Car-5-$NH_2$Pn |
| 11-3 | 1-Np | 5-ImidMe | iBu | 6-HO-5-$NH_2$Hx |
| 11-4 | 1-Np | 5-ImidMe | iBu | 5-HOOC-5-$NH_2$Pn |
| 11-5 | 1-Np | 5-ImidMe | iBu | 5-HOMe-5-$NH_2$Pn |
| 11-6 | 1-Np | 5-ImidMe | iBu | 1-Car-5-$NH_2$Pn |
| 11-7 | 1-Np | 5-ImidMe | iBu | 1-Etc-5-$NH_2$Pn |
| 11-8 | 1-Np | 4-ThizMe | iBu | 1-Etc-5-$NH_2$Pn |
| 11-9 | 1-Np | 4-ThizMe | iBu | 1-Car-5-$NH_2$Pn |
| 11-10 | 1-Np | 4-ThizMe | iBu | 5-Car-5-$NH_2$Pn |
| 11-11 | 1-Np | 4-ThizMe | iBu | 6-HO-5-$NH_2$Hx |
| 11-12 | 1-Np | 4-ThizMe | iBu | 5-HOOC-5-$NH_2$Pn |
| 11-13 | 1-Np | 4-ThizMe | sBu | 5-HOOC-5-$NH_2$Pn |
| 11-14 | 1-Np | 4-ThizMe | sBu | 5-Car-5-$NH_2$Pn |
| 11-15 | 1-Np | 4-ThizMe | sBu | 1-Car-5-$NH_2$Pn |
| 11-16 | 1-Np | 4-ThizMe | sBu | 1-Car-4-$NH_2$Bu |
| 11-17 | 1-Np | 4-ThizMe | sBu | 1-Car-2-$NH_2$Et |
| 11-18 | 1-Np | 4-ThizMe | sBu | 5-HOOC-5-$NH_2$Pn |
| 11-19 | Ph | 4-ThizMe | iBu | 5-Car-5-$NH_2$Pn |
| 11-20 | 2-MeOEtOMe | 4-ThizMe | iBu | 5-Car-5-$NH_2$Pn |
| 11-21 | 3-Pyr | 4-ThizMe | iBu | 5-Car-5-$NH_2$Pn |
| 11-22 | 3-Pyr | 4-ThizMe | iBu | 5-Etc-5-$NH_2$Pn |
| 11-23 | 3-Pyr | 4-ThizMe | iBu | 5-HOOC-5-$NH_2$Pn |

Table 11 (continued)

| Cpd No. | $R^{1a}$ | $R^4$ | $R^6$ | $R^7$ |
|---|---|---|---|---|
| 11-24 | 3-Pyr | 4-ThizMe | iBu | 1-Car-5-$NH_2$Pn |
| 11-25 | 3-Pyr | 4-ThizMe | iBu | 1-HOOC-5-$NH_2$Pn |
| 11-26 | 3-Pyr | 5-ImidMe | iBu | 5-Car-5-$NH_2$Pn |
| 11-27 | MorCO- | 5-ImidMe | iBu | 5-Car-5-$NH_2$Pn |
| 11-28 | MorCO- | 5-ImidMe | iBu | 1-Car-5-$NH_2$Pn |
| 11-29 | MorCO- | 4-ThizMe | iBu | 5-HOOC-5-$NH_2$Pn |
| 11-30 | MorCO- | 4-ThizMe | iBu | 5-Car-5-$NH_2$Pn |
| 11-31 | MorCO- | 4-ThizMe | iBu | 6-HO-5-$NH_2$Hx |
| 11-32 | MorCO- | 4-ThizMe | iBu | 1-Car-5-$NH_2$Pn |
| 11-33 | MorCO- | 4-ThizMe | iBu | 1-HOMe-5-$NH_2$Pn |
| 11-34 | MorCO- | 4-ThizMe | iBu | 1-HOOC-5-$NH_2$Pn |
| 11-35 | MorCO- | 4-ThizMe | sBu | 1-Car-5-$NH_2$Pn |
| 11-36 | 1-PipCO- | 4-ThizMe | iBu | 5-Car-5-$NH_2$Pn |
| 11-37 | 1-PipCO- | 4-ThizMe | iBu | 5-HOOC-5-$NH_2$Pn |
| 11-38 | 1-PyrdCO- | 4-ThizMe | iBu | 5-Car-5-$NH_2$Pn |
| 11-39 | 1-PyrdCO- | 4-ThizMe | iBu | 1-Car-5-$NH_2$Pn |
| 11-40 | 2-PyrdCO- | 4-ThizMe | iBu | 5-HOOC-5-$NH_2$Pn |
| 11-41 | 1-Ac-2-PyrdCO- | 4-ThizMe | iBu | 5-HOOC-5-$NH_2$Pn |
| 11-42 | 1-Nic-2-PyrdCO- | 4-ThizMe | iBu | 5-HOOC-5-$NH_2$Pn |
| 11-43 | 2-PyrdCO- | 4-ThizMe | iBu | 1-Car-5-$NH_2$Pn |
| 11-44 | 1-Ac-2-PyrdCO- | 4-ThizMe | iBu | 1-Car-5-$NH_2$Pn |

Table 12

| Cpd No. | $R^{1a}$ | $R^4$ | $R^6$ | $R^7$ |
|---|---|---|---|---|
| 12-1 | 1-Np | 5-ImidMe | iBu | 6-HO-5-$NH_2$Hx |
| 12-2 | 1-Np | 5-ImidMe | iBu | 5-Car-5-$NH_2$Pn |
| 12-3 | 1-Np | 5-ImidMe | iBu | 1-Car-5-$NH_2$Pn |
| 12-4 | 1-Np | 4-ThizMe | iBu | 1-Car-5-$NH_2$Pn |
| 12-5 | 1-Np | 4-ThizMe | iBu | 5-HOOC-5-$NH_2$Pn |

Table 13

| Cpd No. | $R^{1a}$ | $R^4$ | $R^6$ | $R^7$ |
|---|---|---|---|---|
| 13-1 | 3-Pyr | 4-ThizMe | iBu | 5-Car-5-$NH_2$Pn |
| 13-2 | MorCO- | 4-ThizMe | iBu | 5-Car-5-$NH_2$Pn |
| 13-3 | MorCO- | 4-ThizMe | sBu | 5-Car-5-$NH_2$Pn |
| 13-4 | MorCO- | 5-ImidMe | iBu | 1-Car-5-$NH_2$Pn |
| 13-5 | MorCO- | 5-ImidMe | iBu | 5-Car-5-$NH_2$Pn |

Table 14

| Cpd No. | $R^{1a}$ | $R^4$ | $R^5$ | $R^7$ |
|---|---|---|---|---|
| 14-1 | 1-Np | 5-ImidMe | Ph | 5-Car-5-$NH_2$Pn |
| 14-2 | 1-Np | 4-ThizMe | Ph | 5-Car-5-$NH_2$Pn |
| 14-3 | 1-Np | 4-ThizMe | Ph | 6-HO-5-$NH_2$Hx |
| 14-4 | 3-Pyr | 4-ThizMe | Ph | 5-HOOC-5-$NH_2$Pn |
| 14-5 | 1-Np | 4-ThizMe | cHx | 5-HOOC-5-$NH_2$Pn |
| 14-6 | 1-Np | 4-ThizMe | cHx | 5-Car-5-$NH_2$Pn |
| 14-7 | 3-Pyr | 4-ThizMe | cHx | 5-Car-5-$NH_2$Pn |
| 14-8 | 3-Pyr | 4-ThizMe | cHx | 1-Car-5-$NH_2$Pn |
| 14-9 | MorCO- | 4-ThizMe | cHx | 1-Car-5-$NH_2$Pn |
| 14-10 | MorCO- | 4-ThizMe | cHx | 5-Car-5-$NH_2$Pn |
| 14-11 | MorCO- | 4-ThizMe | cHx | 5-HOOC-5-$NH_2$Pn |

Of the compounds listed above, the most preferred compounds are Compounds No. 2-3, 2-11, 2-21, 2-38, 2-45, 2-52, 2-115, 2-142, 2-235, 2-241, 4-12, 4-59, 5-1, 5-6, 6-5 and 11-3, that is to say:

$N^{\alpha}$-[N-nicotinoyl-3-(1-naphthyl)-alanyl-leucyl-statyl]-lysinamide

$N^{\epsilon}$-[N-nicotinoyl-3-(1-naphthyl)-alanyl-leucyl-statyl]-lysinol

N -(m-aminomethylbenzyl)-[N-nicotinoyl-3-(1-naphthyl)-alanyl-leucyl-statin]amide

$N^{\epsilon}$-[N-nicotinoyl-3-(1-naphthyl)-alanyl-3-(4-thiazolyl)-alanyl-statyl]-lysinol

$N^{\epsilon}$-[N-picolinyl-3-(1-naphthyl)-alanyl-3-(5-imidazolyl)-alanyl-statyl]-lysinol

$N^{\epsilon}$-[N-picolinyl-3-(1-naphthyl)-alanyl-leucyl-statyl]-lysinol

$N^{\alpha}$-[N-bis(1-naphthylmethyl)acetyl-leucyl-statyl]-lysinol

ethyl $N^{\epsilon}$-[N-benzyloxycarbonyl-3-(1-naphthyl)-alanyl-3-(5-imidazolyl)-alanyl-statyl]-lysinate

$N^{\epsilon}$-[N-bis(1-naphthylmethyl)acetyl-leucyl-statyl]-lysine

$N^{\epsilon}$-[N-bis(1-naphthylmethyl)acetyl-3-(4-thiazolyl)-alanyl-statyl]-lysinol

$N^{\epsilon}$-{4-[N-nicotinoyl-3-(1-naphthyl)-alanyl-3-(4-thiazolyl)-alanylamino]-5-cyclohexyl-3-hydroxypentanoyl}-lysinamide

N-(m-aminomethylbenzyl)-4-[N-nicotinoyl-3-(1-naphthyl)-alanyl-3-(4-thiazolyl)-alanylamino]-5-cyclohexyl-3-hydroxypentanamide

$N^{\alpha}$-[N-nicotinoyl-3-(1-naphthyl)-alanyl-3-(5-imidazolyl)-N-methyl-alanyl-statyl]-lysinamide

$N^{\epsilon}$-[N-nicotinoyl-3-(1-naphthyl)-alanyl-3-(4-thiazolyl)-N-methyl-alanyl-statyl]-lysinamide

$N^{\epsilon}$-{4-[N-nicotinoyl-3-(1-naphthyl)-alanyl-3-(4-thiazolyl)-N-methyl-alanylamino]-5-cyclohexyl-3-hydroxypentanoyl}-lysinamide

$N^{\epsilon}$-[N-bis(1-naphthylmethyl)acetyl-3-(5-imidazolyl)-alanyl-statyl-leucyl]-lysinol

and pharmaceutically acceptable salts thereof.

The compounds of the present invention are oligopeptides and may, therefore, be prepared, as is well known in the art by reacting together the component amino acids in any appropriate order, by reacting together two or more lower oligopeptides (again, if necessary, in any appropriate order) or by reacting one or more component amino acids with one or more lower oligopeptides (again, if necessary, in any appropriate order). However, provided that the correct sequence of amino acid residues in the oligopeptide of formula (I) is achieved, there is no particular restriction upon the order in which these reactions are carried out. In general terms, the compounds of the invention may be prepared by reacting together compounds of formulae:

$R^1$-CH($R^2$)-COOH (II)

or a reactive derivative thereof,

HN($R^3$)-CH($R^4$)-COOH (III)

or a reactive derivative thereof,

$H_2N$-CH($CH_2R^5$)-CH(OH)-$(CH_2)_m$-COOH (IV)

or a reactive derivative thereof,

optionally $H_2N$-CH($R^6$)-COOH (V)

or a reactive derivative thereof, and

$H_2N$-$R^7$ (VI)

or a reactive derivative thereof (in the above formulae $R^1$-$R^7$ and m are as defined above)

or by reacting a peptide compound derivable by reaction of some of said compounds of formulae (II), (III), (IV), (V) or (VI) or said reactive derivatives with the remainder of said compounds or said reactive derivative(s) or with a peptide compound derivable by reaction of said remainder or reactive derivative(s) thereof, the reaction(s) being in an order corresponding to the order of the residues derived from said compounds of formulae (II), (III), (IV) and (VI) and optionally (V) in said compound of formula (I).

If required, the resulting compound of formula (I) may be subjected to any one or more of the following optional reactions: acyl exchange, esterification, salification and conversion of any group represented by $R^4$ and/or $R^7$ to any other such group.

In specific embodiments of the process of the present invention, the compounds of the invention may be prepared by any of the following reactions illustrated as Methods A, B and C.

Method A

Specifically, in a first step of this reaction, a carboxylic acid compound of formula (VII):

$R^{1b}$-CH($R^{2a}$)-CO-N$R^3$-CH($R^{4a}$)-COOH (VII)

(in which $R^3$ is as defined above and $R^{1b}$, $R^{2a}$ and $R^{4a}$ represent any group defined above for $R^1$, $R^2$ and $R^4$, respectively, but in which any reactive group is optionally protected) or a reactive derivative thereof is reacted with an amino compound of formula (VIII):

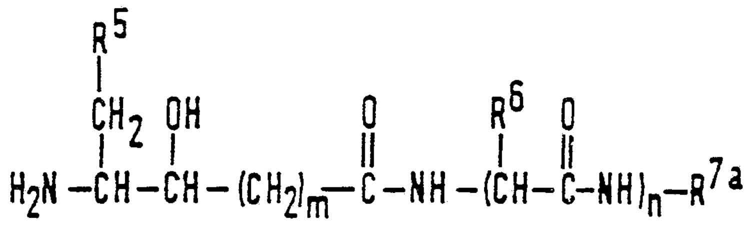 (VIII)

(in which $R^5$, $R^6$, m and n are as defined above and $R^{7a}$ represents any group defined for $R^7$ above, but in which any reactive group is optionally protected) or with a reactive derivative thereof.

Method B

In the first step of this reaction, a carboxylic acid compound of formula (IX):

$R^{1b}$-CH($R^{2a}$)-COOH (IX)

(in which $R^{1b}$ and $R^{2a}$ are as defined above) or a reactive derivative thereof is reacted with an amino compound of formula (X):

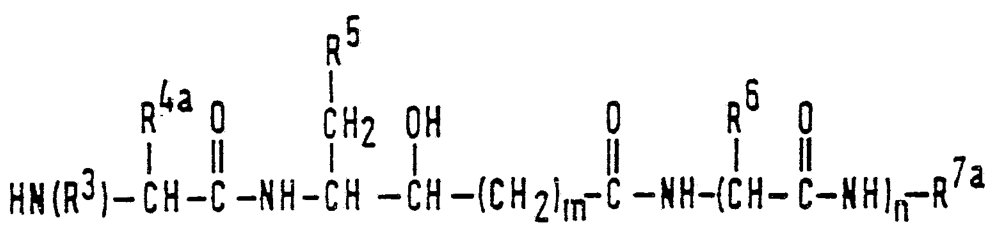 (X)

(in which $R^3$, $R^{4a}$, $R^5$, $R^6$, $R^{7a}$, m and n are as defined above) or with a reactive derivative thereof.

Method C

A further alternative comprises the three reactions:

(a) reacting a compound of formula (IX) (defined in Method B) or a reactive derivative thereof with an amino compound of formula (XI):

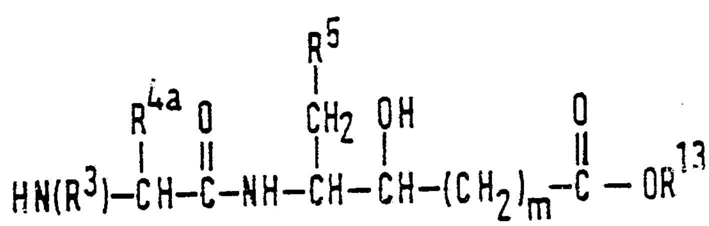 (XI)

(in which $R^3$, $R^{4a}$, $R^5$ and m are as defined above and $R^{13}$ represents a lower, e.g. $C_1$-$C_4$, alkyl group or an aralkyl group, preferably a $C_7$-$C_9$ aralkyl group) or with a reactive derivative thereof, to give a compound of formula (XII):

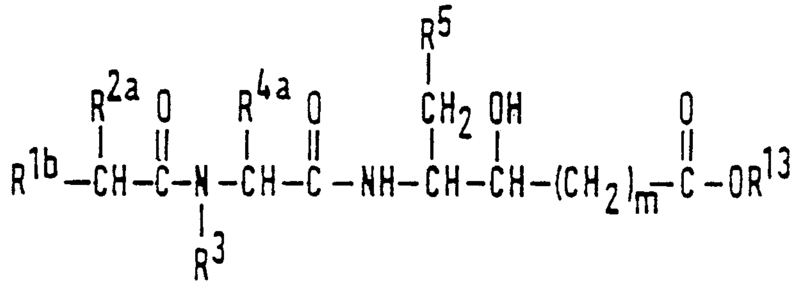

(XII)

(in which $R^{1b}$, $R^{2a}$, $R^3$, $R^{4a}$, $R^5$, $R^{13}$ and m are as defined above);

(b) if necessary, removing the alkyl or aralkyl group $R^{13}$ to convert the compound of formula (XII) to the free acid and/or converting said compound of formula (XII) or said free acid to a reactive derivative thereof; and

(c) reacting the product of step (a) or (b) with an amino compound of formula (XIII):

$H_2N\text{-}[CH(R^6)\text{-}CO\text{-}NH]_n\text{-}R^{7a}$ (XIII)

(in which $R^6$, $R^{7a}$ and n are as defined above) or with a reactive derivative thereof, to give a compound of formula (Ia):

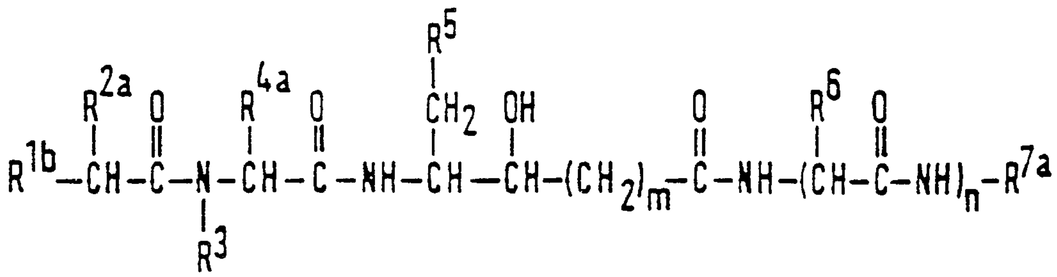

(Ia)

(in which $R^{1b}$, $R^{2a}$, $R^3$, $R^{4a}$, $R^5$, $R^6$, $R^{7a}$, m and n are as defined above).

At the end of the reactions described in Methods A, B and C, if desired, any or all of the protecting groups may be removed by conventional reactions well known to those skilled in the art and chosen having regard to the nature of the protecting group to be removed.

These reactions in Methods A, B and C are standard condensation reactions of the type conventionally used in peptide synthesis and they may be carried out according to any of the well-known techniques employed in peptide synthesis, for example by the azide method, the active ester method, the mixed acid anhydride method, the carbodiimide method or the condensation method. The reactive derivatives employed in these reactions are those reactive derivatives conventionally employed in such methods. Certain of these methods are described in more detail below.

Azide Method

First, the carboxylic acid of formula (VII), (IX) or (XII), usually in the form of its corresponding alkyl ester, is treated with hydrazine in an inert solvent (e.g. dimethylformamide) generally at about ambient temperature, to give the corresponding acid hydrazide. This hydrazide is then reacted with a nitrite, to convert it into an azide, after which the azide is reacted with the amine of formula (VIII), (X), (XI) or (XIII).

Examples of nitrites which may be employed include:
alkali metal nitrites, such as sodium nitrite, and alkyl nitrites, such as isoamyl nitrite.

The reaction of the acid hydrazide with the nitrite and the subsequent reaction of the resulting azide with the amine of formula (VIII), (X), (XI) or (XIII) are commonly carried out in the same reaction solution, without intermediate isolation of the azide. Both reactions are preferably carried out in the presence of an inert solvent. The nature of the solvent is not critical, provided that it does not interfere with the reaction. Suitable solvents include, for example: amides, such as dimethylformamide or dimethylacetamide; sulphoxides, such as dimethyl sulphoxide; and pyrrolidones, such as N -methylpyrrolidone. The reaction with the

nitrite is preferably effected at a relatively low temperature, e.g. from -50°C to 0°C, whilst the reaction of the azide with the amine is preferably effected at a temperature of from -10°C to +10°C. The time required for each of these reactions will vary, depending upon the nature of the reagents and the reaction temperature, but a period of from 5 minutes to 1 hour and a period of from 10 hours to 15 days will normally suffice for the reaction with the nitrite and the reaction of the azide with the amine, respectively.

Active Ester Method

In this method, the carboxylic acid of formula (VII), (IX) or (XII) is first converted to an active ester, after which this active ester is reacted with the amine of formula (VIII), (X), (XI) or (XIII).

Formation of the active ester is preferably effected by reacting the carboxylic acid of formula (VII), (IX) or (XII) with, for example, an N-hydroxyimide compound, such as N-hydroxysuccinimide, 1-hydroxybenzotriazole or N-hydroxy-5-norbornene-2,3-dicarboximide. The reaction to form the active ester is preferably effected in the presence of a condensing agent, such as dicyclohexylcarbodiimide or carbonyldiimidazole.

The reaction is preferably effected in the presence of an inert solvent, the nature of which is not critical, provided that it has no adverse effect upon the reaction. Suitable solvents include, for example: halogenated hydrocarbons, preferably halogenated aliphatic hydrocarbons, such as methylene chloride or chloroform; ethers, such as diethyl ether or tetrahydrofuran; and amides, such as dimethylformamide or dimethylacetamide.

The reaction temperature may vary over a wide range, for example from -10°C to +10°C. The time required for the reaction will vary widely, depending upon the nature of the reagents and upon the reaction temperature, but a period of from 30 minutes to 10 hours will normally suffice.

Reaction of this active ester with the amine of formula (VIII), (X), (XI) or (XIII) may be carried out with or without intermediate isolation of the active ester. Reaction of the active ester with the amine is preferably effected in the presence of an inert solvent, examples of which are as given for the preparation of the active ester itself. The temperature required for the reaction is not particularly critical and, for this reason, we normally prefer to carry out the reaction at about ambient temperature. The time required for the reaction will vary widely, but a period of from 30 minutes to 10 hours will normally suffice.

Mixed Acid Anhydride Method

In this method, the carboxylic acid of formula (VII), (IX) or (XII) is first converted to a mixed acid anhydride, and this is then reacted with the amine of formula (VIII), (X), (XI) or (XIII).

Preparation of the mixed acid anhydride is effected by reacting the acid of formula (VII), (IX) or (XII) with a suitable reagent, preferably in the presence of an inert solvent. Suitable reagents include: lower alkyl haloformates, such as ethyl chloroformate or isobutyl chloroformate; and di(lower alkyl) cyanophosphonates, such as diethyl cyanophosphonate. Examples of suitable inert solvents include the amides and ethers referred to in relation to the active ester method.

This reaction is preferably effected in the presence of an organic amine such as triethylamine or N-methylmorpholine. The reaction temperature may vary over a wide range, for example from -10°C to +10°C. The period required for the reaction will also vary widely, depending upon such factors as the nature of the reagents and the reaction temperature, but a period of from 30 minutes to 5 hours will normally suffice.

Reaction of the resulting mixed acid anhydride with the amine of formula (VIII), (X), (XI) or (XIII) is preferably effected in the presence of an inert solvent, the nature of which is not critical, provided that it does not interfere with the reaction. Suitable solvents include the amides and ethers hereinbefore exemplified in relation to the active ester method. The reaction will take place over a wide range of temperatures, but we generally find it convenient to carry out the reaction at a temperature of from 0°C to about ambient temperature. The time required for the reaction will vary, depending upon many factors, such as the nature of the reagents and the reaction temperature, but a period of from 1 hour to 24 hours will normally suffice.

## Condensation Method

In this method, the carboxylic acid of formula (VII), (IX) or (XII) is directly reacted with the amine of formula (VIII), (X), (XI) or (XIII). Such a reaction is preferably effected in the presence of a condensing agent, such as dicyclohexylcarbodiimide or carbonyldiimidazole. Otherwise, the reaction conditions and solvents are similar to those already described in relation to the active ester method.

## Protecting Reactive Groups

Where the reagents employed in any of the above reactions, that is to say the carboxylic acids of formula (VII), (IX) and (XII) or the amines of formula (VIII), (X), (XI) and (XIII) or their reactive derivatives, contain active groups (e.g. amino, carboxy, sulpho or imino groups including e.g. the imino group in the imidazolyl moiety of histidine) which are not intended to take part in peptide bond formation but which might interfere with the above reactions or undesirably participate in them, it is desirable that these groups should be protected before the reaction to form the peptide linkage and then, after that reaction, that the protected groups should be deprotected.

There is no particular limitation on the nature of the protecting group employed and such groups are well-known in peptide chemistry. For example, suitable amino-protecting groups include: carbonate residues, such as the benzyloxycarbonyl, p-methoxybenzyloxycarbonyl, t-butoxycarbonyl and 9-fluorenylmethyloxycarbonyl groups. Suitable carboxy-protecting and sulpho-protecting groups include the lower alkyl groups, e.g. the methyl, ethyl, propyl or t-butyl groups, and aralkyl groups, such as the benzyl group. Examples of guanidino-protecting groups include sulphonyl groups, such as the p-toluenesulphonyl group. Examples of imino-protecting groups include the 2,4-dinitrophenyl group.

The protecting groups may be inserted and then removed by conventional methods. For example, where the amino-protecting group is a t-butoxycarbonyl group or the carboxy-or sulpho-protecting group is a t-butyl group, these groups may be removed by treatment with an acid (e.g. hydrochloric acid, hydrofluoric acid, trifluoroacetic acid or boron trifluoride diethyl etherate) optionally in the presence of a cation scavenger (e.g. anisole or thioanisole). Such a reaction is preferably effected in an inert solvent (e.g. dioxane, methanol or dimethylformamide) at, for example, a temperature of from 0°C to 30°C. The time required for the reaction may vary widely, depending upon many factors, notably the nature of the reagents and the reaction temperature; however, a period of from 20 minutes to 1 hour will normally suffice.

When the amino group is protected by an aralkyloxycarbonyl group and when the carboxy group or sulpho group is protected by an aralkyl group, the protecting group can be removed by catalytic reduction of the protected compound in the presence of hydrogen (for example under a hydrogen pressure of from atmospheric to 10 atmospheres) and in the presence of a suitable hydrogenation catalyst, for example palladium-on-carbon or palladium black. The reaction is preferably effected in the presence of an inert solvent (e.g. methanol, ethanol or tetrahydrofuran). We generally find it convenient to carry out the reaction at about ambient temperature, although this is not critical. The time required for the reaction may vary widely, but a period of from 2 to 8 hours will normally suffice.

When the carboxy group or sulpho group is protected by a lower alkyl group, the protecting group may be removed by reacting the protected compound with an alkali (e.g. an alkali metal compound, preferably hydroxide such as sodium hydroxide or potassium hydroxide). The reaction is preferably effected in a solvent, the nature of which is not critical, provided that it has no adverse effect on the reaction. An aqueous solvent, such as aqueous methanol or aqueous ethanol is normally preferred. The reaction will take place over a wide range of temperatures, e.g. from 0 to 30°C. The time required for the reaction may vary widely, but a period of from 2 to 5 hours will normally suffice.

When the guanidino group is protected by a sulphonyl group, the protecting group may be removed by reacting the protected compound with an acid (e.g. hydrofluoric acid or trifluoromethanesulphonic acid) in the presence of a cation scavenger (e.g. anisole or thioanisole). The reaction will take place over a wide range of temperatures and the precise temperature chosen is not critical; we generally find it convenient to employ a temperature in the range from 0°C to 40°C. The time required for the reaction may vary widely, but a period of from 15 minutes to 1 hour will normally suffice.

Where the imino nitrogen atom in the imidazole moiety of a histidine residue is protected by a 2,4-dinitrophenyl, this may be removed by treating the protected compound with 2-mercaptoethanol. The reaction temperature is not critical, and we generally find it convenient to carry out the reaction at about ambient temperature.

## Conversion Reactions

If desired, a group represented by $R^4$ and/or $R^7$ in the compound of formula (I) prepared as described above may be converted to any other group represented by $R^4$ and/or $R^7$ by appropriate reactions well-known in the field of peptide synthesis.

If desired, any acyl group within the resulting compound of formula (I) may be converted to any other acyl group; the reactions and reaction conditions involved in such conversions are well known in the art.

If desired, any amino group contained in the groups represented by $R^4$ and $R^7$ can be converted into a guanidino group. This conversion may be achieved by reaction of the amino compound with, for example, 1-guanyl-3,5-dimethylpyrazole nitrate in an inert solvent (e.g. dimethylformamide) and in the presence of a base (e.g. triethylamine). The reaction temperature is preferably from 10°C to 25°C and the time required for the reaction will generally be from 1 to 7 days. Such a process is described, for example, by R.A.B. Bannard et al [Can. J. Chem. 36, 1541 (1958)].

After completion of any of the above reactions or of the final such reaction, the desired compound may be isolated from the reaction mixture by conventional means. For example, one suitable recovery procedure comprises: if necessary, neutralizing the reaction mixture; removing any insoluble residue by filtration; and then distilling off the solvent to give the desired compound. If necessary, this compound may be further purified by such conventional means as recrystallization, reprecipitation or the various chromatography techniques, such as column chromatography or preparative thin layer chromatography.

## INHIBITION OF RENIN ACTIVITY

The ability of various compounds of the invention to inhibit the activity of renin was determined according to the following method, which follows essentially the procedure of Kokubu et al. [Hypertension, 5, 191-197 (1983)].

Specifically, each test compound was dissolved in 60% v/v aqueous ethanol. Human renin activity in the presence and absence of each compound was measured using sheep angiotensinogen. The total volume of 1 ml of assay mixture contained 0.1 mole/litre phosphate buffer (pH 7.3), human renin (equivalent to 0.5 ng angiotensin I per ml per minute), sheep angiotensinogen (equivalent to 200 ng angiotensin I), $1 \times 10^{-6}$M of the test compound, 6% v/v ethanol and angiotensinase inhibitors (10 mmole/litre sodium ethylenediaminetetraacetate and 3.4 mmole/litre 8-hydroxyquinoline). The mixture was allowed to react for 10 minutes at 37°C, and then the reaction was stopped by placing the reaction tube in a boiling water bath for 5 minutes. The mixture was then centrifuged and the supernatant (0.05-0.1 ml) was used to assay remaining angiotensin I.

An identical experiment was carried out, as a control, except that the test compound was omitted. From the values obtained were calculated the % inhibition of renin activity achieved by each test compound. The results are shown in the following Table 15, in which the compounds of the invention are identified by the numbers assigned to them in the foregoing list. The values given are the mean of 3 or 4 experiments.

## BILE EXCRETION

When the compounds of the invention are administered orally and absorbed from the digestive tract, the absorbed compound is carried to the liver by the blood, and then part of the compound thus carried to the liver is excreted in the bile to the duodenum. Hence, the concentration of the drug in the bile gives an indication of the extent of absorption from the digestive tract.

To measure this, the common bile ducts of three rats were cannulated with polyethylene tubing under ether anaesthesia. After the rats had been allowed a brief recovery period, each was given orally one of the compounds shown in the following Table 15 at a dose of 30-50 mg/kg. The rats were then placed in restraint cages. The whole of the bile produced by each rat during the period of 24 hours after administration of the drug was collected via the cannula. The amount of drug in the bile was determined by

high speed liquid chromatography using a methanolic extract of 100 µl of the bile as a sample. The results are reported as the biliary excretion (%), which is the amount of drug in the bile calculated as a percentage of the amount of drug administered. The results are given in Table 15.

PLASMA LEVEL CONCENTRATION

An alternative measure of absorption from the digestive tract is the amount of the drug in the blood plasma.

The test animals were rats under ether anaesthesia. Each rat was given orally one of the drugs shown in the following Table 15 at a dose of 30-50 mg/kg. Blood samples were withdrawn at regular intervals from the jugular vein into heparinized syringes and centrifuged to obtain a plasma. The level of drug in the plasma was measured using a methanol extract of 100 µl of the plasma as a sample, by high speed liquid chromatography. The values were plotted on a graph against time following administration of the drug and the total area under the curve ("AUC") for the period from 0 to 3 hours was determined for each drug. The results are shown in Table 15.

Table 15

| Cpd. No. | % Inhibition ($1x10^{-6}M$) | Biliary excretion % | AUC (ng.h/ml) |
|---|---|---|---|
| 1-8 | 98 | - | - |
| 2-3 | 84 | 3.2 | 161 |
| 2-11 | 84 | 4.0 | 116 |
| 2-38 | 73 | 2.3 | - |
| 2-52 | 81 | - | - |
| 2-55 | 82 | - | - |
| 2-115 | 68 | - | 105 |
| 2-116 | 78 | - | - |
| 2-142 | 89 | - | - |
| 2-211 | 92 | - | - |
| 2-235 | 83 | - | - |
| 2-238 | 89 | - | - |
| 2-241 | 89 | - | - |
| 11-3 | 94 | - | - |
| 11-21 | 86 | - | - |

As can be seen from the results in the Table above, the compounds of the present invention have a substantial inhibitory effect on the activity of human renin and are thus useful for the diagnosis and therapy of renin/angiotensin-induced hypertension in humans and other animals. Furthermore, the results of the biliary excretion and blood plasma experiments indicate that the compounds are well absorbed from the digestive tract upon oral administration and this has been supported by tests in marmosets. Moreover, the compounds of the invention are readily soluble in water. All of these results indicate that the compounds of the invention will be of considerable therapeutic value and that, unlike related compounds proposed previously, they may be administered, in practice, by the oral route, as well be administered, in practice, by the oral route, as well as by the more conventional parenteral route.

The compounds of the invention may be formulated in conventional dosage forms, normally in admixture with a pharmaceutical carrier or diluent. For oral administration, the compounds can be formulated, for example, as tablets, capsules, granules, powders or syrups. For parenteral administration, they may be formulated as injections in a suitable liquid or as suppositories. The dosage will vary, depending upon the age, symptoms and body weight of the patient, as well as upon the desired end result; however, we would normally anticipate administering a dose of from 0.01 mg to 100 mg/kg body weight per day, which may be administered as a single dose or in divided doses.

The invention is further illustrated by the following non-limiting Examples and the preparation of certain of the starting materials used in these Examples is illustrated in the subsequent preparations. In these Examples, all values of optical rotation were measured using the sodium D-line, i.e. all such values are $[\alpha]_D$ . For the avoidance of doubt, the amino acid here described as statine is (3S,4S)-4-amino-3-hydroxy-6-methylheptanoic acid and the statyl group is the (3S,4S)-4-amino-3-hydroxy-6-methylheptanoyl group; norstatine is the corresponding compound from which a methylene group has been eliminated, i.e. (2S ,3S)-3-amino-2-hydroxy-5-methylhexanoic acid; the picolinyl group is the 2-pyridinecarbonyl group.

EXAMPLE 1

N-(m-Aminomethylbenzyl)-{N-[bis(1-naphthylmethyl)acetyl]-L-histidyl-statyl-L-leucin}amide dihydrochloride

1(a) N-[bis(1-Naphthylmethyl)acetyl]-L-histidyl-statine hydrazide

2.0 g (6.6 mmole) of ethyl N-t-butoxycarbonyl statinate were dissolved in 20 ml of a 4N hydrogen chloride/dioxane solution and left at room temperature for 20 minutes to cleave the t-butoxycarbonyl group. The reaction mixture was then concentrated by evaporation under reduced pressure, and the residue was dissolved in 10 ml of dimethylformamide. The resulting solution was neutralized with 0.67 g (6.6 mmole) of triethylamine, whilst ice-cooling. An active ester was prepared from 2.18 g (5.2 mmole) of a 1:1 molar complex of $N^{\alpha}$-t-butoxycarbonyl-$N^{im}$-2,4-dinitrophenyl-L-histidine with isopropanol, 1.18 g (6.6 mmole) of N - hydroxy-5-norbornene-2,3-dicarboximide and 1.48 g (7.2 mmole) of dicyclohexylcarbodiimide. 40 ml of a solution of this active ester in methylene chloride were added to the resulting solution. The mixture was stirred for 8 hours at room temperature, and then the precipitated dicyclohexylurea was filtered off. The filtrate was concentrated by evaporation under reduced pressure. On adding a 10% w/v aqueous solution of citric acid to the residue, an oily product separated, and this was extracted with ethyl acetate. The ethyl acetate extracts were washed, in turn, with water, with a saturated aqueous solution of sodium bicarbonate and with a saturated aqueous solution of sodium chloride, dried over anhydrous sodium sulphate and concentrated by evaporation under reduced pressure. The resulting oily residue was triturated with a 1:1 by volume mixture of diethyl ether and hexane to solidify it, and then the solvent was decanted off. After the addition of diethyl ether, the solid residue was broken up and filtered off.

The yellow powdery product thus obtained amounted to 3.4 g (5.6 mmole). The whole of this was treated with 20 ml of a 4N solution of hydrogen chloride in dioxane, to cleave the N-t-butoxycarbonyl group. The mixture was then evaporated under reduced pressure to dryness. The residue was dissolved in 20 ml of dimethylformamide. To the resulting solution were added 1.9 g (5.6 mmole) of bis(1-naphthylmethyl)-acetic acid and 0.96 g (5.6 mmole) of 90% diethyl cyanophosphonate, and then the mixture was ice-cooled. 1.13 g (11.2 mmole) of triethylamine was added to the mixture, which was then stirred at room temperature for 3 hours. The reaction mixture was then mixed with three times its own volume of ethyl acetate and washed, in turn, with 1N aqueous hydrochloric acid, with water, with a saturated aqueous solution of sodium bicarbonate and with a saturated aqueous solution of sodium chloride. It was then dried over anhydrous sodium sulphate, and the solvent was evaporated off under reduced pressure. A 1:1 by volume mixture of diethyl ether and hexane was added to the residue to solidify it. The solid product was broken up and filtered off to yield 3.84 g of a yellow powder.

The whole of this product (4.4 mmole) was dissolved in 40 ml of methanol, and 3.44 g (44 mmole) of 2-mercaptoethanol were added to the mixture. It was then adjusted to a pH value of 8 by the addition of a saturated aqueous solution of sodium bicarbonate and stirred at room temperature for 2.5 hours to remove the 2,4-dinitrophenyl protecting group. The solvent was evaporated off, and the residue was mixed with water to liberate an oily substance, which was extracted with ethyl acetate. The extracts were washed, in turn, with water and with a saturated aqueous solution of sodium chloride and dried over anhydrous sodium sulphate. The solvent was evaporated off under reduced pressure, and the residue was purified by column

chromatography through silica gel. The desired product was obtained in fractions eluted first with a 95:5:3 by volume mixture of methylene chloride, methanol and acetic acid followed by a 10:1 by volume mixture of methylene chloride and methanol. The solvents were removed by evaporation under reduced pressure, to give 2.12 g of a pale yellow powder.

The whole of this was dissolved in 10 ml of dimethylformamide, and then 3.2 g (64 mmole) of hydrazine hydrate were added, and the mixture was stirred at room temperature for 2 days. The reaction mixture was then concentrated by evaporation under reduced pressure, and water was added to the residue. The resulting precipitate was filtered off and washed with water and with diethyl ether to afford 1.06 g of N-[bis-(1-naphthylmethyl)acetyl]-L-histidyl-statine hydrazide as a pale yellow powder, melting at 129-131°C.

$[\alpha]^{23}$ -54.7° (C=0.3, methanol).

Elemental analysis:
Calculated for $C_{38}H_{44}N_6O_4$:
C, 70.35%; H, 6.84%; N, 12.95%.
Found: C, 70.12%; H, 7.02%; N, 12.74%.

1(b) Benzyl N-(m-L-leucylaminomethylbenzyl)carbamate

3.28 g (10 mmole) of the N-hydroxysuccinimide salt of N-t-butoxycarbonyl-L-leucine were added to a solution of 20.4 g (150 mmole) of m-xylenediamine dissolved in 50 ml of dimethylformamide, and the mixture was stirred at room temperature for 3 hours. The reaction mixture was then concentrated by evaporation under reduced pressure, and then a saturated aqueous solution of sodium bicarbonate was added to the residue, to liberate an oily substance. This was extracted with ethyl acetate. The ethyl acetate extracts were washed, in turn, with a saturated aqueous solution of sodium bicarbonate and with a saturated aqueous solution of sodium chloride and dried over anhydrous sodium sulphate. The solvent was evaporated off under reduced pressure, and the oily residue was purified by column chromatography through silica gel eluted with a 5:1 by volume mixture of methylene chloride and methanol. The resulting oily substance was dissolved in dimethylformamide, and then 1.01 g (10 mmole) of triethylamine was added, followed by the dropwise addition of 1.71 g (10 mmole) of benzyloxycarbonyl chloride, whilst ice-cooling. The mixture was then stirred for 3 hours, after which it was concentrated by evaporation under reduced pressure. Water was added to the residue to liberate an oily substance, which was extracted with ethyl acetate. The ethyl acetate extracts were washed, in turn, with 1N aqueous sulphuric acid, with water, with a saturated aqueous solution of sodium bicarbonate and with a saturated aqueous solution of sodium chloride, and dried over anhydrous sodium sulphate. The resulting solution was then evaporated to dryness under reduced pressure. The resulting oily residue was treated with 30 ml of 4N hydrogen chloride/dioxane to cleave the t-butoxycarbonyl group, and the mixture was evaporated to dryness under reduced pressure. The addition of a saturated aqueous solution of sodium bicarbonate to the residue precipitated a colourless powder, which was filtered off and washed throughly with water to afford 2.5 g of the title compound, melting at 75-77°C.

$[\alpha]^{23}$ +5.3° (C=0.3, methanol).

Elemental analysis:
Calculated for $C_{22}H_{29}N_3O_3$:
C, 68.90%; H, 7.62%; N, 10.96%.
Found: C, 68.71%; H, 7.85%; N, 10.74%.

1(c) N-(m-Aminomethylbenzyl)-{N-[bis(1-naphthylmethyl)acetyl]-L-histidyl-statyl-L-leucin}amide dihydrochloride

320 mg (0.5 mmole) of N-[bis(1-naphthylmethyl)acetyl]-L-histidyl-statine hydrazide [synthesized as described in step (a) above] were dissolved in 3 ml of dimethylformamide. 0.42 ml of a 4N solution of hydrogen chloride in dioxane was added to the resulting solution, whilst cooling with dry ice/acetone. 0.1 ml of isoamyl nitrite was added to the reaction mixture at -60°C, and then the temperature of the mixture was allowed to rise to -20°C, after which the mixture was stirred for 15 minutes at this temperature. When the

hydrazide was no longer detectable in the reaction mixture, the mixture was neutralized by adding 0.17 g of N-methylmorpholine, and then a solution of 0.19 g (0.5 mmole) of benzyl N-(m-L-leucylaminomethylbenzyl)-carbamate [synthesized as described in step (b) above] dissolved in 3 ml of dimethylformamide was added to the reaction mixture. The reaction mixture was stirred at 4°C for 21 hours, and then twice its own volume of ethyl acetate was added. The ethyl acetate layer was separated, washed, in turn, with a 5% w/v aqueous solution of sodium bicarbonate, with water and with a saturated aqueous solution of sodium chloride, dried over anhydrous sodium sulphate and then concentrated by evaporation under reduced pressure.

The residue was purified by column chromatography through silica gel eluted with a 25:1 by volume mixture of methylene chloride and methanol, followed by trituration with diethyl ether, to give 216 mg of pale yellow crystals. These crystals were dissolved in methanol and reduced at room temperature for 4 hours in the presence of 20 mg of 10% w/w palladium-on-carbon suspended in 0.4 ml of 1N aqueous hydrochloric acid and in an atmosphere of hydrogen. At the end of this time, the catalyst was removed by filtration, and the filtrate was concentrated by evaporation under reduced pressure. Water was separated as its benzene azeotrope. The resulting residue was triturated with a 1:1 by volume mixture of ethyl acetate and diethyl ether to afford 158 mg of the title compound as pale yellow crystals, melting at 168-172°C.

$[\alpha]^{23}$ -40.3 (C=0.3, methanol).

Elemental analysis:
Calculated for $C_{52}H_{63}O_5N_7.2HCl.2.5\ H_2O$:
C, 63.46%; H, 7.17%; N, 9.96%.
Found: C, 63.33%; H, 7.20%; N, 9.61%.

## EXAMPLE 2

### N-Nicotinoyl-3-(1-naphthyl)-L-alanyl-L-leucyl-statyl-L-lysinol hydrochloride

#### 2(a) Methyl N-t-butoxycarbonyl-L-leucyl-statinate

11.58 g (0.04 mole) of methyl N-t-butoxycarbonylstatinate were mixed with 92 ml of a 4N solution of hydrogen chloride in dioxane, and the mixture was stirred at room temperature for 1 hour to remove the t-butoxycarbonyl group. The reaction mixture was then evaporated to dryness under reduced pressure. 9.25 g (0.04 mole) of N-t-butoxycarbonyl-L-leucine were added to the residue, and the mixture was dissolved in 300 ml of dimethylformamide. 6.59 g (0.0404 mole) of diethyl cyanophosphonate were added dropwise to the solution followed by 9.7 g (0.096 mole) of triethylamine, whilst ice-cooling. The mixture was stirred at room temperature for 5 hours, and then the solvent was stripped off by evaporation under reduced pressure. Water was added to the residue while stirring to pulverize it. A solution of the resulting powder dissolved in ethyl acetate was washed successively with a 5% w/v aqueous solution of sodium bicarbonate and with water and dried over anhydrous magnesium sulphate. The solvent was then evaporated off under reduced pressure, and the resulting solid residue was recrystallized from a mixture of ethyl acetate and hexane, to give 13.7 g of the title compound, melting at 124-125°C.

#### 2(b) Methyl N-benzyloxycarbonyl-3-(1-naphthyl)-L-alanyl-L-leucyl-statinate

69 ml of a 4N solution of hydrogen chloride in dioxane were added to 12.1 g (0.03 mole) of methyl N-t-butoxycarbonyl-L-leucyl-statinate [prepared as described in step (a) above], and the mixture was stirred for 1 hour to remove the t-butoxycarbonyl group. The solvent was then evaporated off under reduced pressure, and 10.5 g (0.03 mole) of N -benzyloxycarbonyl-3-(1-naphthyl)-L-alanine were added to the residue. The mixture was dissolved in 300 ml of dimethylformamide. To the solution were added dropwise 4.94 g (0.0303 mole) of diethyl cyanophosphonate, followed by 7.3 g (0.072 mole) of triethylamine, whilst ice-cooling. The mixture was stirred at room temperature for 3 hours and then the solvent was evaporated off under reduced pressure. The residue was mixed with water whilst stirring to pulverize it. A solution of the resulting powder dissolved in ethyl acetate was successively washed with a 5% w/v aqueous solution of sodium bicarbonate and with water and dried over anhydrous magnesium sulphate. The solvent was evaporated off under

reduced pressure, and the resulting residue was purified by column chromatography through silica gel eluted with a 10:1 by volume mixture of chloroform and methanol. The product was recrystallized from a mixture of ethyl acetate and hexane to yield 15.4 g of the title compound, melting at 148-149°C.

2(c) Methyl 3-(1-naphthyl)-L-alanyl-L-leucyl-statinate hydrochloride

5 ml of 1N aqueous hydrochloric acid were added to a solution of 3.17 g (5 mmole) of methyl N-benzyloxycarbonyl-3-(1-naphthyl)-L -alanyl-L-leucyl-statinate [prepared as described in step (b) above] dissolved in 300 ml of methanol. The benzyloxycarbonyl group was then removed by stirring the mixture using a magnetic stirrer in an atmosphere of hydrogen and in the presence of 350 mg of 5% w/w palladium-on-carbon at room temperature for 2 hours. At the end of this time, the catalyst was filtered off. The filtrate was concentrated by evaporation under reduced pressure and triturated with a mixture of methanol and benzene. Recrystallization from ethyl acetate afforded 2.6 g of the title compound, melting at 193-194°C.

2(d) Methyl N-nicotinoyl-3-(1-naphthyl)-L-alanyl-L-leucyl-statinate

0.616 g (5 mmole) of nicotinic acid was added to a solution of 2.6 g (5 mmole) of methyl 3-(1-naphthyl)-L-alanyl-L-leucyl-statinate [prepared as described in step (c) above] dissolved in 50 ml of dimethylformamide. 0.816 g (5 mmole) of diethyl cyanophosphonate and 1.01 g (10 mmole) of triethylamine were added dropwise to the solution, whilst stirring and ice-cooling. After the mixture had been stirred at room temperature for 5 hours, the solvent was distilled off. Addition of water followed by stirring gave a powder. A solution of this powder in ethyl acetate was washed with water and dried over anhydrous magnesium sulphate. The solvent was evaporated off under reduced pressure, and the resulting residue was recrystallized from a mixture of ethyl acetate and hexane to yield 2.4 g of the title compound, melting at 140-143°C.

2(e) N-Nicotinoyl-3-(1-naphthyl)-L-alanyl-L-leucyl-statine

2.42 g (4 mmole) of methyl N-nicotinoyl-3-(1-naphthyl)-L -alanyl-L-leucyl-statinate [prepared as described in step (d) above] were dissolved in 30 ml of methanol. To the solution were added 3 ml (6 mmole) of 2N aqueous sodium hydroxide, and the mixture was stirred at room temperature for 4 hours, after which the methanol was evaporated off under reduced pressure. The mixture was adjusted to a pH value of 5.0 by the addition of 1N aqueous hydrochloric acid and extracted with ethyl acetate. The organic layer was washed with water and dried over anhydrous magnesium sulphate. The solvent was distilled off, and the resulting solid residue was recrystallized from ethyl acetate, to give 1.9 g of the title compound, melting at 172-174°C.

$[\alpha]^{25}$ -89.7° (C=0.3, methanol).

Elemental analysis:
Calculated for $C_{33}H_{42}N_4O_6.H_2O$:
C, 65.11%; H, 7.29%; N, 9.21%.
Found: C, 64.95%; H, 7.06%; N, 9.16%.

2(f) N $^{\alpha}$-[N-Nicotinoyl-3-(1-naphthyl)-L-alanyl-L-leucyl-statyl]-N $^{\epsilon}$-benzyloxycarbonyl-L-lysinol

248.1 mg (0.677 mmole) of $N^{\alpha}$-t-butoxycarbonyl-$N^{\epsilon}$-benzyloxycarbonyl-L-lysinol (prepared as described in Preparation 1) were mixed with 5 ml of a 4N solution of hydrogen chloride in dioxane, and the mixture was stirred at room temperature for 1 hour to remove the t-butoxycarbonyl group. The reaction mixture was then evaporated to dryness under reduced pressure, and the residue was dissolved in 20 ml of dimethylformamide. 400 mg of N -nicotinoyl-3-(1-naphthyl)-L-alanyl-L-leucyl-statine [prepared as described in step (e)

above] were added to the solution. 110.4 mg (0.677 mmole) of diethyl cyanophosphonate and subsequently 136.8 mg (1.35 mmole) of triethylamine were added dropwise to the solution, whilst ice-cooling. The mixture was then stirred at room temperature for 5 hours, after which the solvent was distilled off under reduced pressure.

The residue was diluted with water and extracted with ethyl acetate. The organic extract was successively washed with a 5% w/v aqueous solution of sodium carbonate and with water, dried over anhydrous magnesium sulphate and concentrated by evaporation under reduced pressure. The residue was purified by column chromatography through silica gel eluted with a 15:1 by volume mixture of chloroform and methanol, to give 167 mg of the title compound, melting at 165-167°C.

Elemental analysis:
Calculated for $C_{47}H_{62}N_6O_8.H_2O$:
C, 65.87%; H, 7.53%; N, 9.81%.
Found: C, 65.33%; H, 7.41%; N, 9.72%.

2(g) $N^{\alpha}$-[N-Nicotinoyl-3-(1-naphthyl)-L-alanyl-L-leucyl-statyl]-L-lysinol dihydrochloride

150 mg (0.175 mmole) of $N^{\alpha}$-[N-nicotinoyl-3-(1-naphthyl)-L -alanyl-L-leucyl-statyl]-$N^{\epsilon}$-benzyloxycarbonyl-L-lysinol [prepared as described in step (f) above] were dissolved in 15 ml of methanol, and 0.35 ml of 1N aqueous hydrochloric acid was added to the solution. The mixture was stirred using a magnetic stirrer for 2 hours in an atmosphere of hydrogen and in the presence of 100 mg of 10% w/w palladium-on-carbon to remove the benzyloxycarbonyl group. The catalyst was then filtered off and the filtrate was evaporated to dryness under reduced pressure. The residue was reprecipitated with a mixture of ethyl acetate and hexane to afford 95 mg of the title compound, melting at 184-186°C.

$[\alpha]^{25}$ -62.7° (C=0.3, methanol).

Elemental analysis:
Calculated for $C_{39}H_{58}Cl_2N_6O_6.H_2O$:
C, 58.86%; H, 7.60%; N, 10.56%;
Cl, 8.91%.
Found: C, 58.54%; H, 7.92%, N, 10.23%,
Cl, 8.72%.

EXAMPLE 3

$N^{\alpha}$-[N-Nicotinoyl-3-(1-naphthyl)-L-alanyl-L-leucyl-statyl]-L-lysinamide dihydrochloride

270 mg (0.68 mmole) of $N^{\alpha}$-t-butoxycarbonyl-$N^{\epsilon}$-benzyloxycarbonyl-L-lysinamide were treated with 5 ml of a 4N solution of hydrogen chloride in dioxane at room temperature for 20 minutes to remove the t-butoxycarbonyl group, and then the mixture was evaporated to dryness under reduced pressure. The residue was dissolved in 8 ml of dimethylformamide, 400 mg (0.68 mmole) of N-nicotinoyl-3-(1-naphthyl)-L-alanyl-L-leucyl-statine [prepared as described in Example 2(e)] and 130 mg (0.68 mmole) of diethyl cyanophosphonate were added and the mixture was cooled with ice. 138 mg (1.36 mmole) of triethylamine were added dropwise to the mixture, which was then stirred at room temperature for 3 hours. The reaction mixture was then diluted with twice its own volume of ethyl acetate, washed with a 15% w/v aqueous solution of sodium bicarbonate and with a saturated aqueous solution of sodium chloride and dried over anhydrous sodium sulphate. It was then concentrated by evaporation under reduced pressure. The residue was purified by preparative thin layer chromatography on silica gel (developing solvent: a 10:1 by volume mixture of methylene chloride and methanol), yielding 290 mg of $N^{\alpha}$-[N-nicotinoyl-3-(1-naphthyl)-L-alanyl-L-leucyl-statyl]-$N^{\epsilon}$-benzyloxycarbonyl-L-lysinamide as a colourless powder.

The whole (0.33 mmole) of this powder was dissolved in methanol. The resulting methanolic solution was mixed with 0.66 ml (0.66 mmole) of 1N aqueous hydrochloric acid, and the mixture was reduced in the presence of 10% w/w palladium-on-carbon and in an atmosphere of hydrogen. The catalyst was filtered off and the filtrate was concentrated by evaporation under reduced pressure. Trituration of the residue with ethyl acetate gave a precipitate, which was isolated by filtration to give 160 mg of the title compound as

colourless crystals, melting at 147-149°C.

$[\alpha]^{25}$ -60.3° (C=0.3, methanol).

Elemental analysis:
Calculated for $C_{39}H_{55}N_7O_6.2HCl.1.5H_2O$:
C, 57.28%; H, 7.39%; N, 11.99%;
Cl, 8.67%.
Found: C, 57.57%; H, 7.29%; N, 11.43%;
Cl, 8.73%.

EXAMPLE 4

Ethyl $N^{\epsilon}$-[N-benzyloxycarbonyl-3-(1-naphthyl)-L-alanyl-L-histidyl-statyl]-L-lysinate dihydrochloride

4(a) Hydrazide of N-benzyloxycarbonyl-3-(1-naphthyl)-L-alanyl-L-histidyl-statine

8.08 ml (32.3 mmole) of a 4N hydrogen chloride/dioxane solution and 1.76 ml (10.5 mmole) of isoamyl nitrite were added at -60°C to a solution of 4.76 g (9.5 mmole) of the hydrazide of N-benzyloxycarbonyl-3-(1-naphthyl)-L-alanyl-L-histidine dissolved in 30 ml of dimethylformamide, and then the mixture was stirred at -20°C for 10 minutes. After disappearance of the hydrazide had been confirmed, the reaction mixture was cooled again to -60°C and neutralized by adding 3.34 g (33 mmole) of N-methylmorpholine. To the resulting azide solution were added a solution of 2.14 g (9.5 mmole) of methyl statinate hydrochloride dissolved in 20 ml of dimethylformamide and 0.96 g (9.5 mmole) of N-methylmorpholine, and the mixture was stirred at 4°C for 3 days. The mixture was then concentrated by evaporation under reduced pressure, after which a 5% w/v aqueous solution of sodium bicarbonate was added, to liberate an oily substance, which was extracted with ethyl acetate. The ethyl acetate extracts were washed with a saturated aqueous solution of sodium chloride, dried over anhydrous sodium sulphate and concentrated by evaporation under reduced pressure. To the residue was added a 2:1 by volume mixture of diethyl ether and ethyl acetate, and the resulting jellied precipitate was separated by filtration. 3.67 g of methyl N-benzyloxycarbonyl-3-(1-naphthyl)-L-alanyl-L-histidyl-statinate were obtained from the precipitate as a pale yellow powder.

The whole (5.6 mmole) of this product was dissolved in 30 ml of dimethylformamide, and the resulting solution was mixed with 5.6 g (112 mmole) of hydrazine hydrate. The mixture was stirred at room temperature for 2 days and then concentrated by evaporation under reduced pressure. Addition of water to the residue gave a precipitate, which was collected by filtration to give 3.68 g of N-benzyloxycarbonyl-3-(1-naphthyl)-L -alanyl-L-histidyl-statine hydrazide as a colourless powder, melting at 178-181°C.

$[\alpha]^{25}$ -69.3° (C=0.3, methanol).

Elemental analysis:
Calculated for $C_{35}H_{43}N_7O_6$:
C, 63.91%; H, 6.59%; N, 14.91%.
Found: C, 63.65%: H, 6.45%; N, 15.03%.

4(b) Ethyl $N^{\epsilon}$-[N-benzyloxycarbonyl-3-(1-naphthyl)-L-alanyl-L-histidyl-statyl]-L-lysinate dihydrochloride

0.43 ml (1.7 mmole) of a 4N solution of hydrogen chloride in dioxane and 0.08 ml (0.55 mmole) of isoamyl nitrite were added to a solution of 329 mg (0.5 mmole) of N-benzyloxycarbonyl-3-(1-naphthyl)-L-alanyl-L-histidyl-statine hydrazide [prepared as described in step (a) above] dissolved in 8 ml of dimethylformamide and cooled to -60°C. The reaction mixture was stirred at -20°C for 10 minutes, cooled again to -60°C and then neutralized by adding 172 mg (1.7 mmole) of N-methylmorpholine. Ethyl N $^{\alpha}$-t-butoxycarbonyl-L-lysinate hydrochloride was prepared from 225 mg (0.55 mmole) of ethyl $N^{\alpha}$-t-butoxycarbonyl-$N^{\epsilon}$-benzyloxycarbonyl-L-lysinate by hydrogenolysis in the presence of 10% w/w palladium-on-carbon. A solution of the whole of this compound dissolved in 5 ml of dimethylformamide and 51 mg (0.50 mmole) of N -methylmorpholine were added to the reaction mixture. The mixture was then stirred at 4°C for 2 days, after which it was diluted with twice its own volume of ethyl acetate, washed, in turn, with a

5% w/v aqueous solution of sodium bicarbonate and with a saturated aqueous solution of sodium chloride and dried over anhydrous sodium sulphate. The solvent was then removed by evaporation under reduced pressure. The residue was purified by preparative thin layer chromatography on silica gel (developing solvent: a 10:1 by volume mixture of methylene chloride and methanol), to give 117 mg of ethyl $\underline{N}^{\epsilon}$-[$\underline{N}$-benzyloxycarbonyl-3-(1-naphthyl)-$\underline{L}$-alanyl-$\underline{L}$-histidyl-statyl]-$\underline{N}^{\alpha}$-t-butoxycarbonyl-$\underline{L}$-lysinate as a colourless powder. The whole of this compound was treated with a 4N hydrogen chloride/dioxane solution at room temperature for 20 minutes, followed by evaporation to dryness under reduced pressure. The resulting solid residue was broken up in the presence of diethyl ether and collected by filtration, to give 166 mg of the title compound as a colourless powder, melting at 131-133°C.

$[\alpha]^{25}$ -61.3° (C=0.3, methanol).

Elemental analysis:
Calculated for $C_{43}H_{57}N_7O_8.2HCl.2H_2O$:
C, 56.82%; H, 6.98%; N, 10.27%;
Cl, 7.80%.
Found: C, 56.54%; H, 6.79%; N, 9.48%;
Cl, 8.05%.

## EXAMPLE 5

### $N^{\alpha}$-{N-[Bis(1-naphthylmethyl)acetyl]-L-leucyl-statyl}-L-lysinamide hydrochloride

#### 5(a) Benzyl N-[bis(1-naphthylmethyl)acetyl]-L-leucinate

6.81 g (20 mmole) of bis(1-naphthylmethyl)acetic acid and 7.90 g (20 mmole) of the p-toluenesulphonic acid salt of benzyl L-leucinate were suspended in anhydrous tetrahydrofuran in an atmosphere of nitrogen. To the suspension were added 3.33 ml (22 mmole) of diethyl cyanophosphonate and 6.14 ml (44 mmole) of triethylamine, whilst ice-cooling, and the mixture was stirred overnight at room temperature. The solvent was then evaporated off under reduced pressure. The residue was dissolved in ethyl acetate and the solution was washed with a 10% w/v aqueous solution of citric acid, with water and with a saturated aqueous solution of sodium bicarbonate, in that order, and dried over anhydrous magnesium sulphate. The solvent was distilled off under reduced pressure, and then the residue was purified by column chromatography through silica gel eluted with a 1:3 by volume mixture of ethyl acetate and hexane, to give 8.3 g - (76%) of the title compound as white crystals, melting at 88-91°C.

#### 5(b) N-[Bis(1-naphthylmethyl)acetyl]-L-leucine

8.3 g of benzyl N-[bis(1-naphthylmethyl)acetyl]-L-leucinate [prepared as described in step (a) above] were dissolved in 200 ml of ethanol. The solution was stirred overnight at room temperature in an atmosphere of hydrogen and in the presence of 1.0 g of 10% w/w palladium-on-carbon. At the end of this time, the catalyst was filtered off and the filtrate was concentrated by evaporation under reduced pressure to give 6.6 g (95%) of the title compound as white crystals, melting at 184-186°C.

#### 5(c) Methyl N-[bis(1-naphthylmethyl)acetyl]-L-leucyl-statinate

4.70 g (10.4 mmole) of N-[bis(1-naphthylmethyl)acetyl]-L-leucine [prepared as described in step (b) above] and 2.35 g (10.4 mmole) of methyl statinate hydrochloride were dissolved in 100 ml of anhydrous tetrahydrofuran. 1.73 ml (11.4 mmole) of diethyl cyanophosphonate and 3.18 ml of triethylamine were added to the solution, whilst ice-cooling and under a nitrogen atmosphere. The mixture was then stirred overnight at room temperature. The solvent was then distilled off under reduced pressure, and the residue was purified by column chromatography through silica gel eluted with a 1:4 by volume mixture of ethyl acetate and hexane, to give 4.50 g (69%) of the hemihydrate of the title compound as white crystals, melting at 71-75°C.

Elemental analysis:
Calculated for $C_{39}H_{48}N_2O_5.1/2H_2O$:
C, 73.90%; H, 7.79%; N, 4.42%.
Found: C, 73.86%; H, 7.73%; N, 4.51%.

5(d) N-[Bis(1-naphthylmethyl)acetyl]-L-leucyl-statine

1.00 g (1.60 mmole) of methyl N-[bis(1-naphthylmethyl)acetyl]-L -leucyl-statinate [prepared as described in step (c) above] was dissolved in 50 ml of a 1:4 by volume mixture of water and methanol. To this solution was added a solution of 640 mg (16 mmole) of sodium hydroxide dissolved in 10 ml of water, whilst ice-cooling, and the mixture was then stirred for 1 hour. The solvent was then distilled off under reduced pressure. The residue was diluted with water and adjusted to a pH value of 1 with concentrated aqueous hydrochloric acid to precipitate crystals, which were collected by filtration and dried to give 900 mg (92%) of the title compound as white crystals, melting at 99-104°C.

$[\alpha]^{25}$ -96.0° (C=0.3, methanol).

Elemental analysis:
Calculated for $C_{38}H_{46}N_2O_5$:
C, 74.73%; H. 7.59%; N, 4.59%.
Found: C, 74.11%; H, 7.70%; N, 4.58%.

5(e) $N^{\alpha}$-{N-[Bis(1-naphthylmethyl)acetyl]-L-leucyl-statyl}-$N^{\epsilon}$-benzyloxycarbonyl-L-lysinamide

The t-butoxycarbonyl group was removed from 200 mg (0.51 mmole) of $N^{\alpha}$-t-butoxycarbonyl-$N^{\epsilon}$-benzyloxycarbonyl-L-lysinamide by treatment with a 4N hydrogen chloride/dioxane solution to give the hydrochloride of $N^{\epsilon}$-benzyloxycarbonyl-L-lysinamide. The whole of this and 310 mg (0.51 mmole) of N-[bis-(1-naphthylmethyl)acetyl]-L-leucyl-statine [prepared as described in step (d) above] were dissolved in anhydrous tetrahydrofuran. To the solution were added 0.09 ml (0.59 mmole) of diethyl cyanophosphonate and 0.16 ml (1.15 mmole) of triethylamine, whilst ice-cooling and in an atmosphere of nitrogen. The mixture was stirred overnight at room temperature, and then the solvent was stripped off by evaporation under reduced pressure. The residue was purified by preparative thin layer chromatography (developing solvent: a 10:1 by volume mixture of chloroform and methanol) to give 318 mg (72%) of the monohydrate of the title compound as white crystals, melting at 101-103°C.

Elemental analysis:
Calculated for $C_{52}H_{65}N_5O_7.H_2O$:
C, 70.17%; H, 7.59%; N, 7.87%.
Found: C, 69.61%; H, 7.45%; N, 7.61%.

5(f) $N^{\alpha}$-{N-[Bis(1-naphthylmethyl)acetyl]-L-leucyl-statyl}-L-lysinamide hydrochloride

283 mg (0.33 mmole) of $N^{\alpha}$-{N-[bis(1-naphthylmethyl)acetyl]-L-leucyl-statyl}-$N^{\epsilon}$-benzyloxycarbonyl-L-lysinamide [prepared as described in step (e) above] were dissolved in 10 ml of ethanol, and then 0.33 ml of 1N aqueous hydrochloric acid was added. The mixture was stirred for 3 hours in an atmosphere of hydrogen and in the presence of 30 mg of 10% w/w palladium-on-carbon. The catalyst was filtered off, and then the filtrate was evaporated to dryness under reduced pressure to give 187 mg (74%) of the monohydrate of the title compound as white crystals, melting at 151-154°C.

$[\alpha]^{25}$ -87.3°C (C=0.3, methanol).

Elemental analysis:
Calculated for $C_{44}H_{60}N_5O_5Cl.H_2O$:
C, 67.46%; H, 7.85%; N, 8.94%;

Cl, 4.53%.
Found: C, 67.78%; H, 8.13%; N, 8.13%;
Cl, 4.93%.

EXAMPLE 6

$N^{\alpha}$-{N-[Bis(1-naphthylmethyl)acetyl]-L-leucyl-statyl}-L-lysinol hydrochloride

6(a) $N^{\alpha}$-{N-[Bis(1-naphthylmethyl)acetyl]-L-leucyl-statyl}-$N^{\epsilon}$-benzyloxycarbonyl-L-lysinol

A mixture of 400 mg (0.64 mmole) of N-[bis(1-naphthylmethyl)acetyl]-L-leucyl-statine [synthesized by the method described in Example 5(d)] and $N^{\epsilon}$-benzyloxycarbonyl-L-lysinol hydrochloride [prepared from 233 mg (0.64 mmole) of $N^{\alpha}$-t-butoxycarbonyl-$N^{\epsilon}$-benzyloxycarbonyl-L-lysinol by removing the t-butoxycarbonyl group with a 4N hydrogen chloride/dioxane solution in the usual way] was dissolved in 20 ml of dimethylformamide. To the solution were added dropwise 195 mg (1.20 mmole) of 90% diethyl cyanophosphonate and 500 mg (4.95 mmole) of triethylamine, whilst ice-cooling, and the mixture was stirred at 4°C for 1 hour and then at room temperature overnight. The reaction mixture was then poured into ice-water and extracted with ethyl acetate. The extracts were washed, in turn, with a 10% w/v aqueous solution of citric acid, with a 10% w/v aqueous solution of sodium bicarbonate and with a saturated aqueous solution of sodium chloride and then dried over anhydrous sodium sulphate. The solvent was then removed by evaporation under reduced pressure. The residue was purified by preparative thin layer chromatography over silica gel (developing solvent: a 20:1 by volume mixture of chloroform and methanol), to afford 350 mg of the title compound as a white powder, melting at 75-80°C.

Elemental analysis:
Calculated for $C_{52}H_{66}N_4O_7$:
C, 72.70%; H, 7.74%; N, 6.52%.
Found: C, 72.43%: H, 7.95%; N, 6.29%.

6(b) $N^{\alpha}$-{N-[bis(1-Naphthylmethyl)acetyl]-L-leucyl-statyl}-L-lysinol hydrochloride

190 mg (0.22 mmole) of $N^{\alpha}$-{N-[bis(1-naphthylmethyl)acetyl]-L -leucyl-statyl}-$N^{\epsilon}$-benzyloxycarbonyl-L-lysinol [prepared as described in step (a) above] were dissolved in 10 ml of methanol, and then 0.23 ml of 1N aqueous hydrochloric acid were added, and the mixture was reduced catalytically for 4 hours in a hydrogen atmosphere and in the presence of 50 mg of 10% w/w palladium on-carbon. The mixture was then filtered and the filtrate was concentrated by evaporation under reduced pressure. Addition of diethyl ether to the residue precipitated 110 mg of the title compound as a colourless powder, melting at 105-110°C.

$[\alpha]^{23}$ -69.3° (C=0.3, methanol).

Elemental analysis:
Calculated for $C_{44}H_{60}N_4O_5$.HCl:
C, 69.40%; H, 8.08%; N, 7.36%.
Found: C, 69.41%: H, 8.24%; N, 7.14%.

EXAMPLE 7

α-{N-[Bis(1-naphthylmethyl)acetyl]-L-leucyl-statylamino}-ε-aminopimelic acid hydrochloride hydrate

7(a) α,ε-Dimethyl α-{N-[bis(1-naphthylmethyl)acetyl]-L-leucyl-statylamino}-ε-t-butoxycarbonylaminopimelate

To a solution of 500 mg (0.80 mmole) of N-[bis(1-naphthylmethyl)acetyl]-L-leucyl-statine [prepared as described in Example 5(d)] and 253 mg (0.80 mmole) of α,ε-dimethyl α-amino-ε-t-butoxycarbonylaminopimelate in 15 ml of dimethylformamide were added 155 mg (0.95 mmole) of 90% diethyl cyanophosphonate and 0.5 ml of triethylamine, whilst ice-cooling and stirring, and stirring of the mixture was continued for 1 hour at the same temperature and then for 3 hours at room temperature. The reaction mixture was then poured into ice-water and extracted with ethyl acetate. The extracts were washed with a 10% w/v aqueous solution of citric acid, with a 10% w/v aqueous solution of sodium bicarbonate and with a saturated aqueous solution of sodium chloride, dried over anhydrous sodium sulphate and concentrated by evaporation under reduced pressure. The residue was purified by preparative thin layer chromatography (developing solvent: a 20:1 by volume mixture of chloroform and methanol) to give 450 mg of the title compound as a white powder.

7(b) α-{N-[Bis(1-naphthylmethyl)acetyl]-L-leucyl-statylamino}-ε-t-butoxycarbonylaminopimelic acid

450mg (0.49 mmole) of α,ε-dimethyl α-{ N-[bis(1-naphthylmethyl)acetyl]-L-leucyl-statylamino}-ε-t-butoxycarbonylaminopimelate [prepared as described in step (a) above] were mixed with 10 ml of a 10% w/v solution of sodium hydroxide in a 1:5 by volume mixture of water and methanol. The mixture was stirred at room temperature for 30 minutes and then concentrated by evaporation under reduced pressure. Ethyl acetate was added to dissolve the residue, and the ethyl acetate solution was washed successively with a 10% w/v aqueous solution of citric acid and with a saturated aqueous solution of sodium chloride and then dried over anhydrous sodium sulphate. The solvent was then removed by evaporation under reduced pressure, and the resulting residue was reprecipitated from a mixture of methylene chloride and hexane, to give 360 mg of the title compound as white crystals, melting at 115-120°C.

Elemental analysis:
Calculated for $C_{50}H_{66}N_4O_{10}.3/2H_2O$:
C, 65.98%; H, 7.64%; N, 6.16%.
Found: C, 66.39%; H, 7.56%; N, 5.72%.

7(c) α-{N-[Bis(1-naphthylmethyl)acetyl]-L-leucyl-statylamino}-ε-aminopimelic acid hydrochloride hydrate

A mixture of 250 mg (0.28 mmole) of α-{N-[bis-(1-naphthylmethyl)acetyl]-L -leucyl-statylamino}-ε-t-butoxycarbonylaminopimelic acid [prepared as described in step (b) above] and 10 ml of a 4N hydrogen chloride/dioxane solution was stirred for 30 minutes at room temperature. The reaction mixture was then concentrated by evaporation under reduced pressure and the residue was washed with diethyl ether and reprecipitated from a mixture of chloroform and hexane to yield 200 mg of the title compound as a white powder melting at 140-150°C.

$[\alpha]^{23}$ -66.7° (C=0.3, methanol).

Elemental analysis:
Calculated for $C_{45}H_{58}N_4O_8.HCl.H_2O$:
C, 64.54%; H, 7.34%; N, 6.69%.
Found: C, 64.28%; H, 7.29%; N, 6.45%.

EXAMPLE 8

$N^{\epsilon}$-{$N^{\alpha}$-[Bis(1-naphthylmethyl)acetyl]-L-histidyl-statyl-L-leucyl}-L-lysinol

8(a) $N^{\epsilon}$-(N-Benzyloxycarbonyl-L-leucyl)-$N^{\alpha}$-t-butoxycarbonyl-L-lysinol

1.81 g (5 mmole) of the N-hydroxysuccinimide ester of N-benzyloxycarbonyl-L-leucine and 1.16 g (5 mmole) of $N^{\alpha}$-t-butoxycarbonyl-L-lysinol were dissolved in 25 ml of methylene chloride, and the solution was stirred at room temperature for one day. The reaction mixture was then concentrated by evaporation under reduced pressure, and ethyl acetate was added to the residue. The resulting ethyl acetate solution was washed successively with 1N aqueous sulphuric acid, with water, with a 5% w/v aqueous solution of sodium bicarbonate and with a saturated aqueous solution of sodium chloride and dried over anhydrous sodium sulphate. The solvent was then distilled off under reduced pressure. The resulting solid residue was broken up in the presence of diethyl ether and collected by filtration to give 2.13 g of the title compound as a colourless powder, melting at 119-123°C.

$[\alpha]^{22}$ -22.0° (C=0.5, methanol).

Elemental analysis:
Calculated for $C_{25}H_{41}N_3O_6.1/4H_2O$:
C, 62.02%; H, 8.64%; N, 8.68%.
Found: C, 62.21%; H, 8.68%; N, 8.68%.

8(b) $N^{\epsilon}$-{N-[Bis(1-naphthylmethyl)acetyl]-L-histidyl-statyl-L-leucyl}-L-lysinol

0.84 ml of a 4N solution of hydrogen chloride in dioxane and 0.2 ml of isoamyl nitrite were added at -60°C to a solution of 0.65 g (1 mmole) of $N^{\alpha}$-bis(1-naphthylmethyl)acetyl-L -histidyl-statine hydrazide in 6 ml of dimethylformamide. The reaction temperature was allowed to rise to -20°C, and the mixture was stirred at that temperature for 10 minutes. The mixture was cooled again to -60°C, and then it was neutralized by adding 0.34 g (3.4 mmole) of N-methylmorpholine. A solution of L-leucyl-$N^{\alpha}$-t-butoxycarbonyl-L-lysinol hydrochloride [which was prepared by the hydrogenolysis of the benzyloxycarbonyl group from 0.48 g (1 mmole) of $N^{\epsilon}$-(N-benzyloxycarbonyl-L-leucyl)-$N^{\alpha}$-t-butoxycarbonyl-L-lysinol in the presence of 10% w/w palladium-on-carbon] in 5 ml of dimethylformamide and 0.11 g (1.1 mmole) of N-methylmorpholine were added to the resulting mixture. The mixture was then stirred at 4°C for 2 days, after which it was diluted with twice its own volume of ethyl acetate. The ethyl acetate solution was washed successively with 1N aqueous sulphuric acid, with a 5% w/v aqueous solution of sodium bicarbonate and with a saturated aqueous solution of sodium chloride and dried over anhydrous sodium sulphate. The solvent was then stripped off by evaporation under reduced pressure. The residue was purified by flash column chromatography (eluted with a 10:1 by volume mixture of methylene chloride and methanol) to give 0.40 g of $N^{\epsilon}$-{N-[bis(1-naphthylmethyl)acetyl]-L-histidyl-statyl-L-leucyl}-$N^{\alpha}$-t-butoxycarbonyl-L-lysinol as a pale yellow powder. The whole of this product was treated with 10 ml of a 4N solution of hydrogen chloride in dioxane at room temperature for 30 minutes to remove the t-butoxycarbonyl group, and then the solution was evaporated to dryness under reduced pressure. The resulting solid residue was broken up in the presence of ethyl acetate and filtered off, to give 0.33 g of the title compound as a pale orange powder melting at 127-130°C.

$[\alpha]^{22}$ -36.6° (C=0.5, methanol).

Elemental analysis:
Calculated for $C_{50}H_{67}N_7O_6.2HCl.3H_2O$:
C, 60.71%; H, 7.64%; N, 9.91%;
Cl, 7.17%.
Found: C, 60.83%; H, 7.48%; N, 9.65%;
Cl, 7.32%.

EXAMPLE 9

$N^{\epsilon}$-{N-[2(RS)-(1-Naphthyl)methyl-3-(3-pyridyl)propionyl]-3-(4-thiazolyl)-DL-alanyl-statyl-L-leucyl}-lysinamide dihydrochloride

9(a) Methyl N-[2(RS)-(1-naphthyl)methyl-3-(3-pyridyl)propionyl]-3-(4-thiazolyl)-DL-alanate.

0.82 g (5 mmole) of diethyl cyanophosphonate and 1.66 g (16.5 mmole) of triethylamine were added, whilst ice-cooling, to a solution of 1.3 g (5 mmole) of methyl 3-(4-thiazolyl)-DL-alanate hydrochloride and 1.46 g (5 mmole) of 2(RS)-(1-naphthyl)methyl-3-(3-pyridyl)propionic acid (prepared as described in Preparation 3) dissolved in 25 ml of dimethylformamide, and the mixture was stirred at room temperature for 6 hours. The reaction mixture was then concentrated by evaporation under reduced pressure. Water was added to the resulting residue, and the mixture was then extracted with ethyl acetate. The ethyl acetate extracts were washed with water and dried over anhydrous magnesium sulphate, and then the solvent was stripped off by evaporation under reduced pressure. The residue was purified by column chromatography through silica gel eluted with a 20:1 by volume mixture of chloroform and methanol. The desired fractions were collected and concentrated by evaporation under reduced pressure to afford 1.58 g of the title compound, melting at 133-135°C.

Elemental analysis:
Calculated for $C_{26}H_{25}N_3O_3S$:
C, 67.95%; H, 5.48%; N, 9.14%;
S, 6.98%.
Found: C, 67.86%; H, 5.61%; N, 8.95%;
S, 6.69%.

9(b) N-[2(RS)-(1-Naphthyl)methyl-3-(3-pyridyl)propionyl]-3-(4-thiazolyl)-DL-alanine hydrazide

3.75 g (60 mmole) of 80% hydrazine hydrate were added, whilst ice-cooling, to a solution of 1.38 g (3 mmole) of methyl N-[2(RS)-(1-naphthyl)methyl-3-(3-pyridyl)propionyl]-3-(4-thiazolyl)-DL-alanate [prepared as described in step (a) above] dissolved in 15 ml of dimethylformamide, and the mixture was stirred at room temperature for 4 hours and then allowed to stand overnight. The mixture was then concentrated by evaporation under reduced pressure. Water was added to the resulting residue to precipitate crystals, which were separated by filtration and then recrystallized from water to give 1.05 g of the title compound, melting at 174-176°C.

Elemental analysis:
Calculated for $C_{25}H_{25}N_5O_2S.H_2O$:
C, 62.87%; H, 5.70%; N, 14.66%;
S, 6.72%.
Found: C, 62.71%; H, 5.43%; N, 14.72%;
S, 6.62%.

9(c) $N^{\epsilon}$-(N-t-Butoxycarbonyl-statyl-L-leucyl)-$N^{\alpha}$-benzyloxycarbonyl-L-lysinamide

1.83 g (18.3 mmole) of triethylamine and 2.5 g (18.3 mmole) of isobutyl chloroformate were added dropwise, whilst ice-cooling, to a solution of 6.0 g (15.8 mmole) of $N^{\alpha}$-benzyloxycarbonyl-$N^{\epsilon}$-t-butoxycarbonyl-L-lysine in 50 ml of methylene chloride, and the reaction mixture was stirred for 30 minutes. At the end of this time, 10 ml of a methanolic solution saturated with ammonia were added, whilst ice-cooling and the mixture was stirred at room temperature for 30 minutes. The mixture was then concentrated by evaporation under reduced pressure, and the resulting residue was mixed with a 10% w/v aqueous solution of sodium bicarbonate. The crystals which precipitated were separated by filtration. These crystals were washed with diethyl ether to give 5.1 g of $N^{\alpha}$-benzyloxycarbonyl-$N^{\epsilon}$-t-butoxycarbonyl-L-lysinamide as white crystals.

Without purification, 2.8 g (10.0 mmole) of this amide were mixed with 20 ml of a 4N solution of hydrogen chloride in dioxane, and then the mixture was stirred at room temperature for 30 minutes to remove the t-butoxycarbonyl group The reaction mixture was then evaporated to dryness under reduced pressure. 1.7 g (7.4 mmole) of N-t-butoxycarbonyl-L-leucine, 40 ml of methylene chloride and 1.78 g (17.8 mmole) of triethylamine were added, in that order, to the resulting residue, and then 1.45 g (9.1 mmole) of diethyl cyanophosphonate was added, whilst ice-cooling, to the resulting suspension; the mixture was then stirred at room temperature for 3 hours. At the end of this time, the reaction mixture was concentrated by evaporation under reduced pressure, and the residue was mixed with a 10% w/v aqueous solution of sodium bicarbonate. The resulting crystals were filtered off and washed with diethyl ether, to give 3.5 g of $N^{\epsilon}$-(N-t-butoxycarbonyl-L-leucyl)-$N^{\alpha}$-benzyloxycarbonyl-L-lysinamide as white crystals.

Without further purification, 20 ml of a 4N solution of hydrogen chloride in dioxane were added to 2.3 g (4.5 mmole) of this amide, and the mixture was stirred at room temperature for 30 minutes to remove the t-butoxycarbonyl group. The mixture was then evaporated to dryness under reduced pressure. 1.24 g (4.5 mmole) of N-t-butoxycarbonylstatine, 30 ml of methylene chloride and 1.08 g (1.07 mmole) of triethylamine were added, in that order, to the residue. 0.88 g (5.4 mmole) of diethyl cyanophosphonate were added, whilst ice-cooling, to the resulting suspension. The mixture was then stirred at room temperature for 3 hours, after which it was concentrated by evaporation under reduced pressure. The residue was mixed with a 10% w/v aqueous solution of sodium bicarbonate, separated by filtration and washed with diethyl ether, to give 1.90 g of $N^{\epsilon}$-(N-t-butoxycarbonyl-statyl-L-leucyl)-$N^{\alpha}$-benzyloxycarbonyl-L-lysinamide as white crystals.

9(d) $N^{\epsilon}$-{N-[2-(RS)-(1-Naphthyl)methyl-3-(3-pyridyl)propionyl]-3-(4-thiazolyl)-DL-alanyl-statyl-L-leucyl}-N $^{\alpha}$-benzyloxycarbonyl-L-lysinamide

A solution of 0.46 g (1 mmole) of N-[2(RS)-(1-naphthyl)methyl-3-(3-pyridyl)propionyl]-3-(4-thiazolyl)-DL-alanine hydrazide [prepared as described in step (b) above] dissolved in 10 ml of dimethylformamide was cooled at -5°C, and then 0.85 ml of a 4N solution of hydrogen chloride in dioxane and 0.18 ml of isoamyl nitrite were added to it. The mixture was stirred at the same temperature for 10 minutes and was then neutralized by the addition of 0.35 g (3.5 mmole) of N -methylmorpholine. A solution of $N^{\epsilon}$-(statyl-L-leucyl)-$N^{\alpha}$-t-butoxycarbonyl-L-lysinamide hydrochloride [which was prepared from 0.63 g (1 mmole) of $N^{\epsilon}$-(N-t-butoxycarbonyl-statyl-L-leucyl)-$N^{\alpha}$-benzyloxycarbonyl-L-lysinamide, synthesized as described in step -(c) above, by treatment with a 4N solution of hydrogen chloride in dioxane to remove the t-butoxycarbonyl group] dissolved in 5 ml of dimethylformamide and 0.1 g (1 mmole) of N-methylmorpholine were added to the resulting solution, and the mixture was stirred at 4°C for 2 days. The mixture was then diluted with twice its own volume of ethyl acetate and washed successively with 1N aqueous sulphuric acid, with a 5% w/v aqueous solution of sodium bicarbonate and with a saturated aqueous solution of sodium chloride. The mixture was then dried over anhydrous sodium sulphate, and the solvent was distilled off under reduced pressure. The residue was purified by flash column chromatography (eluted with a 10:1 by volume mixture of methylene chloride and methanol), to give 0.53 g of $N^{\epsilon}$-{N-[2(RS)-(1-naphthyl)methyl-3-(3-pyridyl)-propionyl]-3-(4-thiazolyl)-DL-alanyl-statyl-L-leucyl}-$N^{\alpha}$-benzyloxycarbonyl-L-lysinamide, melting at 140 - 145°C.

Elemental analysis:
Calculated for $C_{53}H_{68}N_8O_8S.1/2H_2O$:
C, 64.55%; H, 7.05%; N, 11.36%;
S, 3.25%.
Found: C, 64.55%; H, 7.14%; N, 11.36%;
S, 3.25%.

9(e) $N^{\epsilon}$-{N-[2(RS)-(1-Naphthyl)methyl-3-(3-pyridyl)propionyl]-3-(4-thiazolyl)-DL-alanyl-statyl-L-leucyl}-L-lysinamide dihydrofluoride

A solution of 0.53 g of $N^{\epsilon}$-{N-[2(RS)-(1-naphthyl)methyl-3-(3-pyridyl)propionyl]-3-(4-thiazolyl)-DL-alanyl-statyl-L-leucyl}-$N^{\alpha}$-benzyloxycarbonyl-L-lysinamide [prepared as described in step (d) above] dissolved in 10 ml of hydrofluoric acid was stirred for 30 minutes, whilst ice-cooling, in the presence of a small amount of anisole as a scavenging agent for cations. The mixture was then evaporated to dryness under reduced pressure. The resulting residue was broken up in the presence of diethyl ether and separated by filtration to give 0.5 g of the title compound as white crystals, melting at 150-155°C.

EXAMPLE 10

$N^{\epsilon}$-{N-[2(R)-Benzyl-1,4-dioxo-4-morpholinobutyl]-3-(4-thiazolyl)-DL-alanyl-statyl-L-leucyl}-L-lysinamide dihydrofluoride

10(a) Methyl N-[2(R)-benzyl-1,4-dioxo-4-morpholinobutyl]-3-(4-thiazolyl)-DL-alanate

1.05 ml (6.92 mmole) of diethyl cyanophosphonate and 2.66 ml (1.91 mmole) of triethylamine were added to a solution of 1.60 g (5.77 mmole) of (2R)-2-benzyl-4-morpholino-4-oxobutyric acid (prepared as described in preparation 7) and 1.79 g (6.91 mmole) of methyl 3-(4-thiazolyl)-DL-alanate dihydrochloride in 50 ml of tetrahydrofuran, whilst ice-cooling and under an atmosphere of nitrogen, and the mixture was stirred at the same temperature for 3 hours. The solvent was then stripped off by evaporation under reduced pressure, and the residue was purified by column chromatography through silica gel (eluted with a 20:1 by volume mixture of chloroform and methanol), to give 2.10 g (82%) of the title compound as a colourless oil.

Mass spectrum, m/e: 445 ($M^+$).

10(b) N-[2(R)-Benzyl-1,4-dioxo-4-morpholinobutyl]-3-(4-thiazolyl)-DL-alanine hydrazide

1.59 g (31.4 mmole) of hydrazine monohydrate was added to a solution of 2.00 g (4.49 mmole) of methyl N-[2( R)-benzyl-1,4-dioxo-4-morpholinobutyl]-3-(4-thiazolyl)-DL-alanate [prepared as described in step (a) above] dissolved in 15 ml of methanol, and the mixture was stirred at room temperature for 5 hours. At the end of this time, the solvent was distilled off under reduced pressure, and the resulting residue was washed with diethyl ether and then dried, to give 1.70 g (85%) of the title compound.

Mass spectrum, m/e: 445 ($M^+$).

10(c) $N^{\epsilon}$-{N-[2(R)-Benzyl-1,4-dioxo-4-morpholinobutyl]-3-(4-thiazolyl)-DL-alanyl-statyl-L-leucyl}-$N^{\alpha}$-benzyloxycarbonyl-L-lysinamide

A solution of 0.31g (0.7 mmole) of N-[2(R)-benzyl-1,4-dioxo-4-morpholinobutyl]-3-(4-thiazolyl)-DL-alanine hydrazide [prepared as described in step (b) above] dissolved in 8 ml of dimethylformamide was cooled to -60°C, and 0.60 ml of a 4N solution of hydrogen chloride in dioxane and 0.11 ml of isoamyl nitrite were added to it. The mixture was stirred at -20° for 10 minutes, cooled again to -60°C and neutralized by the addition of 0.30 ml (2.7 mmole) of N -methylmorpholine. $N^{\epsilon}$-(statyl-L-leucyl)-$N^{\alpha}$-benzyloxycarbonyl-L-lysinamide hydrochloride was prepared from 0.59 g (0.87 mmole) of $N^{\epsilon}$-(N-t-butoxycarbonyl-statyl-L-leucyl)-$N^{\alpha}$-benzyloxycarbonyl-L-lysinamide [synthesized as described in Example 9(c)] by treatment with a 4N solution of hydrogen chloride in dioxane to remove the t-butoxycarbonyl group. A solution of the whole of this compound in 5 ml of dimethylformamide and 0.10 ml (0.91 mmole) of N-methylmorpholine were added to the reaction mixture, which was then stirred at 4°C overnight. The reaction mixture was then diluted with twice its volume of ethyl acetate, washed successively with 1N aqueous sulphuric acid, with a 5% w/v aqueous solution of sodium bicarbonate and with a saturated aqueous solution of sodium chloride and dried over anhydrous sodium sulphate. The solvent was then stripped off by evaporation under reduced pressure. The residue was purified by silica gel preparative thin layer chromatography (developing solvent; a 10:1 by

volume mixture of chloroform and methanol), to give 250 mg (37%) of $N^{\epsilon}$-{N-[2(R)-benzyl-1,4-dioxo-4-morpholinobutyl]-3-(4-thiazolyl)-DL-alanyl-statyl-L-leucyl}-$N^{\alpha}$-benzyloxycarbonyl-L-lysinamide trihydrate, melting at 98-100°C.

Elemental analysis:
Calculated for $C_{49}H_{70}N_8O_{10}S.3H_2O$:
C, 57.85%; H, 7.53%; N, 11.02%;
S, 3.15%.
Found: C, 57.94%; H, 7.41% N, 10.81%;
S, 3.06%.

10(d) $N^{\epsilon}$-{N-[2(R)-Benzyl-1,4-dioxo-4-morpholinobutyl]-3-(4-thiazolyl)-DL-alanyl-statyl-L-leucyl}-L-lysinamide dihydrofluoride

140 mg (0.14 mmole) of $N^{\epsilon}$-{N-[2(R)-benzyl-1,4-dioxo-4-morpholinobutyl]-3-(4-thiazolyl)-DL-alanyl-statyl-L-leucyl}-$N^{\alpha}$-benzyloxycarbonyl-L-lysinamide trihydrate [prepared as described in step (c) above] were treated with anisole and hydrofluoric acid for 30 minutes, and then the reaction mixture was concentrated by evaporation under reduced pressure. The residue was washed with hexane, and addition of diethyl ether gave crystals, which were filtered off, yielding 90 mg (75%) of the title compound, melting at 156-159°C.

EXAMPLE 11

$N^{\epsilon}$-{N-[2(R)-Benzyl-1,4-dioxo-4-morpholinobutyl]-3-(4-thiazolyl)-DL-alanyl-statyl-L-isoleucyl}-L-lysinamide dihydrofluoride

$N^{\epsilon}$-( N-t-butoxycarbonyl-statyl-L-isoleucyl)-N $^{\alpha}$-benzyloxycarbonyl-L-lysinamide was prepared by the procedure described in Example 9(c), but replacing the t-butoxycarbonyl-L-leucine by the same amount of t-butoxycarbonyl-L-isoleucine. The t-butoxycarbonyl group was then removed from this by reaction with a 4N solution of hydrogen chloride in dioxane, in the usual way, and the resulting $N^{\epsilon}$-(statyl-L-isoleucyl)-$N^{\alpha}$-benzyloxycarbonyl-L-lysinamide was reacted with N-[2(R)-benzyl-1,4-dioxo-4-morpholinobutyl]-3-(4-thiazolyl)-DL-alanine hydrazide [prepared as described in Example 10(b)], following the procedure described in Example 10(c). This reaction gave $N^{\epsilon}$-{N-[2(R)-benzyl-1,4-dioxo-4-morpholinobutyl]-3-(4-thiazolyl)-DL-alanyl-statyl-L-isoleucyl}-$N^{\alpha}$-benzyloxycarbonyl-L-lysinamide monohydrate, melting at 184-187°C. This compound was then treated as described in Example 10(d), to give the title compound, melting at 137-142°C.

EXAMPLE 12

$N^{\alpha}$-[N-Nicotinoyl-3-(1-naphthyl)-L-alanyl-L-glutaminyl-statyl]-L-lysinamide hydrochloride trihydrate

The procedure described in Examples 2 (a)-(g) was repeated, with the following exceptions:

(i) In Example 2(a), the N-t-butoxycarbonyl-L-leucine was replaced by N-t-butoxycarbonyl-5-glutamine; and

(ii) In Example 2(f), the $N^{\epsilon}$-benzyloxycarbonyl-L-lysinol was replaced by $N^{\epsilon}$-benzyloxycarbonyl-L-lysinamide, to give the title compound, melting at 54-55°C. Elemental analysis:
Calculated for $C_{38}H_{52}N_8O_7.HCl.3H_2O$:
C, 55.43%; H, 7.22%; N, 13.61%, Cl, 4.31%.
Found: C, 55.42%; H, 7.42%; N, 13.89%; Cl, 4.59%.

EXAMPLE 13

$N^{\alpha}$-[N-Nicotinoyl-3-(1-naphthyl)-L-alanyl-L-glutaminyl-statyl]-L-lysinol dihydrochloride trihydrate

The procedure described in Examples 2 (a)-(g) was repeated, except that, in Example 2(a), the N -t-butoxy carbonyl-L-leucine was replaced by N-t-butoxycarbonyl-5-glutamine, to give the title compound, melting at 120-124°C.

Elemental Analysis:
Calculated for $C_{38}H_{53}N_7O_7.2HCl.3H_2O$;
C, 53.90%; H, 7.26%; N, 11.58%; Cl, 8.37%.
Found: C, 53.69%; H, 7.55%; N, 11.32%; Cl, 8.57%.

EXAMPLE 14

$N^{\epsilon}$-[N-Nicotinoyl-3-(1-naphthyl)-L-alanyl-L-histidyl-statyl]-L-lysinol trihydrochloride hydrate

(a) $N^{\epsilon}$-[N-benzyloxycarbonyl-3-(1-naphthyl)-L-alanyl-L-histidyl-statyl]-$N^{\alpha}$-t-butoxycarbonyl-L-lysinol was prepared by the procedure described in Examples 4 (a) and (b) except that, in Example 4(b), the ethyl $N^{\alpha}$-t-butoxycarbonyl-L-lysinate hydrochloride was replaced by $N^{\alpha}$-t-butoxycarbonyl-L-lysinol.

(b) The resulting compound was then subjected to the procedure described in Examples 2 (c) and (d), except that dicyclohexylcarbodiimide was used as a condensation reagent in Example 2(d), and then the resulting compound was treated in the usual way with a solution of hydrogen chloride in dioxane, to give the title compound, melting at 134-138°C.

EXAMPLE 15

$N^{\epsilon}$-[N-Isonicotinoyl-3-(1-naphthyl)-L-alanyl-L-histidyl-statyl]-L-lysinol trihydrochloride hydrate

The procedure decribed in Example 14 was repeated, except that 4-pyridinecarboxylic acid (isonicotinic acid) was used in place of nicotinic acid in the step corresponding to Example 2(d), to give the title compound, melting at 137-140°C.

EXAMPLE 16

$N^{\epsilon}$-[N-picolinoyl-3-(1-naphthyl)-L-alanyl-L-histidyl-statyl]-L-lysinol trihydrochloride hydrate

The procedure decribed in Example 14 was repeated, except that 2-pyridinecarboxylic acid (picolinic acid) was used in place of nicotinic acid in the step corresponding to Example 2 (d), to give the title compound, melting at 141-145°C.

EXAMPLE 17

Methyl $N^{\epsilon}$-[N-nicotinoyl-3-(1-naphthyl)-L-alanyl-L-histidyl-statyl]-L-lysinate 2.5 hydrate

The procedure describe in Example 4(b) was repeated, except that the ethyl $N^{\alpha}$-t-butoxycarbonyl-L-lysinate was replaced by the corresponding methyl ester, to give methyl $N^{\epsilon}$-[N-benzyloxycarbonyl-3-(1-naphthyl)-L-alanyl-L-histidyl-statyl]-L-lysinate. This was then treated as described in Examples 2 (c) and (d), but using a mixture of dicyclohexylcarbodiimide and N-hydroxy-5-norbornene-2,3-dicarboximide as the condensation reagent, to give the title compound, melting at 119-123°C.

Elemental Analysis:

Calculated for $C_{40}H_{52}N_8O_7.2.5H_2O$:
C, 59.91%; H, 7.16%; N, 13.97%.
Found: C, 60.18%; H, 6.68%; N, 13.55%.

EXAMPLE 18

$N^{\alpha}$[N-Nicotinoyl-3-(1-naphthyl)-L-alanyl-L-histidyl-statyl]-L-lysinamide trihydrochloride tetrahydrate

The procedure described in Example 4(b) was repeated, but using $N^{\epsilon}$-t-butoxycarbonyl-L-lysinamide in place of the ethyl N $^{\alpha}$-t-butoxycarbonyl-L-lysinate, to give $N^{\alpha}$-[N-benzyloxycarbonyl-3-(1-naphthyl)-L-alanyl-L-histidyl-statyl]-L-lysinamide. This was then treated as described in Examples 2(c) and 2(d), but using a mixture of dicyclohexylcarbodiimide and N-hydroxy-5-norbornene-2,3-dicarboximide as the condensation agent, to give the title compound as an amorphous powder.

Elemental Analysis:
Calculated for $C_{39}H_{51}N_9O_6.3HCl.4H_2O$:
C, 50.73%; H, 6.77%; N, 13.65%.
Found: C, 50.48%; H, 6.21%; N, 13.38%.

EXAMPLE 19

$N^{\alpha}$-[N-t-Butoxycarbonyl-3-(1-naphthyl)-L-alanyl-N-methyl-L-leucyl-statyl]-L-lysinol hydrochloride sesquihydrate

The procedure described in Examples 2(a) and 2(b) was repeated, except that, in Example 2(a), the N-t-butoxycarbonyl-L-leucine was replaced by N -t-butoxycarbonyl-N-methyl-L-leucine and that, in Example 2-(b), the N-benzyloxycarbonyl-3-(1-naphthyl)-L-alanine was replaced by N-t-butoxycarbonyl-3-(1-naphthyl)-L-alanine, to give methyl N-t-butoxycarbonyl-3-(1-naphthyl)-L-alanyl-N-methyl-L-leucyl-statinate. This was then treated as described in Examples 2(e), 2(f) and 2(g), to give the title compound, melting at 118-120°C.

Elemental Analysis:
Calculated for $C_{39}H_{63}N_5O_7.HCl.1.5H_2O$:
C, 60.25%; H, 8.69%; N, 9.01%; Cl, 4.56%.
Found: C, 60.41%; H, 8.72%; N, 8.92%; Cl, 4.56%.

EXAMPLE 20

$N^{\alpha}$-[N-Nicotinoyl-3-(1-naphthyl)-L-alanyl-3-(4-thiazolyl)-DL-alanyl-(2R,3S)-norstatyl]-L-lysinamide trihydrochloride dihydrate

N-t-butoxycarbonyl-3-(1-naphthyl)-L-alanyl-3-(4-thiazolyl)-DL-alanine hydrazide and methyl (2R,3S)-norstatinate were reacted together and treated as described in the first and second steps of Example 4(a), to give methyl N-t-butoxycarbonyl-3-(1-naphthyl)-L-alanyl-3-(4-thiazolyl)-DL-alanyl-norstatinate. The t-butoxycarbonyl group was removed by treatment with a solution of hydrogen chloride in dioxane, and then the product was reacted with nicotinic acid, following the procedure of Example 2(d), to give methyl N-nicotinoyl-3-(1-naphthyl)-L-alanyl-3-(4-thiazolyl)-DL-alanyl-norstatinate. This was then reacted with hydrazine, as described in the final step of Example 4(a), and the product was then treated as described in Example 4-(b), but replacing the ethyl $N^{\alpha}$-t-butoxycarbonyl-L-lysinate by $N^{\epsilon}$-t-butoxycarbonyl-L-lysinamide, to give the title compound, melting at 92-96°C.

Elemental Analysis:
Calculated for $C_{38}H_{48}N_8O_6S.3HCl.2H_2O$:
C, 51.26%; H, 6.23%; N, 12.59%; S, 3.60%;

Cl, 11.95%.
Found: C, 51.11%; H, 6.21%; N, 12.25%; S, 3.96%;
Cl, 12.20%.

EXAMPLE 21

Ethyl $N^{\epsilon}$-[N-benzyloxycarbonyl-3-(1-naphthyl)-L-alanyl-L-histidyl-statylamino]-$N^{\alpha}$-t-butoxycarbonyl-L-lysinate

The title compound, melting at 152-154°C, was prepared from N-benzyloxycarbonyl-3-(1-naphthyl)-L-alanyl-L-histidine hydrazide and ethyl $N^{\epsilon}$-statyl-$N^{\alpha}$-t-butoxycarbonyl-L-lysinate by the procedure described in the first and second steps of Example 4(a).

EXAMPLE 22

$N^{\alpha}$-{N-[2-(2-Methoxyethoxy)ethoxycarbonyl]-3-(1-naphthyl)-L-alanyl-L-leucyl-statyl}-L-lysinol hydrochloride

The title compound, melting at 122-128°C, was prepared by: reacting N-[2-(2-methoxyethoxy)-ethoxycarbonyl]-3-(1-naphthyl)-L-alanine and benzyl L-leucinate by the procedure described in Example 2-(f); removing the benzyl group by the procedure described in Example 2(g); reacting the product with $N^{\alpha}$-statyl-$N^{\epsilon}$-benzyloxycarbonyl-L-lysinol, following the procedure described in Example 2(f); and finally removing the benzyloxycarbonyl group by the procedure described in Example 2(g).

EXAMPLE 23

Methyl N $^{\epsilon}$-[N-(5-amino-2-nitrobenzoyl)-3-(1-naphthyl)-L-alanyl-L-histidyl-statyl]-L-lysinate trihydrochloride

The title compound, melting at 175-180°C, was prepared from methyl $N^{\epsilon}$-[3-(1-naphthyl)-L-alanyl-L-histidyl-statyl]-$N^{\alpha}$-t-butoxycarbonyl-L-lysinate and 2-nitro-5-aminobenzoic acid by the procedure described in Example 2(d), followed by treatment with a solution of hydrogen chloride in dioxane to remove the t-butoxycarbonyl group.

EXAMPLE 24

$N^{\epsilon}$-[N-(5-Amino-2-nitrobenzoyl)-3-(1-naphthyl)-L-alanyl-L-histidyl-statyl]-L-lysine trihydrochloride

Following the procedure described in Example 2(e), methyl $N^{\epsilon}$-[ N-(5-amino-2-nitrobenzoyl)-3-(1-naphthyl)-L-alanyl-L-histidyl-statyl]-L-lysinate trihydrochloride (prepared as described in Example 23) was hydrolyzed, to give the title compound, melting at 180-185°C.

EXAMPLE 25

$N^{\alpha}$-[N-(Morpholinoacetyl-3-(1-naphthyl)-L-alanyl-L-leucyl-statyl]-L-lysinol dihydrochloride hydrate

The procedure described in Examples 2(d), 2(e), 2(f) and 2(g) was repeated, but replacing the nicotinic acid in Example 2(d) by morpholinoacetic acid, to give the title compound, melting at 100-105°C.

Elemental analysis:
Calculated for $C_{39}H_{62}N_6O_7.2HCl.H_2O$:
C, 57.27%; H, 8.13%; N, 10.28%; Cl, 8.67%.
Found: C, 57.63%; H, 8.06%: N, 9.59%; Cl, 9.00%.

EXAMPLE 26

$N^{\alpha}$[N-(Morpholinoacetyl)-3-(1-napthyl)-L-alanyl-L-glutaminyl-statyl]-L-lysinamide sesquihydrochloride sesquihydrate

The procedure described in Examples 2(d), 2(e), 2(f) and 2(g) was repeated, except that the starting materials in Example 2(d) were methyl 3-(1-naphthyl)-L -alanyl-L-glutaminyl-statinate and morpholinoacetic acid and that the $N^{\epsilon}$-benzyloxycarbonyl-L -lysinol employed in Example 2(f) was replaced by $N^{\epsilon}$-benzyloxycarbonyl-L-lysinamide, to give the title compound, melting at 86-88°C.

Elemental analysis:
Calculated for $C_{38}H_{58}N_8O_8.1\frac{1}{2}HCl.1\frac{1}{2}H_2O$:
C, 54.55%; H, 7.53%; N, 13.39%: Cl, 6.36%.
Found: C, 54.45%, H, 7.73%; N, 13.15%; Cl, 6.07%.

EXAMPLE 27

$N^{\alpha}$-[N-(Morpholinoacetyl)-3-(1-naphthyl)-L-alanyl-L-glutaminyl-statyl]-L-lysinol tetrahydrochloride

The procedure described in Examples 2(d), 2(e), 2(f) and 2(g) was repeated, except that the starting materials employed in Example 2(d) were methyl 3-(1-naphthyl)-L-alanyl-L-glutaminyl-statinate and morpholinoacetic acid, to give the title compound, melting at 123-126°C.

Elemental analysis:
Calculated for $C_{38}H_{59}N_7O_8.4HCl$:
C, 51.41%; H, 7.15%; N, 11.04%; Cl, 15.97%.
Found: C, 51.70%; H, 7.45%; N, 10.81%; Cl, 16.22%.

EXAMPLE 28

$N^{\epsilon}$-{N-[(RS)-Bis(1-naphthylmethyl)acetyl]-L-isoleucyl-statyl}-L-lysine 1.3 trifluoroacetate hydrate

The procedure described in Examples 5(a) and 5(b) was repeated, except that the starting materials in Example 5(a) were bis(1-naphthylmethyl)acetic acid and benzyl L-isoleucinate. The resulting N-[bis(1-naphthylmethyl)acetyl]-L-isoleucinate was then reacted with t-butyl $N^{\epsilon}$-statyl-$N^{\alpha}$-t-butoxycarbonyl-L-lysinate, following the procedure described in Example 5(a), and then the product was treated with trifluoroacetic acid to remove the t-butyl and t-butoxycarbonyl groups and yield the title compound, melting at 68-69°C.

Elemental analysis:
Calculated for $C_{44}H_{58}N_4O_6.1.3\ CF_3CO_2H.H_2O$:
C, 61.83%; H, 6.83%; N, 6.19%; F, 8.19%.
Found: C, 61.64%; H, 7.05%; N, 6.18%; F, 8.36%.

EXAMPLE 29

$N^{\alpha}$-{N-[(RS)-Bis(1-naphthylmethyl)acetyl]-L-isoleucyl-statyl}-L-lysine sesquitrifluoroacetate hemihydrate

The title compound, melting at 142-144°C, was prepared from N-[bis(1-naphthylmethyl)acetyl]-L-isoleucine and t-butyl $N^{\alpha}$-statyl-$N^{\epsilon}$-t-butoxycarbonyl-L-lysinate by the procedure described in Example 5(a), and then the product was treated with trifluoroacetic acid in the presence of anisole to remove the t-butoxycarbonyl and t-butyl groups.

Elemental analysis:
Calculated for

$C_{44}H_{58}N_4O_6.1\frac{1}{2}CF_3CO_2H.\frac{1}{2}H_2O$:
C, 61.42%; H, 6.64%; N, 6.10%; F, 9.30%.
Found: C, 61.45%; H, 6.70%; N, 6.08%; F, 9.25%.

EXAMPLE 30

N$^{\epsilon}$-[N-(Morpholinoacetyl)-3-(1-naphthyl)-L-alanyl-3-(4-thiazolyl)-DL-alanyl-statyl]-L-lysinol trihydrochloride trihydrate

Methyl 3-(1-naphthyl)-L-alanyl-3-(4-thiazolyl)-DL-alanyl-statinate and morpholinoacetic acid were reacted together by the procedure described in Example 2(d). The product was then reacted with hydrazine by the procedure described in the third step of Example 4(a), and the resulting hydrazide was then treated as described in Example 4(b), but replacing the ethyl N$^{\alpha}$-t-butoxycarbonyl-L-lysinate by N$^{\alpha}$-t-butoxycarbonyl-L-lysinol, to give the title compound, melting at 92-96°C.

Elemental analysis:
Calculated for $C_{38}H_{48}N_8O_6S.3HCl.3H_2O$:
C, 49.75%; H, 7.03%; N, 10,69%; S, 3.49%;
Cl, 11.59%.
Found: C, 49.20%; H, 6.85%; N, 10.52%; S, 3.15%;
Cl, 11.76%.

EXAMPLE 31

N$^{\alpha}$-{N-[Bis(1-naphthylmethyl)acetyl]-L-leucyl-statyl}-L-lysine trifluoroacetate

The title compound, melting at 130-135°C, was prepared from N-[bis(1-naphthylmethyl)acetyl]-L-leucine and t-butyl N$^{\alpha}$-statyl-N$^{\epsilon}$-t-butoxycarbonyl-L-lysinate by the procedure described in Example 5(c), and then the product was treated with trifluoroacetic acid to remove the t-butoxycarbonyl and t-butyl groups.

EXAMPLE 32

N$^{\epsilon}$-{N-[Bis(1-naphthylmethyl)acetyl]-L-leucyl-statyl}-L-lysine trifluoroacetate

The title compound, melting at 125-130°C, was prepared from N-[bis(1-naphthylmethyl)acetyl]-L-leucine and t-butyl N$^{\epsilon}$-statyl-N$^{\alpha}$-t-butoxycarbonyl-L-lysinate by the procedure described in Example 5(c), and then the product was treated with trifluoroacetic acid to remove the t-butoxycarbonyl and t-butyl groups.

EXAMPLE 33

N-[2-(2-Aminoethylamino)ethyl]-{N-[bis(1-naphthylmethyl)acetyl]-L-histidyl-statin}amide diacetate

The title compound, melting at 129-132°C, was prepared from N-[bis(1-naphthylmethyl)acetyl]-L-histidyl-statine hydrazide and diethylene triamine by the procedure described in the first and second steps of Example 4(a).

Elemental analysis:
Calculated for $C_{42}H_{53}N_7O_4.2CH_3COOH$:
C, 65.77%; H, 7.32%; N, 11.67%.
Found: C, 65.35%; H, 6.84%; N, 11.37%.

EXAMPLE 34

N$^{\epsilon}$-{N-[2-(1-Naphthylmethyl)-6-phenylhexanoyl]-L-leucyl-statyl}-L-lysine hemihydrate

N-Benzyloxycarbonyl-L -leucine and t-butyl N$^{\epsilon}$-statyl-N$^{\alpha}$-t-butoxycarbonyl-L-lysinate were reacted together and treated by the procedures described in Examples 2(f) and 2(g). The product was then reacted with 2-(1-naphthylmethyl)-6-phenylhexanoic acid, following the procedure described in Example 2(d), and then the resulting product was treated with hydrogen fluoride to remove the t-butoxycarbonyl and t-butyl groups, and give the title compound, melting at 150-160°C.

Elemental analysis:
Calculated for $C_{43}H_{62}N_4O_6.\frac{1}{2}H_2O$:
C, 70.17%; H, 8.63%; N, 7.61%.
Found: C, 70.19%; H, 8.54%; N, 7.58%.

EXAMPLE 35

N$^{\alpha}$-[N-Nicotinoyl-3-(1-naphthyl)-L-alanyl-L-leucyl-statyl]-L-lysinamide dihydrochloride sesquihydrate

The title compound, melting at 138-141°C, was prepared from N$^{\alpha}$-t-butoxycarbonyl-N$^{\epsilon}$-benzyloxy carbonyl-L-lysinamide and N-nicotinoyl-3-(1-naphthyl)-L-alanyl-L-leucyl-statine by the procedure described in Examples 2(b) and 2(c).

Elemental analysis:
Calculated for $C_{39}H_{55}N_7O_6.2HCl.\ 1.5H_2O$:
C, 57.28%; H, 7.39%; N, 11.99%; Cl, 8.67%.
Found: C. 57.57%; H, 7.29%; N, 11.43%; Cl, 8.73%.

EXAMPLE 36

N$^{\epsilon}$-[N-Nicotinoyl-3-(1-naphthyl)-L-alanyl-L-leucyl-statyl]-L-lysinol dihydrochloride dihydrate

The title compound, melting at 93-95°C, was prepared from N$^{\epsilon}$-[3-(1-naphthyl)-L-alanyl-L-leucyl-statyl]-N$^{\alpha}$-t-butoxycarbonyl-L-lysinol and nicotinic acid by the procedure described in Example 2(d), followed by treatment with a solution of hydrogen chloride in dioxane to remove the t-butoxycarbonyl group.

Elemental analysis:
Calculated for $C_{39}H_{56}N_6O_6.2HCl.2H_2O$:
C, 57.56%; H, 7.68%; N, 10.33%; Cl, 8.71%.
Found: C, 57.73%: H, 7.56%; N, 10.04%, Cl, 8.99%.

EXAMPLE 37

N-(m-Aminomethylbenzyl)-[N-nicotinoyl-3-(1-naphthyl)-L-alanyl-L-leucyl-statin]amide dihydrochloride

N-Nicotinoyl-3-(1-naphthyl)-L-alanyl-L-leucyl-statine and mono-t-butoxycarbonyl-m-xylylene diamine (which was prepared from m-xylylene diamine and di-t-butyl carbonate) were reacted together by the procedure described in Example 2(f), and then the product was treated with a solution of hydrogen chloride in dioxane to remove the t-butoxycarbonyl group and give the title compound, melting at 144-148°C.

EXAMPLE 38

$N^{\epsilon}$-[N-Benzyloxycarbonyl-3-(1-naphthyl)-L-alanyl-L-isoleucyl-statyl]-L-lysine 1.2 trifluoroacetate hydrate

The title compound, melting at 180-183°C, was prepared by:

(a) reacting N-benzyloxycarbonyl-3-(1-naphthyl)-L-alanine and benzyl isoleucinate as described in Examples 2(f) and 2(c);

(b) reacting the product of step (a) with t-butyl $N^{\epsilon}$-statyl-$N^{\alpha}$-t-butoxycarbonyl-L-lysinate by the procedure described in Example 2(f); and

(c) treating the product of step (b) with trifluoroacetic acid in the presence of anisole to remove the t-butoxycarbonyl and t-butoxy groups. Elemental analysis:

Calculated for $C_{41}H_{57}N_5O_8$. $1.2CF_3CO_2H.H_2O$:

C, 57.74%; H, 6.72%; N, 7.76%; F, 7.58%.

Found: C, 57.56%; H, 6.76%; N, 7.78%; F, 7.80%.

EXAMPLE 39

$N^{\epsilon}$-[N-Nicotinoyl-3-(1-naphthyl)-L-alanyl-3-(4-thiazolyl)-DL-alanyl-statyl]-L-lysinol trihydrochloride trihydrate

(a) The procedure described in the first and second steps of Example 4(a) was repeated, except that the hydrazide used as starting material was the hydrazide of N -t-butoxycarbonyl-3-(1-naphthyl)-L-alanyl-3-(4-thiazolyl)-DL-alanine. The product was then treated with a solution of hydrogen chloride in dioxane to remove the t-butoxycarbonyl group and give methyl 3-(1-naphthyl)-L-alanyl-3-(4-thiazolyl)-DL-alanyl-statinate.

(b) The compound prepared as in step (a) above was then reacted with nicotinic acid by the procedure described in Example 2(d): the product was treated with hydrazine as in the third step of Example 4(a): and the resulting hydrazide was reacted with $N^{\alpha}$-t-butoxycarbonyl-L -lysinol by the procedure described in Example 4(b), to give the title compound, melting at 94-98°C.

Elemental analysis:

Calculated for $C_{39}H_{51}N_7O_6S.3HCl.3H_2O$:

C, 51.51%; H, 6.65%; N, 10.78%; S, 3.53%;
Cl, 11.70%.

Found: C, 50 89%; H, 6.94%; N, 10.58%; S, 3.20%;
Cl, 11.30%.

EXAMPLE 40

$N^{\epsilon}$-[N-picolinoyl-3-(1-naphthyl)-L-alanyl-L-leucyl-statyl]-L-lysinol dihydrochloride tetrahydrate

Methyl N-benzyloxycarbonyl-3-(1-naphthyl)-L-alanyl-L-leucyl-statinate [prepared as described in Example 2(b) above] was treated as described in Example 2(e) to convert the carboxy terminal end to the free carboxylic acid. This was then reacted with $N^{\alpha}$-t-butoxycarbonyl-L-lysinol by the procedure described in Example 2(f), the benzyloxycarbonyl group was removed by the procedure described in Example 2(g), and then the product was reacted with 2-pyridinecarboxylic acid following the procedure described in Example 2(f). The product was then treated with a solution of hydrogen chloride in dioxane to remove the t-butoxycarbonyl group and give the title compound, melting at 88-90°C.

Elemental analysis:

Calculated for $C_{39}H_{56}N_6O_6.2HCl.4H_2O$:

C, 54.99%; H, 8.06%; N, 9.86%; Cl, 8.32%.

Found: C, 54.70%; H, 7.81%; N, 9.64%; Cl, 8.60%.

EXAMPLE 41

$N^{\epsilon}$-[N-Isonicotinoyl-3-(1-naphthyl)-L-alanyl-L-leucyl-statyl]-L-lysinol dihydrochloride dihydrate

The title compound, melting at 90-92°C, was prepared from $N^{\epsilon}$-[3-(1-naphthyl)-L-alanyl-L-leucyl-statyl]-$N^{\alpha}$-t-butoxycarbonyl-L-lysinol and isonicotinic acid by the procedure described in Example 2(f), followed by treatment with a solution of hydrogen chloride in dioxane to remove the t-butoxycarbonyl group.

Elemental analysis:
Calculated for $C_{39}H_{56}N_6O_6.2HCl.2H_2O$:
C, 57.56%; H, 7.68%; N, 10.33%; Cl, 8.71%.
Found: C, 57.40%; H, 7.54%; N, 10.16%; Cl, 9.01%.

EXAMPLE 42

α-{N-[Bis(1-naphthylmethyl)acetyl]-L-leucyl-statylamino}-ε-aminopimelic acid hydrochloride hydrate

The procedure described in Example 5(e) was repeated, except that the $N^{\epsilon}$-benzyloxycarbonyl-L-lysinamide was replaced by α,ε-dimethyl α-amino-ε-t-butoxycarbonylaminopimelate, and then the product was treated, in turn, with sodium hydroxide and with a solution of hydrogen chloride in dioxane, to give the title compound, melting at 140-150°C.

Elemental analysis:
Calculated for $C_{45}H_{58}N_4O_8.HCl.H_2O$:
C, 64.54%; H, 7.34%; N, 6.69%.
Found: C, 64.28%; H, 7.29%; N, 6.46%.

EXAMPLE 43

$N^{\alpha}$-{N-[Bis(1-naphthylmethyl)acetyl]-L-leucyl-statyl}-L-lysinol hydrochloride

The procedure described in Examples 2(f) and 2(g) was repeated, except that the statine derivative used as starting material in Example 2(f) was replaced by N-[bis(1-naphthylmethyl)acetyl]-L-leucyl-statine - [prepared as decribed in Example 5(d)] to give the title compound, melting at 105-110°C.

Elemental analysis:
Calculated for $C_{44}H_{60}N_4O_5.HCl$:
C, 69.40%; H, 8.08%; N, 7.36%;
Cl, 4.66%.
Found: C, 69.41%; H, 8.24%; N, 7.24%;
Cl, 4.02%.

EXAMPLE 44

$N^{\alpha}$-[N-Nicotinoyl-3-(1-naphthyl)-L-alanyl-N-methyl-L-histidyl-statyl]-$N^{\epsilon}$-t-butoxycarbonyl-L-lysinamide

The title compound, melting at 125-130°C, was prepared by removing the benzyloxycarbonyl group from $N^{\alpha}$-(N6-benzyloxycarbonyl-statyl)-$N^{\epsilon}$-t-butoxycarbonyl-L-lysinamide by the procedure described in Example 2(c) and then reacting the resulting product with N-nicotinoyl-3-(1-naphthyl)-L-alanyl-N-methyl-L-histidine hydrazide by the procedure described in Example 4(b).

EXAMPLE 45

$N^{\alpha}$-[N-Nicotinoyl-3-(1-naphthyl)-L-alanyl-N-methyl-L-histidyl-statyl]-L-lysinamide dihydrochloride

The title compound, melting at 185-190°C, was prepared by treating $N^{\alpha}$-[N-nicotinoyl-3-(1-naphthyl)-L-alanyl-N-methyl-L-histidyl-statyl]-$N^{\epsilon}$-t-butoxycarbonyl-L-lysinamide (prepared as described in Example 44) with a solution of hydrogen chloride in dioxane to remove the t-butoxycarbonyl group.

EXAMPLE 46

$N^{\epsilon}$-[N-Nicotinoyl-3-(1-naphthyl)-L-alanyl-N-methyl-3-(4-thiazolyl)-DL-alanyl-statyl]-$N^{\alpha}$-benzyloxycarbonyl-L-lysinamide

The title compound, melting at 110-114°C, was prepared by reacting $N^{\epsilon}$-[3-(1-naphthyl)-L-alanyl-N-methyl-3-(4-thiazolyl)-DL-alanyl-statyl]-$N^{\alpha}$-benzyloxycarbonyl-L-lysinamide and nicotinic acid by the procedure described in Example 2(d).

EXAMPLE 47

$N^{\epsilon}$-[N-Nicotinoyl-3-(1-naphthyl)-L-alanyl-N-methyl-3-(4-thiazolyl)-DL-alanyl-statyl]-L-lysinamide trihydrochloride

The title compound, melting at 166-170°C, was prepared from $N^{\epsilon}$-[N -nicotinoyl-3-(1-naphthyl)-L-alanyl-N-methyl-3-(4-thiazolyl)-DL-alanyl-statyl]-$N^{\alpha}$-benzyloxycarbonyl-L-lysinamide (prepared as described in Example 46) by catalytic hydrogenation using palladium black as the catalyst, followed by treatment with hydrochloric acid.

EXAMPLE 48

$N^{\alpha}$-{(3S,4S)-4-[N-Nicotinoyl-3-(1-naphthyl)-L-alanyl-3-(4-thiazolyl)-DL-alanylamino]-5-cyclohexyl-3-hydroxypentanoyl}-L-lysinamide

(a) N-t-Butoxycarbonyl-3-(1-naphthyl)-L-alanyl-3-(4-thiazolyl)-DL-alanine hydrazide and methyl -(3S,4S)-4-amino-5-cyclohexyl-3-hydroxypentanoate can be reacted as described in the first and second steps of Example 4(a) and the product treated with a solution of hydrogen chloride in dioxane to remove the t-butoxycarbonyl group and give methyl (3S,4S)-4-[3-(1-naphthyl)-L-alanyl-3-(4-thiazolyl)-DL-alanylamino]-5-cyclohexyl-3-hydroxy pentanoate.

(b) The title compound can be prepared by reacting the compound prepared as described in step (a) above with nicotinic acid by the procedure described in Example 2(d), treating the product with hydrazine as described in the third step of Example 4(a) and then reacting the product with $N^{\epsilon}$-t-butoxycarbonyl-L-lysinamide by the procedure described in Example 4(b).

EXAMPLE 49

$N^{\epsilon}$-{(3S,4S)-4-[N-Nicotinoyl-3-(1-naphthyl)-L-alanyl-3-(4-thiazolyl)-DL-alanylamino]-5-cyclohexyl-3-hydroxypentanoyl}-L-lysinamide

(a) Reaction of N-t-butoxycarbonyl-3-(1-naphthyl)-L-alanyl-3-(4-thiazolyl)-DL-alanine hydrazide with methyl (3S,4S)-4-amino-5-cyclohexyl-3-hydroxypentanoate by the procedure described in the first and second steps of Example 4(a), followed by treatment with hydrogen chloride in dioxane and reaction with nicotinic acid by the procedure described in Example 2(d) can give methyl (3S,4S)-4-[N-nicotinoyl-3-(1-naphthyl)-L-alanyl-3-(4-thiazolyl)-DL-alanylamino]-5-cyclohexyl-3-hydroxypentanoate.

(b) Reaction of the compound prepared as described in step (a) above with hydrazine as described in the third step of Example 4(a), followed by treatment as described in Example 4(b) but replacing the ethyl $N^{\epsilon}$-t-butoxycarbonyl-L-lysinate by $N^{\alpha}$-t-butoxycarbonyl-L-lysinamide can give the title compound.

EXAMPLE 50

$N^{\epsilon}$-{(3S,4S)-4-(N-Nicotinoyl-3-(1-naphthyl)-L-alanyl-3-(4-thiazolyl)-DL-alanylamino]-5-cyclohexyl-3-hydroxypentanoyl}-L-lysinol

The title compound can be prepared by the procedure described in Example 48, but replacing the $N^{\epsilon}$-t-butoxycarbonyl-L-lysinamide by $N^{\alpha}$-t-butoxycarbonyl-L-lysinol.

EXAMPLE 51

$N^{\epsilon}$-{(3S,4S)-4-(N-Nicotinoyl-3-(1-naphthyl)-L-alanyl-L-histidylamino]-5-cyclohexyl-3-hydroxypentanoyl}-L-lysinamide

The title compound can be prepared by following the procedure described in Example 49 but replacing the N-t-butoxycarbonyl-3-(1-naphthyl)-L-alanyl-3-(4-thiazolyl)-DL-alanine hydrazide by N-t-butoxycarbonyl-3-(1-naphthyl)-L-alanyl-L-histidine hydrazide.

EXAMPLE 52

$N^{\epsilon}$-{(3S,4S)-4-[N-Nicotinoyl-3-(1-naphthyl)-L-alanyl-3-(5-isoxazolyl)-L-alanylamino]-5-cyclohexyl-3-hydroxypentanoyl}-L-lysinamide

The title compound can be prepared by following the procedure described in Example 49, but using N-t-butoxycarbonyl-3-(1-naphthyl)-L-alanyl-3-(5-isoxazolyl)-L-alanine hydrazide as the hydrazide.

EXAMPLE 53

$N^{\epsilon}$-{(3S,4S)-4-[N-Nicotinoyl-3-(1-naphthyl)-L-alanyl-N-methyl-3-(4-thiazolyl)-DL-alanylamino]-5-cyclohexyl-3-hydroxypentanoyl}-L-lysinamide

The title compound can be prepared by following the procedure described in Example 49, but using N-t-butoxycarbonyl-3-(1-naphthyl)-L-alanyl-N-methyl-3-(4-thiazolyl)-DL-alanine hydrazide as the hydrazide.

EXAMPLE 54

(3S,4S)-N-(m-Aminomethylbenzyl)-4-[N-nicotinoyl-3-(1-naphthyl)-L-alanyl-3-(4-thiazolyl)-DL-alanylamino]-5-cyclohexyl-3-hydroxypentanamide

The title compound can be prepared by hydrolyzing methyl (3S,4S)-4-[N-nicotinoyl-3-(1-naphthyl)-L-alanyl-3-(4-thiazolyl)-DL -alanylamino]-5-cyclohexyl-3-hydroxypentanoate [prepared as described in Example 49(a)] to give (3S,4S)-4-[N -nicotinoyl-3-(1-naphthyl)-L-alanyl-3-(4-thiazolyl)-DL-alaylamino]-5-cyclohexyl-3-hydroxypentanoic acid, reacting this with mono-t-butoxycarbonyl-m-xylylene diamine by the procedure described in Example 2(f), and then treating the product with hydrogen chloride in dioxane to remove the t-butoxycarbonyl group.

PREPARATION 1

$N^{\alpha}$-t-Butoxycarbonyl-$N^{\epsilon}$-benzyloxycarbonyl-L-lysinol

9.0 g (0.24 mmole) of sodium borohydride were added to a solution of 10.0 g (0.24 mmole) of lithium chloride dissolved in 340 ml of a 3:2 by volume mixture of ethanol and tetrahydrofuran, and the mixture was stirred for 30 minutes at room temperature. A solution of 31.6 g (0.08 mmole) of methyl $\underline{N}^{\alpha}$-t-butoxycarbonyl-$\underline{N}^{\epsilon}$-benzyloxycarbonyl-L-lysinate dissolved in 160 ml of a 3:2 by volume mixture of ethanol and tetrahydrofuran was added, and the resulting mixture was stirred at room temperature for one day. 20 ml of acetone were added to the mixture, which was then stirred for 10 minutes, after which it was concentrated by evaporation under reduced pressure. The residue was dissolved in water and then extracted with benzene. The benzene extracts were washed once with a saturated aqueous solution of sodium chloride, dried over anhydrous sodium sulphate and concentrated by evaporation under reduced pressure. The residue was recrystallized from a mixture of ethyl acetate and hexane to give 11.0 g of the title compound as white needles, melting at 65-67°C.

$[\alpha]^{25}$ -11.0° (C=0.3, methanol).

PREPARATION 2

Diethyl (1-naphthyl)methyl-(3-pyridyl)methylmalonate

A solution of 9.0 g (30 mmole) of diethyl (1-naphthyl)methylmalonate in 10 ml of dimethylformamide was added dropwise, whilst ice-cooling, to a solution of sodium ethoxide [which was prepared by dissolving 0.69 g (30 mmole) of sodium in 20 ml of ethanol and then evaporating the solution to dryness] in 20 ml of dimethylformamide. The reaction mixture was stirred for minutes at the same temperature, and then at room temperature for 1 hour. 3-Picolyl chloride was prepared by adding 4.92 g (30 mmole) of picolyl chloride hydrochloride to a cooled solution of sodium ethoxide prepared from 0.69 g of metallic sodium and 20 ml of anhydrous ethanol, stirring the resulting mixture at room temperature for 30 minutes and then evaporating it to dryness under reduced pressure. A solution of the whole of this 3-picolyl chloride dissolved in 20 ml of dimethylformamide was then added to the reaction mixture. The mixture was then stirred for 1 hour whilst ice-cooling, and then for 8 hours at 50-60°C. The reaction mixture was freed from dimethylformamide by distilling it off under reduced pressure, and 100 ml of water was added to the residue, which was then extracted with ethyl acetate. The organic extracts were washed with water, dried over anhydrous magnesium sulphate and concentrated by evaporation under reduced pressure. The residue was purified by column chromatography through silica gel (eluted with a 3:1 by volume mixture of hexane and ethyl acetate). Concentration of the fractions containing the desired compound gave 5.9 g (50.2%) of the title compound, as an oil.

Elemental analysis:
Calculated for $C_{24}H_{25}NO_4$:
C, 73.64%; H, 6.44%; N, 3.58%.
Found: C, 73.65%; H, 6.46%; N, 3.57%.

PREPARATION 3

2(RS)-(1-Naphthyl)methyl-3-(3-pyridyl)propionic acid

5.9 g (15 mmole) of diethyl (1-naphthyl)methyl-(3-pyridyl)methylmalonate (prepared as described in preparation 2) were added to a solution of 5.9 g (89 mmole) of 85% potassium hydroxide in a mixture of 40 ml of water and 40 ml of butanol and the mixture was heated under reflux for 7 hours. The mixture was then freed from butanol by evaporation under reduced pressure, after which it was acidified with concentrated aqueous hydrochloric acid and then adjusted to a pH value of 6-7 by the addition of a 2N aqueous sodium hydroxide solution. The resulting precipitate was removed by filtration and purified by column chromatography through silica gel (eluted with a 9:1 by volume mixture of chloroform and methanol). Concentration of the fractions containing the desired compound by evaporation under reduced pressure gave 2.04 g -

(46.7%) of the title compound, melting at 157-158°C.

Elemental analysis:
Calculated for $C_{19}H_{17}NO_2$:
C, 78.33%; H, 5.88%; N, 4.81%.
Found: C, 78.33%; H, 6.02%; N, 4.72%.

PREPARATION 4

N-3-phenylpropionyl-(S)-(-)-4-benzyl-2-oxazolidinone

In an atmosphere of nitrogen, a solution of 77 mmole of butyllithium in hexane was added dropwise to a solution of 12.41 g (70 mmole) of (S)-(-)-4-benzyl-2-oxazolidinone in 200 ml of anhydrous tetrahydrofuran at -78°C, and then the mixture was stirred for 30 minutes. A solution of 13.0 g (77 mmole) of 3-phenylpropionyl chloride in 100 ml of anhydrous tetrahydrofuran was slowly added dropwise to the mixture at the same temperature, and the mixture was stirred for 1 hour. At the end of this time, the mixture was mixed with a saturated aqueous solution of sodium chloride and extracted with ethyl acetate. The organic extracts were dried over anhydrous magnesium sulphate and concentrated by evaporation under reduced pressure. The residue was purified by column chromatography through silica gel (eluted with a 1:4 by volume mixture of ethyl acetate and hexane) and recrystallized from a mixture of ethyl acetate and hexane, to give 16.9 g (78%) of the title compound as white crystals, melting at 92-95°C.

Elemental analysis:
Calculated for $C_{19}H_{19}NO_3$:
C, 73.77%; H, 6.19%; N, 4.53%.
Found: C, 73.57%; H, 6.28%; N, 4.41%.

Mass spectrum, m/e: 309 ($M^+$).

PREPARATION 5

(2R)-N-[3-Benzyloxycarbonyl-2-benzyl-1-oxopropyl]-(S)-(-)-4-benzyl-2-oxazolidinone

In an atmosphere of nitrogen, 2.91 ml (20.8 mmole) of diisopropylamine and 13.40 ml of a solution of butyllithium in hexane were added to 50 ml of anhydrous tetrahydrofuran, and the mixture was stirred for 30 minutes. A solution of 5.35 g (17.3 mmole) of N-(3-phenylpropionyl)-(S)-(-)-4-benzyl-2-oxazolidinone - (prepared as described in preparation 4) in 20 ml of anhydrous tetrahydrofuran was added to the mixture, which was then stirred for 1 hour at the same temperature. At the end of this time, a solution of 5.74 ml - (36.2 mmole) of benzyl bromoacetate dissolved in 10 ml of anhydrous tetrahydrofuran was added dropwise to the mixture, and stirring was continued for 3 hours. At the end of this time, a saturated aqueous solution of sodium chloride was added to the reaction mixture, which was then extracted with ethyl acetate. The organic extracts were dried over anhydrous magnesium sulphate, and the solvent was evaporated off under reduced pressure. The residue was purified by medium pressure column chromatography through silica gel (eluted with a 1:5 by volume mixture of ethyl acetate and hexane), to afford 5.47 g (69%) of the title compound as a colourless oil.

Mass spectrum, m/e: 457 ($M^+$).

PREPARATION 6

(2R)-N-[2-Benzyl-1,4-dioxo-4-morpholinobutyl]-(S)-(-)-4-benzyl-2-oxazolidinone

5.10 g (11.1 mmole) of (2R)-N -[3-benzyltoxycarbonyl-2-benzyl-1-oxopropyl]-(S)-(-)-4-benzyl-2-oxazolidinone (prepared as described in preparation 5) were dissolved in 100 ml of ethanol, and the solution was stirred for 3 hours at room temperature in an atmosphere of hydrogen and in the presence of 500 mg of 10% w/w palladium-on-carbon. At the end of this time, the catalyst was filtered off and the filtrate was concentrated by evaporation under reduced pressure. The residue was dissolved in 50 ml of tetrahydrofuran, and then 1.16 ml (13.3 mmole) of morpholine, 2.02 ml (13.3 mmole) of diethyl cyanophosphonate and 1.86 ml (13.3 mmole) of triethylamine were added, in that order, to the resulting solution, under a nitrogen atmosphere and whilst ice-cooling, and the mixture was stirred for a further 2 hours. At the end of this time, the solvent was stripped from the reaction mixture by evaporation under reduced pressure. The residue was purified by medium pressure column chromatography through silica gel (eluted with a 1:1 by volume mixture of ethyl acetate and hexane), to afford 3.90 g (80%) of the title compound as white crystals, melting at 56-59°C

Elemental analysis:
Calculated for $C_{25}H_{28}N_2O_5$:
C, 68.79%; H, 6.47%; N, 6.42%.
Found: C, 68.59%; H, 6.75%; N, 6.50%.

Mass spectrum, m/e: 436 ($M^+$).

PREPARATION 7

(2R)-2-Benzyl-4-morpholino-4-oxobutyric acid

3.70 g (8.5 mmole) of (2R)-N-(2-benzyl-1,4-dioxo-4-morpholinobutyl]-(S)-(-)-4-benzyl-2-oxazolidinone (prepared as described in preparation 6) were dissolved in a mixture of 80 ml of tetrahydrofuran and 30 ml of water, and then 711 mg (16.9 mmole) of lithium hydroxide monohydrate were added to the mixture, whilst ice-cooling. The mixture was then stirred at the same temperature for 3 hours. At the end of this time, the reaction mixture was freed from tetrahydrofuran by distillation under reduced pressure, and a 10% w/v aqueous solution of sodium hydroxide was added to the residue. The resulting mixture was then extracted with methylene chloride. The aqueous layer was adjusted to a pH value of 1, whilst ice-cooling, by the addition of concentrated aqueous hydrochloric acid and extracted with methylene chloride. The extracts were combined and dried over anhydrous magnesium sulphate. The solvent was then distilled off under reduced pressure, to afford 1.75 g (75%) of the title compound as a white oil.

Mass spectrum, m/e: 277 ($M^+$).

**Claims**

1. Compounds of formula (I):

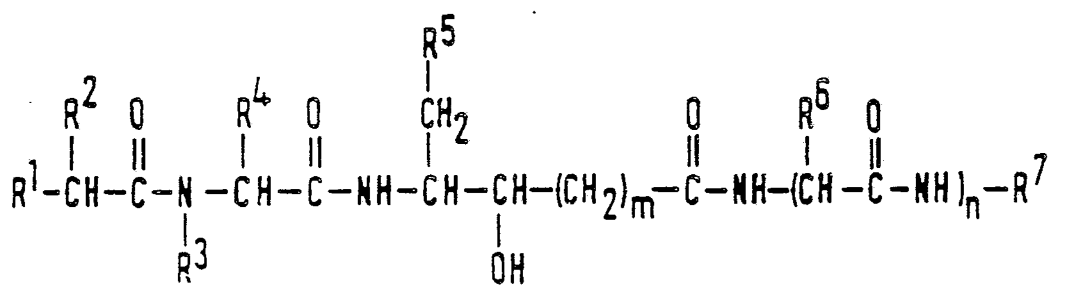
(I)

wherein:
m is the cypher 0 or the integer 1;
n is the cypher 0 or the integer 1;

$R^1$ represents a group of formula $-A-R^8$,
wherein: A represents a $C_1$-$C_8$ alkylene group; and $R^8$ represents an aryl group, a heterocyclic group, a $C_2$-$C_5$ aliphatic acyl group, a heterocyclic acyl group or an alkoxyalkoxy group in which each alkoxy part is $C_1$-$C_4$,

a group of formula $-CONR^9R^{10}$,
wherein $R^9$ and $R^{10}$ are the same or different and each represents a hydrogen atom, a $C_1$-$C_4$ alkyl group or a $C_1$-$C_4$ alkyl group having at least one of substituents (a), defined below,

or a group of formula $-NHCOR^{11}$,
wherein $R^{11}$ represents a $C_1$-$C_{10}$ alkyl group, a $C_1$-$C_{10}$ alkyl group having at least one of substituents (b), defined below, an aryl group, a heterocyclic group, a $C_1$-$C_4$ alkoxy group, a $C_1$-$C_4$ alkoxy group having at least one of substituents (c), defined below, an aralkyloxy group or an aryloxy group,
provided that, when both m = 1 and n = 1, $R^1$ represents said group of formula $-A-R^8$ or said group of formula $-CONR^9R^{10}$;

$R^2$ represents a group of formula $-B-R^{12}$,
wherein B represents a $C_1$-$C_{10}$ alkylene group and $R^{12}$ represents an aryl group or a heterocyclic group;

$R^3$ represents a hydrogen atom or a $C_1$-$C_4$ alkyl group;

$R^4$ represents a $C_1$-$C_{10}$ alkyl group, a $C_1$-$C_{10}$ alkyl group having at least one of substituents (d), defined below, a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

$R^5$ represents an isopropyl group, a $C_3$-$C_8$ cycloalkyl group or a phenyl group;

$R^6$ represents a $C_1$-$C_6$ alkyl group;

$R^7$ represents a $C_1$-$C_{10}$ alkyl group having at least one of substituents (e), defined below, or a $C_1$-$C_{10}$ alkyl group having at least one of substituents (e) and at least one of substituents (f), defined below;

said aryl groups and the aryl parts of said aryloxy, aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one of substituents (d) and/or (g) and/or (h), defined below;

the alkyl parts of said aralkyloxy, aralkylthio, aralkyloxycarbonylamino and aralkyloxycarbonyl groups have from 1 to 4 carbon atoms;

said heterocyclic groups have from 5 to 14 ring atoms, of which from 1 to 5 are nitrogen and/or oxygen and/or sulphur hetero-atoms, and are unsubstituted or have at least one of substituents (d) and/or (g) and/or (h) and/or (i), defined below;

substituents (a):carboxy groups, $C_2$-$C_5$ alkoxycarbonyl groups, carbamoyl groups, alkylcarbamoyl groups where the alkyl part is $C_1$-$C_4$ and dialkylcarbamoyl groups where each alkyl part is $C_1$-$C_4$;

substituents (b):$C_1$-$C_4$ alkoxy groups, alkoxyalkoxy groups in which each alkoxy part is $C_1$-$C_4$ alkoxy, halogen atoms, $C_2$-$C_5$ alkoxycarbonyl groups, aralkyloxycarbonyl groups, aryl groups, heterocyclic groups, $C_1$-$C_7$ aliphatic carboxylic acyl groups, aromatic carboxylic acyl groups, heterocyclic carboxylic acyl groups and aryloxy groups;

substituents (c):$C_1$-$C_4$ alkoxy groups and alkoxyalkoxy groups where each alkoxy part is $C_1$-$C_4$;

substituents (d):hydroxy groups, $C_1$-$C_4$ alkoxy groups, aryloxy groups, aralkyloxy groups where the alkyl part is $C_1$-$C_4$, $C_1$-$C_7$ aliphatic carboxylic acyloxy groups, $C_1$-$C_4$ alkylthio groups, arylthio groups, aralkylthio groups where the alkyl part is $C_1$-$C_4$, $C_1$-$C_4$ alkylsulphinyl groups, $C_1$-$C_4$ alkylsulphonyl groups, arylsulphinyl groups, arylsulphonyl groups, $C_1$-$C_7$ aliphatic carboxylic acylamino groups, aromatic carboxylic acylamino groups, $C_2$-$C_7$ alkoxycarbonylamino groups, aralkyloxycarbonylamino groups, $C_2$-$C_7$ alkoxycarbonyl groups, aryloxycarbonyl groups, aralkyloxycarbonyl groups, $C_1$-$C_7$ aliphatic carboxylic acyl groups, aromatic carboxylic acyl groups, heterocyclic carboxylic acyl groups, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di-and tri-halomethyl groups, mercapto groups, amino groups, $C_1$-$C_4$ alkylamino groups, dialkylamino groups where each alkyl part is $C_1$-$C_4$, carboxy groups, guanidino groups and guanidino groups having at least one $C_1$-$C_4$ alkyl substituent;

substituents (e): amino groups, protected amino groups, aminoalkylamino groups where the alkyl part is $C_1$-$C_4$ and one or both amino parts may be unsubstituted or have a $C_1$-$C_4$ alkyl substituent, aminoalkylaminoalkylamino groups where each alkyl part is $C_1$-$C_4$ and any amino part may be unsubstituted or have a $C_1$-$C_4$ alkyl substituent, guanidino groups, guanidino groups having at least one $C_1$-$C_4$ alkyl substituent, phenyl groups substituted by at least one $C_1$-$C_4$ aminoalkyl substituent, phenyl groups substituted by at least one substituted $C_1$-$C_4$ hydroxyalkoxy group said hydroxyalkoxy group having at least one of substituents (j), defined below, and sulpho groups;

substituents (f):hydroxy groups, carboxy groups, $C_2$-$C_5$ alkoxycarbonyl groups, carbamoyl groups, alkylcarbamoyl groups where the alkyl part is $C_1$-$C_4$ and dialkylcarbamoyl groups where each alkyl part is $C_1$-$C_4$;

substituents (g):hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

substituents (h): $C_1$-$C_4$ alkyl groups, nitro groups, cyano groups, $C_3$-$C_5$ alkenoyl groups, $C_3$-$C_5$ alkenoyl groups having at least one of substituents (d) and $C_1$-$C_4$ alkyl groups having at least one of substituents (g);

substituents (i):oxygen atoms; and

substituents (j):amino groups, $C_1$-$C_4$ alkylamino groups, dialkylamino groups where each alkyl part is $C_1$-$C_4$, heterocyclic groups and heterocyclic-substituted $C_1$-$C_4$ alkylamino groups;

and pharmaceutically acceptable salts thereof.

2. Compounds as claimed in Claim 1, wherein:

m is the cypher 0 or the integer 1;

n is the cypher 0 or the integer 1;

$R^1$ represents a group of formula $-A-R^8$ wherein A represents a $C_1$-$C_4$ alkylene group and $R^8$ represents an aryl group, an aromatic heterocyclic group, a $C_2$-$C_5$ aliphatic carboxylic acyl group or a heterocyclic carboxylic acyl group,

an alkylcarbamoyl group in which the alkyl part is $C_1$-$C_4$ alkyl, a dialkylcarbamoyl group in which each alkyl part is $C_1$-$C_4$ alkyl, a substituted alkylcarbamoyl group in which the alkyl part is $C_1$-$C_4$ alkyl and has at least one carboxy substituent, a substituted dialkylcarbamoyl group in which each alkyl part is $C_1$-$C_4$ alkyl and in which at least one of said alkyl parts has at least one carboxy substituent, or a group of formula $-NHCOR^{11}$ wherein $R^{11}$ represents a $C_1$-$C_4$ alkyl group, a $C_7$-$C_9$ aralkyl group, a $C_1$-$C_4$ alkyl group having a heterocyclic substituent, an aryl group, a heterocyclic group, a $C_1$-$C_4$ alkoxy group, an alkoxyalkoxyalkoxy group in which each alkoxy part is $C_1$-$C_4$ or a $C_7$-$C_9$ aralkyloxy group,
provided that, when both $m = 1$ and $n = 1$, $R^1$ represents said group of formula $-A-R^8$ or said alkylcarbamoyl, dialkylcarbamoyl, substituted alkylcarbamoyl or substituted dialkylcarbamoyl group,

$R^2$ represents a group of formula $-B-R^{12}$, in which B represents a $C_1$-$C_4$ alkylene group and $R^{12}$ represents an aryl group or an aromatic heterocyclic group;

$R^3$ represents a hydrogen atom or a methyl group;

$R^4$ represents a hydrogen atom, a $C_1$-$C_6$ alkyl group, a $C_1$-$C_6$ alkyl group having at least one of substituents (k), a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^6$ represents a $C_1$-$C_6$ alkyl group;

$R^7$ represents a substituted $C_2$-$C_6$ alkyl group having at least one of substituents (m) or a substituted $C_2$-$C_6$ alkyl group having at least one of substituents (m) and at least one of substituents (f), defined in Claim 1;

said heterocyclic groups have 5 or 6 ring atoms, of which from 1 to 3 are nitrogen and/or oxygen and/or sulphur hetero-atoms, and are unsubstituted or have at least one of substituents (d) and/or (g) and/or (h) and/or (i), defined in Claim 1;

substituents (k): hydroxy groups, $C_1$-$C_4$ alkoxy groups, $C_1$-$C_4$ alkylthio groups, $C_1$-$C_7$ aliphatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, $C_3$-$C_6$ cycloalkyl groups, aryl groups, aromatic heterocyclic groups, mercapto groups, amino groups, alkylamino groups in which the alkyl part is $C_1$-$C_4$ alkyl, dialkylamino groups in which each alkyl part is $C_1$-$C_4$ alkyl, carboxy groups, guanidino groups, alkylguanidino groups in which the alkyl part is $C_1$-$C_4$ and dialkylguanidino groups in which each alkyl part is $C_1$-$C_4$;

substituents (m): amino groups, protected amino groups, $C_1$-$C_4$ aminoalkylamino groups, aminoalkylaminoalkylamino groups in which each alkyl part is $C_1$-$C_4$,
guanidino groups, alkylguanidino groups in which the alkyl part is $C_1$-$C_4$, dialkylguanidino groups in which each alkyl part is $C_1$-$C_4$, substituted phenyl groups having at least one of substituents (n) and sulpho groups; and

substituents (n): $C_1$-$C_4$ aminoalkyl groups and di-substituted $C_1$-$C_4$ alkoxy groups, in which one substituent is the hydroxy group and the other is selected from amino groups, $C_1$-$C_4$ alkylamino groups, dialkylamino groups in which each alkyl part is $C_1$-$C_4$ and heterocyclic groups.

3. Compounds as claimed in Claim 1, wherein:

m is 1;

n is 0;

$R^1$ represents a group of formula -A-$R^8$ in which A represents a $C_1$ or $C_2$ alkylene group and $R^8$ represents a phenyl group, a naphthyl group or a nitrogen-containing aromatic heterocyclic group,

an alkylcarbamoyl group in which the alkyl part is $C_1$-$C_4$ alkyl, a dialkylcarbamoyl group in which each alkyl part is $C_1$-$C_4$ alkyl, a carboxyalkylcarbamoyl group in which the alkyl part is $C_1$-$C_4$ alkyl, or a group of formula -NHCOR$^{11}$
in which $R^{11}$ represents a $C_1$-$C_4$ alkyl group, a $C_1$-$C_4$ alkyl group having a heterocyclic substituent, a phenyl group, a phenyl group having at least one of substituents (d) and/or (g) and/or (h) defined in Claim 1, a nitrogen-containing heterocyclic group or a $C_7$-$C_9$ aralkyloxy group;

$R^2$ represents a group of formula -B-$R^{12}$, in which B represents a $C_1$ or $C_2$ alkylene group, and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ alkyl and the phenyl group is unsubstituted or has at least one of substituents (d) and/or (g) and/or (h) defined in Claim 1, a $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^7$ represents a substituted $C_1$-$C_6$ alkyl group having at least one of substituents (o) or a substituted $C_2$-$C_6$ alkyl group having at least one of substituents (o) and at least one of substituents (p);

said heterocyclic groups have 5 or 6 ring atoms of which 1 or 2 are nitrogen and/or oxygen and/or sulphur hetero-atoms, and are unsubstituted or have at least one of substituents (d) and/or (g) and/or (h) and/or (i), defined in Claim 1;

substituents (o): amino groups, protected amino groups, guanidino groups and phenyl groups having a $C_1$ or $C_2$ aminoalkyl substituent; and

substituents (p): hydroxy groups, carboxy groups, $C_2$-$C_5$ alkoxycarbonyl groups and carbamoyl groups.

4. Compounds as claimed in Claim 1, wherein:

m is 1;

n is 1;

$R^1$ represents a group of formula $-A-R^8$ in which A represents a $C_1$ or $C_2$ alkylene group and $R^8$ represents a phenyl group, a naphthyl group or a nitrogen-containing aromatic heterocyclic group, an alkylcarbamoyl group in which the alkyl part is $C_1$-$C_4$, a dialkylcarbamoyl group in which each alkyl part is $C_1$-$C_4$ or a carboxyalkylcarbamoyl group in which the alkyl part is $C_1$-$C_4$;

$R^2$ represents a group of formula $-B-R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h) defined in Claim 1, a $C_1$-$C_6$ alkyl group having an aromatic heterocyclic substituent, a $C_3$ or $C_4$ alkenyl group or a $C_3$-$C_4$ alkynyl group;

$R^5$ represents an isopropyl group, a cyclopentyl group, a cyclohexyl group or a phenyl group;

$R^6$ represents a $C_3$ or $C_4$ alkyl group;

$R^7$ represents a substituted $C_2$-$C_6$ alkyl group having at least one of substituents (o) or a substituted $C_2$-$C_6$ alkyl group having at least one of substituents (o) and at least one of substituents (p);

said heterocyclic groups have 5 or 6 ring atoms, of which 1 or 2 are nitrogen and/or oxygen and/or sulphur hetero-atoms, said heterocyclic group being unsubstituted or having at least one of substituents (d) and/or -(g) and/or (h) defined in Claim 1;

substituents (o): amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

5. Compounds as claimed in Claim 1, wherein:

m and n are both 0;

$R^1$ represents a group of formula $-A-R^8$, in which A represents a $C_1$ or $C_2$ alkylene group, and $R^8$ represents a phenyl group, a naphthyl group or a nitrogen-containing aromatic heterocyclic group, an alkylcarbamoyl group in which the alkyl part is $C_1$-$C_4$, a dialkylcarbamoyl group in which each alkyl part is $C_1$-$C_4$, a carboxyalkylcarbamoyl group in which the alkyl part is $C_1$-$C_4$ or a group of formula $-NHCOR^{11}$, in which $R^{11}$ represents a $C_1$-$C_4$ alkyl group, a $C_1$-$C_4$ alkyl group having a heterocyclic substituent, a phenyl group a phenyl group having at least one of substituents (d) and/or (g) and/or (h) defined in Claim 1, a nitrogen-containing heterocyclic group or a $C_7$-$C_9$ aralkyloxy group;

$R^2$ represents a group of formula $-B-R^{12}$, in which B represents a $C_1$ or $C_2$ alkylene group and represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h) defined in Claim 1, $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a cyclopentyl group, a cyclohexyl group or a phenyl group;

$R^7$ represents a substituted $C_2$-$C_6$ alkyl group having at least one of substituents (o) or a substituted $C_2$-$C_6$ alkyl group having at least one of substituents (o) and at least one of substituents (p);

said heterocyclic groups and said heterocyclic substituents have 5 or 6 ring atoms of which 1 or 2 are nitrogen and/or oxygen and/or sulphur hetero-atoms, and are unsubstituted or have at least one of substituents (d) and/or (g) and/or (h), defined in Claim 1;

substituents (o):amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

6. Compounds as claimed in Claim 1, wherein:

m is 1;

n is 0;

$R^1$ represents a group of formula -A-$R^8$
in which A represents a $C_1$ or $C_2$ alkylene group and $R^8$ represents a phenyl group, a naphthyl group or a nitrogen-containing aromatic heterocyclic group,
an alkylcarbamoyl group in which the alkyl part is $C_1$-$C_4$ alkyl, a dialkylcarbamoyl group in which each alkyl part is $C_1$-$C_4$ alkyl or a group of formula -NHCOR$^{11}$,
in which $R^{11}$ represents a $C_1$-$C_4$ alkyl group, a $C_1$-$C_4$ alkyl group having a heterocyclic substituent or a nitrogen-containing heterocyclic group;

$R^2$ represents a group of formula -B-$R^{12}$ in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_3$ or $C_4$ alkyl group, a phenylalkyl group in which the alkyl part is $C_1$ or $C_2$ alkyl and the phenyl part is unsubstituted or has at least one of substituents (d) and/or (g) and/or (h) defined in Claim 1, a $C_1$ or $C_2$ alkyl group having an aromatic heterocyclic substituent, a $C_3$ or $C_4$ alkenyl group or a $C_3$ or $C_4$ alkynyl group;

$R^5$ represents an isopropyl group or a cyclohexyl group;

$R^7$ represents a substituted $C_2$-$C_6$ alkyl group having at least one substituent selected from amino groups and protected amino groups or a substituted $C_2$-$C_6$ alkyl group having at least one substituent selected from amino groups and protected amino groups and at least one of substituents (p); and

substituents (p):hydroxy groups, carboxy groups, $C_2$-$C_5$ alkoxycarbonyl groups and carbamoyl groups.

7. Compounds as claimed in Claim 1, wherein:

m is 1;

n is the cypher 0 or the integer 1;

$R^1$ represents a group of formula -A-$R^8$,
wherein A represents a $C_1$-$C_4$ alkylene group and $R^8$ represents an aryl group or an aromatic heterocyclic group having 5 or 6 ring atoms,

or a group of formula $-NHCOR^{11}$,
wherein $R^{11}$ represents a $C_1$-$C_4$ alkyl group, a $C_7$-$C_9$ aralkyl group, a $C_1$-$C_4$ alkyl group having a heterocyclic substituent wherein the heterocyclic substituent has 5 or 6 ring atoms, an aryl group, an aromatic heterocyclic group having from 5 to 10 ring atoms, a $C_1$-$C_4$ alkoxy group or a $C_7$-$C_9$ aralkyloxy group, provided that, when both $\underline{m}=1$ and $\underline{n}=1$, $R^1$ represents said group of formula $-A-R^8$;

$R^2$ represents a group of formula $-B-R^{12}$, in which B represents a $C_1$-$C_4$ alkylene group, and $R^{12}$ represents an aryl group or an aromatic heterocyclic group having 5 or 6 ring atoms;

$R^3$ represents a hydrogen atom;

$R^4$ represents a hydrogen atom, a $C_1$-$C_6$ alkyl group, a $C_1$-$C_6$ alkyl group having at least one of substituents - (q), a $C_2$-$C_5$ alkenyl group, a $C_2$-$C_5$ alkenyl group having at least one halogen substituent or a $C_2$-$C_5$ alkynyl group;

$R^5$ represents an isopropyl group, a cyclopentyl group, a cyclohexyl group or a phenyl group;

$R^6$ represents a $C_1$-$C_5$ alkyl group;

$R^7$ represents a substituted $C_2$-$C_6$ alkyl group having at least one of substituents (r) or a substituted $C_2$-$C_6$ alkyl group having at least one of substituents (r) and at least one of substituents (f), defined in Claim 1;

said heterocyclic groups and heterocyclic substituents have from 1 to 3 ring nitrogen and/or oxygen and/or sulphur hetero-atoms and are unsubstituted or have at least one of substituents (d) and/or (g) and/or (h) and/or (i), defined in Claim 1;

<u>substituents (q):</u>hydroxy groups, $C_1$-$C_4$ alkoxy groups, $C_7$-$C_9$ aralkyloxy groups, $C_1$-$C_7$ aliphatic carboxylic acyloxy groups, $C_1$-$C_4$ alkylthio groups, $C_7$-$C_9$ aralkylthio groups, $C_1$-$C_4$ alkylsulphinyl groups, $C_1$-$C_4$ alkylsulphonyl groups, $C_1$-$C_7$ aliphatic carboxylic acylamino groups, aromatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, aryloxycarbonyl groups, $C_8$-$C_{10}$ aralkyloxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, thiocarbamoyl groups, alkyl(thiocarbamoyl) groups in which the alkyl part is $C_1$-$C_4$ alkyl, dialkyl(thiocarbamoyl) groups in which each alkyl part is $C_1$-$C_4$, ureido groups, thioureido groups, $C_3$-$C_8$ cycloalkyl groups, $C_5$-$C_8$ cycloalkenyl groups, aryl groups, aromatic heterocyclic groups, halogen atoms, halomethyl groups, mercapto groups, amino groups, $C_1$-$C_4$ alkylamino groups, dialkylamino groups in which each alkyl part is $C_1$-$C_4$ alkyl, carboxy groups and guanidino groups;

<u>substituents (r):</u>amino groups, protected amino groups, $C_1$-$C_4$ aminoalkylamino groups, aminoalkylaminoalkylamino groups in which each alkyl part is $C_1$-$C_4$, guanidino groups, substituted phenyl groups having at least one of substituents (n), and sulpho groups; and

<u>substituents (n):</u>$C_1$-$C_4$ aminoalkyl groups and di-substituted $C_1$-$C_4$ alkoxy groups, in which one substituent is the hydroxy group and the other is selected from amino groups, $C_1$-$C_4$ alkylamino groups, dialkylamino groups in which each alkyl part is $C_1$-$C_4$ and heterocyclic groups.

8. $\underline{N}^{\alpha}$-[$\underline{N}$-Nicotinoyl-3-(1-naphthyl)-alanyl-leucyl-statyl]-lysinamide and pharmaceutically acceptable salts thereof.

9. $\underline{N}^{\epsilon}$-[$\underline{N}$-Nicotinoyl-3-(1-naphthyl)-alanyl-leucyl-statyl]-lysinol and pharmaceutically acceptable salts thereof.

10. $\underline{N}$-($\underline{m}$-Aminomethylbenzyl)-[$\underline{N}$-nicotinoyl-3-(1-naphthyl)-alanyl-leucyl-statin]amide and pharmaceutically acceptable salts thereof.

11. $N^{\epsilon}$-[N-Nicotinoyl-3-(1-naphthyl)-alanyl-3-(4-thiazolyl)-alanyl-statyl]-lysinol and pharmaceutically acceptable salts thereof.

12. $N^{\epsilon}$-[N-Picolinyl-3-(1-naphthyl)-alanyl-3-(5-imidazolyl)-alanyl-statyl]-lysinol and pharmaceutically acceptable salts thereof.

13. $N^{\epsilon}$-[N-Picolinyl-3-(1-naphthyl)-alanyl-leucyl-statyl]-lysinol and pharmaceutically acceptable salts thereof.

14. $N^{\alpha}$-[N-Bis(1-naphthylmethyl)acetyl-leucyl-statyl]-lysinol and pharmaceutically acceptable salts thereof.

15. Ethyl $N^{\epsilon}$-[N-benzyloxycarbonyl-3-(1-naphthyl)-alanyl-3-(5-imidazolyl)-alanyl-statyl]-lysinate and pharmaceutically acceptable salts thereof.

16. $N^{\epsilon}$-[N-Bis(1-naphthylmethyl)acetyl-leucyl-statyl]-lysine and pharmaceutically acceptable salts thereof.

17. $N^{\epsilon}$-[N-Bis(1-naphthylmethyl)acetyl-3-(4-thiazolyl)-alanyl-statyl]-lysinol and pharmaceutically acceptable salts thereof.

18. $N^{\epsilon}$-{4-[N-Nicotinoyl-3-(1-naphthyl)-alanyl-3-(4-thiazolyl)-alanylamino]-5-cyclohexyl-3-hydroxypentanoyl}-lysinamide and pharmaceutically acceptable salts thereof.

19. N -(m-Aminomethylbenzyl)-4-[N-nicotinoyl-3-(1-naphthyl)-alanyl-3-(4-thiazolyl)-alanylamino]-5-cyclohexyl-3-hydroxypentanamide and pharmaceutically acceptable salts thereof.

20. $N^{\alpha}$-[N-Nicotinoyl-3-(1-naphthyl)-alanyl-3-(5-imidazolyl)-N-methyl-alanyl-statyl]-lysinamide and pharmaceutically acceptable salts thereof.

21. $N^{\epsilon}$-[N -Nicotinoyl-3-(1-naphthyl)-alanyl-3-(4-thiazolyl)-N-methyl-alanyl-statyl]-lysinamide and pharmaceutically acceptable salts thereof.

22. $N^{\epsilon}$-{4-[N-Nicotinoyl-3-(1-naphthyl)-alanyl-3-(4-thiazolyl)-N-methyl-alanylamino]-5-cyclohexyl-3-hydroxypentanoyl}-lysinamide and pharmaceutically acceptable salts thereof.

23. $N^{\epsilon}$-[N-Bis(1-naphthylmethyl)acetyl-3-(5-imidazolyl)-alanyl-statyl-leucyl]-lysinol and pharmaceutically acceptable salts thereof.

24. A pharmaceutical composition for the treatment of angiotensin-induced hypertension comprising at least one renin inhibitor in admixture with a pharmaceutically acceptable carrier or diluent, wherein said renin inhibitor is at least one compound as claimed in any one of the preceding Claims.

25. A composition according to Claim 24, formulated for oral administration.

26. A composition according to Claim 24, formulated for parenteral administration.

27. The use for the manufacture of a medicament for the treatment of angiotensin-induced hypertension of a compound according to any one of Claims 1 to 23.

28. The use according to Claim 27, in which said medicament is formulated for oral administration.

29. The use according to Claim 27, in which said medicament is formulated for parenteral administration.

30. A process for preparing a compound as claimed in any one of Claims 1 to 23, which comprises reacting together two compounds, one having a terminal carboxy group or reactive derivative thereof and the other having a terminal amino group or reactive derivative thereof, under conditions conventional for peptide synthesis, said two compounds corresponding to the fragments derivable by cleavage of any one of the peptide bonds A, B, C and D:

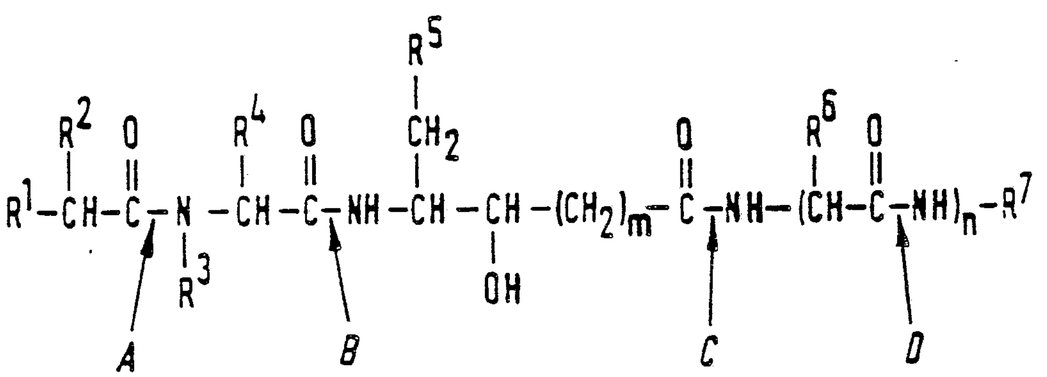

(I)

(in which $R^1$-$R^7$ m and n are as defined in Claim 1), and optionally subjecting the product to one or more of the reactions; acyl exchange, esterification, salification or conversion of any group represented by $R^4$ or $R^7$

to any other such group.

31. A process for preparing a compound as claimed in any one of Claims 1 to 23, which comprises reacting together compounds of formulae:

$R^1$-CH($R^2$)-COOH (II)
or a reactive derivative thereof,

HN($R^3$)-CH($R^4$)-COOH (III)
or a reactive derivative thereof,

$H_2N$-CH($CH_2R^5$)-CH(OH)-$(CH_2)_m$-COOH (IV)
or a reactive derivative thereof,

optionally $H_2N$-CH($R^6$)-COOH (V)
or a reactive derivative thereof, and

$H_2N$-$R^7$ (VI)
or a reactive derivative thereof

(in which $R^1$-$R^7$ and m are as defined in Claim 1) or reacting a peptide compound derivable by reaction of some of said compounds of formulae (II), (III), (IV), (V) or (VI) or said reactive derivatives with the remainder of said compounds or said reactive derivative(s) or with a peptide compound derivable by reaction of said remainder or reactive derivative(s) thereof, the reaction(s) being in an order corresponding to the order of the residues derived from said compounds of formulae (II), (III), (IV) and (VI) and optionally (V) in said compound of formula (I), and optionally subjecting the product to one or more of the reactions; acyl exchange, esterification, salification or conversion of any group represented by $R^4$ or $R^7$ to any other such group.

Claims for the following Contracting States : AT; ES:

1. Process for preparing compounds of formula (I):

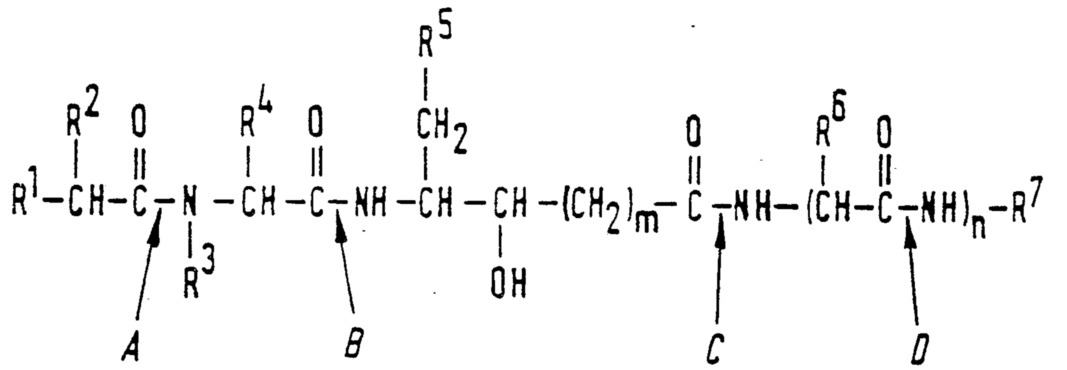
(I)

wherein:
m is the cypher 0 or the integer 1;
n is the cypher 0 or the integer 1;

$R^1$ represents a group of formula -A-$R^8$,
wherein: A represents a $C_1$-$C_8$ alkylene group; and $R^8$ represents an aryl group, a heterocyclic group, a $C_2$-$C_5$ aliphatic acyl group, a heterocyclic acyl group or an alkoxyalkoxy group in which each alkoxy part is $C_1$-$C_4$,

a group of formula -CONR$^9$R$^{10}$,
wherein $R^9$ and $R^{10}$ are the same or different and each represents a hydrogen atom, a $C_1$-$C_4$ alkyl group or a $C_1$-$C_4$ alkyl group having at least one of substituents (a), defined below,

or a group of formula -NHCOR$^{11}$,
wherein R$^{11}$ represents a $C_1$-$C_{10}$ alkyl group, a $C_1$-$C_{10}$ alkyl group having at least one of substituents (b), defined below, an aryl group, a heterocyclic group, a $C_1$-$C_4$ alkoxy group, a $C_1$-$C_4$ alkoxy group having at least one of substituents (c), defined below, an aralkyloxy group or an aryloxy group,
provided that, when both m = 1 and n = 1, R$^1$ represents said group of formula -A-R$^8$ or said group of formula -CONR$^9$R$^{10}$;

R$^2$ represents a group of formula -B-R$^{12}$,
wherein B represents a $C_1$-$C_{10}$ alkylene group and R$^{12}$ represents an aryl group or a heterocyclic group;

R$^3$ represents a hydrogen atom or a $C_1$-$C_4$ alkyl group;

R$^4$ represents a $C_1$-$C_{10}$ alkyl group, a $C_1$-$C_{10}$ alkyl group having at least one of substituents (d), defined below, a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

R$^5$ represents an isopropyl group, a $C_3$-$C_8$ cycloalkyl group or a phenyl group;

R$^6$ represents a $C_1$-$C_6$ alkyl group;

R$^7$ represents a $C_1$-$C_{10}$, alkyl group having at least one of substituents (e), defined below, or a $C_1$-$C_{10}$ alkyl group having at least one of substituents (e) and at least one of substituents (f), defined below;

said aryl groups and the aryl parts of said aryloxy, aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one of substituents (d) and/or (g) and/or (h), defined below;

the alkyl parts of said aralkyloxy, aralkylthio, aralkyloxycarbonylamino and aralkyloxycarbonyl groups have from 1 to 4 carbon atoms;

said heterocyclic groups have from 5 to 14 ring atoms, of which from 1 to 5 are nitrogen and/or oxygen and/or sulphur hetero-atoms, and are unsubstituted or have at least one of substituents (d) and/or (g) and/or (h) and/or (i), defined below;

substituents (a):carboxy groups, $C_2$-$C_5$ alkoxycarbonyl groups, carbamoyl groups, alkylcarbamoyl groups where the alkyl part is $C_1$-$C_4$ and dialkylcarbamoyl groups where each alkyl part is $C_1$-$C_4$;

substituents (b):$C_1$-$C_4$ alkoxy groups, alkoxyalkoxy groups in which each alkoxy part is $C_1$-$C_4$ alkoxy, halogen atoms, $C_2$-$C_5$ alkoxycarbonyl groups, aralkyloxycarbonyl groups, aryl groups, heterocyclic groups, $C_1$-$C_7$ aliphatic carboxylic acyl groups, aromatic carboxylic acyl groups, heterocyclic carboxylic acyl groups and aryloxy groups;

substituents (c):$C_1$-$C_4$ alkoxy groups and alkoxyalkoxy groups where each alkoxy part is $C_1$-$C_4$;

substituents (d):hydroxy groups, $C_1$-$C_4$ alkoxy groups, aryloxy groups, aralkyloxy groups where the alkyl part is $C_1$-$C_4$, $C_1$-$C_7$ aliphatic carboxylic acyloxy groups, $C_1$-$C_4$ alkylthio groups, arylthio groups, aralkylthio groups where the alkyl part is $C_1$-$C_4$, $C_1$-$C_4$ alkylsulphinyl groups, $C_1$-$C_4$ alkylsulphonyl groups, arylsulphinyl groups, arylsulphonyl groups, $C_1$-$C_7$ aliphatic carboxylic acylamino groups, aromatic carboxylic acylamino groups,

$C_2$-$C_7$ alkoxycarbonylamino groups, aralkyloxycarbonylamino groups, $C_2$-$C_7$ alkoxycarbonyl groups, aryloxycarbonyl groups, aralkyloxycarbonyl groups, $C_1$-$C_7$ aliphatic carboxylic acyl groups, aromatic carboxylic acyl groups, heterocyclic carboxylic acyl groups, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl-(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di-and tri-halomethyl groups, mercapto groups, amino groups, $C_1$-$C_4$ alkylamino groups, dialkylamino groups where each alkyl part is $C_1$-$C_4$, carboxy groups, guanidino groups and guanidino groups having at least one $C_1$-$C_4$ alkyl substituent;

substituents (e): amino groups, protected amino groups, aminoalkylamino groups where the alkyl part is $C_1$-$C_4$ and one or both amino parts may be unsubstituted or have a $C_1$-$C_4$ alkyl substituent, aminoalkylaminoalkylamino groups where each alkyl part is $C_1$-$C_4$ and any amino part may be unsubstituted or have a $C_1$-$C_4$ alkyl substituent, guanidino groups, guanidino groups having at least one $C_1$-$C_4$ alkyl substituent, phenyl groups substituted by at least one $C_1$-$C_4$ aminoalkyl substituent, phenyl groups substituted by at least one substituted $C_1$-$C_4$ hydroxyalkoxy group said hydroxyalkoxy group having at least one of substituents (j), defined below, and sulpho groups;

substituents (f):hydroxy groups, carboxy groups, $C_2$-$C_5$ alkoxycarbonyl groups, carbamoyl groups, alkylcarbamoyl group where the alkyl part is $C_1$-$C_4$ and dialkylcarbamoyl groups where each alkyl part is $C_1$-$C_4$;

substituents (g): hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

substituents (h): $C_1$-$C_4$ alkyl groups, nitro groups, cyano groups, $C_3$-$C_5$ alkenoyl groups, $C_3$-$C_5$ alkenoyl groups having at least one of substituents (d) and $C_1$-$C_4$ alkyl groups having at least one of substituents (g);

substituents (i):oxygen atoms; and

substituents (j):amino groups, $C_1$-$C_4$ alkylamino groups, dialkylamino groups where each alkyl part is $C_1$-$C_4$, heterocyclic groups and heterocyclic-substituted $C_1$-$C_4$ alkylamino groups;
and pharmaceutically acceptable salts thereof, which comprises reacting together two compounds, one having a terminal carboxy group or reactive derivative thereof and the other having a terminal amino group or reactive derivative thereof, under conditions conventional for peptide synthesis, said two compounds corresponding to the fragments derivable by cleavage of any one of the peptide bonds A, B, C and D, and optionally subjecting the product to one or more of the reactions: acyl exchange, esterification, salification or conversion of any group represented by $R^4$ and $R^7$ to any other group within the respective definitions.

2. A process as claimed in Claim 1, which comprises reacting together compounds of formulae:

$R^1$-CH($R^2$)-COOH (II)
or a reactive derivative thereof,

HN($R^3$)-CH($R^4$)-COOH (III)

or a reactive derivative thereof,

$H_2N\text{-}CH(CH_2R^5)\text{-}CH(OH)\text{-}(CH_2)_m\text{-}COOH$ (IV)
or a reactive derivative thereof,

optionally $H_2N\text{-}CH(R^6)\text{-}COOH$ (V)
or a reactive derivative thereof, and

$H_2N\text{-}R^7$ (VI)
or a reactive derivative thereof

(in which $R^1$, $R^2$, $R^3$, $R^4$, $R^5$, $R^6$, $R^7$ and $m$ are as defined in Claim 1) or reacting a peptide compound derivable by reaction of some of said compounds of formulae (II), (III), (IV), (V) or (VI) or said reactive derivatives with the remainder of said compounds or said reactive derivative(s) or with a peptide compound derivable by reaction of said remainder or reactive derivative(s) thereof, the reaction(s) being in an order corresponding to the order of the residues derived from said compounds of formulae (II), (III), (IV) and (VI) and optionally (V) in said compound of formula (I), and optionally subjecting the product to one or more of the reactions: acyl exchange, esterification, salification or conversion of any group represented by $R^4$ and $R^7$ to any other group within the respective definitions.

3. A process according to Claim 2, which comprises reacting a carboxylic acid compound of formula - (VII):

$R^{1b}\text{-}CH(R^{2a})\text{-}CO\text{-}NR^3\text{-}CH(R^{4a})\text{-}COOH$ (VII)

(in which $R^3$ is as defined in Claim 1 and $R^{1b}$, $R^{2a}$ and $R^{4a}$ represent any group defined in Claim 1 for $R^1$, $R^2$ and $R^4$, respectively, but in which any reactive group is optionally protected) or a reactive derivative thereof with an amino compound of formula (VIII):

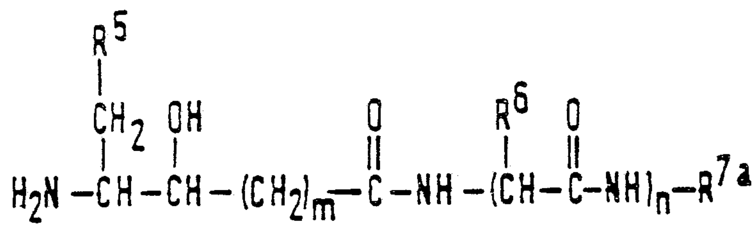

(VIII)

(in which $R^5$, $R^6$, $m$ and $n$ are as defined in Claim 1 and $R^{7a}$ represents any group defined for $R^7$ in Claim 1, but in which any reactive group is optionally protected) or with a reactive derivative thereof.

4. A process according to Claim 2, which comprises reacting a carboxylic acid compound of formula - (IX):

$R^{1b}\text{-}CH(R^{2a})\text{-}COOH$ (IX)

(in which $R^{1b}$ and $R^{2a}$ are as defined in Claim 3) or a reactive derivative thereof is reacted with an amino compound of formula (X):

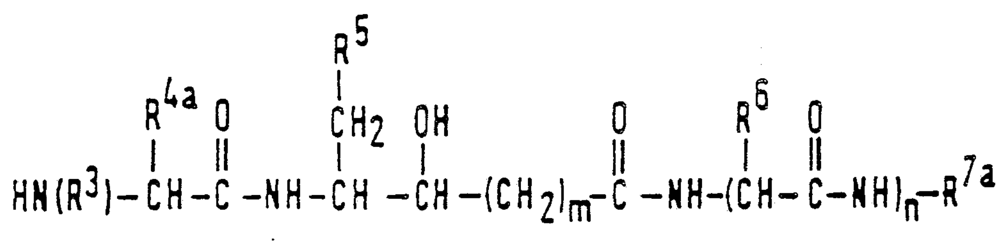

(X)

(in which $R^3$, $R^{4a}$, $R^5$, $R^6$, $R^{7a}$, $m$ and $n$ are as defined in Claims 1 and 3) or with a reactive derivative thereof.

5. A process according to Claim 2, which comprises the steps:

(a) reacting a compound of formula (IX) (defined in Claim 4) or a reactive derivative thereof with an amino compound of formula (XI):

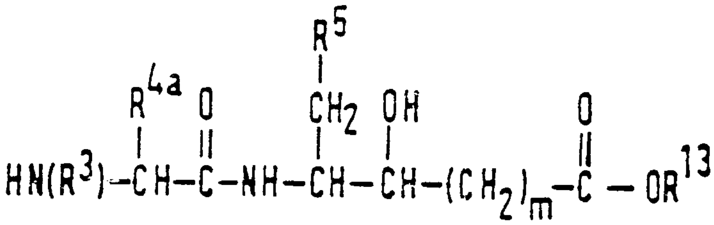

(XI)

(in which $R^3$, $R^{4a}$, $R^5$ and m are as defined in Claims 1 and 3 and $R^{13}$ represents a lower alkyl group or an aralkyl group) or with a reactive derivative thereof, to give a compound of formula (XII):

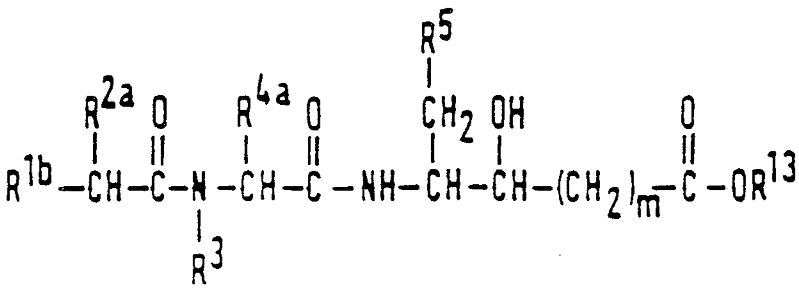

(XII)

(in which $R^{1b}$, $R^{2a}$ $R^3$, $R^{4a}$, $R^5$, $R^{13}$ and m are as defined in Claims 1 and 3);

(b) if necessary, removing the alkyl or aralkyl group $R^{13}$ to convert the compound of formula (XII) to the free acid and/or converting said compound of formula (XII) or said free acid to a reactive derivative thereof; and

(c) reacting the product of step (a) or (b) with an amino compound of formula (XIII):

$H_2N$-[CH($R^6$)-CO-NH]$_n$-$R^{7a}$ (XIII)

(in which $R^6$, $R^{7a}$ and n are as defined in Claims 1 and 3) or with a reactive derivative thereof, to give a compound of formula (Ia):

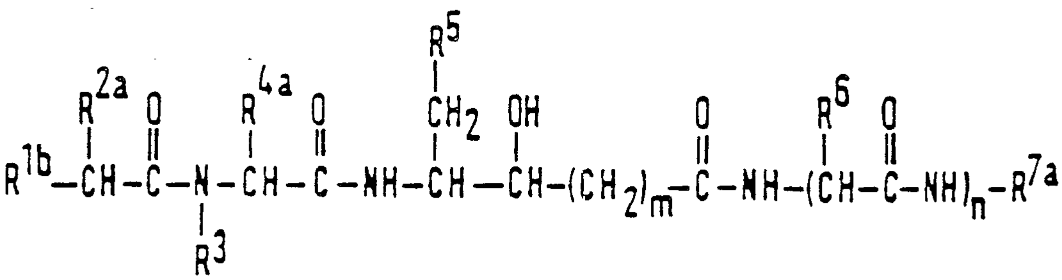

(Ia)

(in which $R^{1b}$, $R^{2a}$, $R^3$, $R^{4a}$, $R^5$, $R^6$, $R^{7a}$, m and n are as defined in Claims 1 and 3).

6. A process as claimed in any one of Claims 1 to 5, wherein:

m is the cypher 0 or the integer 1;

n is the cypher 0 or the integer 1;

$R^1$ represents a group of formula -A-$R^8$
wherein A represents a $C_1$-$C_4$ alkylene group and $R^8$ represents an aryl group, an aromatic heterocyclic group, a $C_2$-$C_5$ aliphatic carboxylic acyl group or a heterocyclic carboxylic acyl group,

an alkylcarbamoyl group in which the alkyl part is $C_1$-$C_4$ alkyl, a dialkylcarbamoyl group in which each alkyl part is $C_1$-$C_4$ alkyl, a substituted alkylcarbamoyl group in which the alkyl part is $C_1$-$C_4$ alkyl and has at least one carboxy substituent, a substituted dialkylcarbamoyl group in which each alkyl part is $C_1$-$C_4$ alkyl and in which at least one of said alkyl parts has at least one carboxy substituent, or a group of formula -NHCOR$^{11}$ wherein $R^{11}$ represents a $C_1$-$C_4$ alkyl group, a $C_7$-$C_9$ aralkyl group, a $C_1$-$C_4$ alkyl group having a heterocyclic substituent, an aryl group, a heterocyclic group, a $C_1$-$C_4$ alkoxy group, an alkoxyalkoxyalkoxy group in which each alkoxy part is $C_1$-$C_4$ or a $C_7$-$C_9$ aralkyloxy group,
provided that, when both $\underline{m}=1$ and $\underline{n}=1$, $R^1$ represents said group of formula -A-$R^8$ or said alkylcarbamoyl, dialkylcarbamoyl, substituted alkylcarbamoyl or substituted dialkylcarbamoyl group;

$R^2$ represents a group of formula -B-$R^{12}$, in which B represents a $C^1$-$C_4$ alkylene group and $R^{12}$ represents an aryl group or an aromatic heterocyclic group;

$R^3$ represents a hydrogen atom or a methyl group;

$R^4$ represents a hydrogen atom, a $C_1$-$C_6$ alkyl group, a $C_1$-$C_6$ alkyl group having at least one of substituents - (k), a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^6$ represents a $C_1$-$C_6$ alkyl group;

$R^7$ represents a substituted $C_2$-$C_6$ alkyl group having at least one of substituents (m) or a substituted $C_2$-$C_6$ alkyl group having at least one of substituents (m) and at least one of substituents (f), defined in Claim 1;

said heterocyclic groups have 5 or 6 ring atoms, of which from 1 to 3 are nitrogen and/or oxygen and/or sulphur hetero-atoms, and are unsubstituted or have at least one of substituents (d) and/or (g) and/or (h) and/or (i), defined in Claim 1;

<u>substituents (k):</u> hydroxy groups, $C_1$-$C_4$ alkoxy groups, $C_1$-$C_4$ alkylthio groups, $C_1$-$C_7$ aliphatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, $C_3$-$C_6$ cycloalkyl groups, aryl groups, aromatic heterocyclic groups, mercapto groups, amino groups, alkylamino groups in which the alkyl part is $C_1$-$C_4$ alkyl, dialkylamino groups in which each alkyl part is $C_1$-$C_4$ alkyl, carboxy groups, guanidino groups, alkylguanidino groups in which the alkyl part is $C_1$-$C_4$ and dialkylguanidino groups in which each alkyl part is $C_1$-$C_4$;

<u>substituents (m):</u> amino groups, protected amino groups, $C_1$-$C_4$ aminoalkylamino groups, aminoalkylaminoalkylamino groups in which each alkyl part is $C_1$-$C_4$, guanidino groups, alkylguanidino groups in which the alkyl part is $C_1$-$C_4$, dialkylguanidino groups in which each alkyl part is $C_1$-$C_4$, substituted phenyl groups having at least one of substituents (n) and sulpho groups; and

substituents (n):$C_1$-$C_4$ aminoalkyl groups and di-substituted $C_1$-$C_4$ alkoxy groups, in which one substituent is the hydroxy group and the other is selected from amino groups, $C_1$-$C_4$ alkylamino groups, dialkylamino groups in which each alkyl part is $C_1$-$C_4$ and heterocyclic groups.

7. A process as claimed in any one of Claims 1 to 5, wherein:

m is 1;

n is 0;

$R^1$ represents a group of formula $-A-R^8$ in which A represents a $C_1$ or $C_2$ alkylene group and $R^8$ represents a phenyl group, a naphthyl group or a nitrogen-containing aromatic heterocyclic group, an alkylcarbamoyl group in which the alkyl part is $C_1$-$C_4$ alkyl, a dialkylcarbamoyl group in which each alkyl part is $C_1$-$C_4$ alkyl, a carboxyalkylcarbamoyl group in which the alkyl part is $C_1$-$C_4$ alkyl, or a group of formula $-NHCOR^{11}$ in which $R^{11}$ represents a $C_1$-$C_4$ alkyl group, a $C_1$-$C_4$ alkyl group having a heterocyclic substituent, a phenyl group, a phenyl group having at least one of substituents (d) and/or (g) and/or (h) defined in Claim 1, a nitrogen-containing heterocyclic group or a $C_7$-$C_9$ aralkyloxy group;

$R^2$ represents a group of formula $-B-R^{12}$ in which B represents a $C_1$ or $C_2$ alkylene group, and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ alkyl and the phenyl group is unsubstituted or has at least one of substituents (d) and/or (g) and/or (h) defined in Claim 1, a $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a $C_5$ or $C_6$ cycloalkyl group or a phenyl group;

$R^7$ represents a substituted $C_2$-$C_6$ alkyl group having at least one of substituents (o) or a substituted $C_2$-$C_6$ alkyl group having at least one of substituents (o) and at least one of substituents (p);

said heterocyclic groups have 5 or 6 ring atoms of which 1 or 2 are nitrogen and/or oxygen and/or sulphur hetero-atoms, and are unsubstituted or have at least one of substituents (d) and/or (g) and/or (h) and/or (i), defined in Claim 1;

substituents (o): amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

8. A process as claimed in any one of Claims 1 to 5, wherein:

m is 1;

n is 1;

$R^1$ represents a group of formula $-A-R^8$ in which A represents a $C_1$ or $C_2$ alkylene group and $R^8$ represents a phenyl group, a naphthyl group or a nitrogen-containing aromatic heterocyclic group, an alkylcarbamoyl group in which the alkyl part is $C_1$-$C_4$, a dialkylcarbamoyl group in which each alkyl part is $C_1$-$C_4$ or a carboxyalkylcarbamoyl group in which the alkyl part is $C_1$-$C_4$;

$R^2$ represents a group of formula $-B-R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h) defined in Claim 1, a $C_1$-$C_6$ alkyl group having an aromatic heterocyclic substituent, a $C_3$ or $C_4$ alkenyl group or a $C_3$-$C_4$ alkynyl group;

$R^5$ represents an isopropyl group, a cyclopentyl group, a cyclohexyl group or a phenyl group;

$R^6$ represents a $C_3$ or $C_4$ alkyl group;

$R^7$ represents a substituted $C_2$-$C_6$ alkyl group having at least one of substituents (o) or a substituted $C_2$-$C_6$ alkyl group having at least one of substituents (o) and at least one of substituents (p);

said heterocyclic groups have 5 or 6 ring atoms, of which 1 or 2 are nitrogen and/or oxygen and/or sulphur hetero-atoms, said heterocyclic group being unsubstituted or having at least one of substituents (d) and/or - (g) and/or (h) defined in Claim 1;

substituents (o):amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

9. A process as claimed in any one of Claims 1 to 5, wherein:

m and n are both 0;

$R^1$ represents a group of formula $-A-R^8$,
in which A represents a $C_1$ or $C_2$ alkylene group, and $R^8$ represents a phenyl group, a naphthyl group or a nitrogen-containing aromatic heterocyclic group,
an alkylcarbamoyl group in which the alkyl part is $C_1$-$C_4$, a dialkylcarbamoyl group in which each alkyl part is $C_1$-$C_4$, a carboxyalkylcarbamoyl group in which the alkyl part is $C_1$-$C_4$ or a group of formula $-NHCOR^{11}$,
in which $R^{11}$ represents a $C_1$-$C_4$ alkyl group, a $C_1$-$C_4$ alkyl group having a heterocyclic substituent, a phenyl group, a phenyl group having at least one of substituents (d) and/or (g) and/or (h) defined in Claim 1, a nitrogen-containing heterocyclic group or a $C_7$-$C_9$ aralkyloxy group;

$R^2$ represents a group of formula $-B-R^{12}$ in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_1$-$C_6$ alkyl group, a phenylalkyl group in which the alkyl part is $C_1$-$C_6$ and the phenyl part is unsubstituted or has at least one of substituents (d) and/or (g) and/or (h) defined in Claim 1, a $C_1$-$C_6$ alkyl group having an aromatic heterocyclic substituent, a $C_3$ or $C_4$ alkenyl group or a $C_3$ or $C_4$ alkynyl group;

$R^5$ represents an isopropyl group, a cyclopentyl group, a cyclohexyl group or a phenyl group;

$R^7$ represents a substituted $C_2$-$C_6$ alkyl group having at least one of substituents (o) or a substituted $C_2$-$C_6$ alkyl group having at least one of substituents (o) and at least one of substituents (p);

said heterocyclic groups and said heterocyclic substituents have 5 or 6 ring atoms of which 1 or 2 are nitrogen and/or oxygen and/or sulphur hetero-atoms, and are unsubstituted or have at least one of

substituents (d) and/or (g) and/or (h), defined in Claim 1;

substituents (o):amino groups, protected amino groups, guanidino groups and phenyl groups having a $C_1$ or $C_2$ aminoalkyl substituent; and

substituents (p):hydroxy groups, carboxy groups, $C_2$-$C_5$ alkoxycarbonyl groups and carbamoyl groups.

10. A process as claimed in any one of Claims 1 to 5, wherein:

m is 1;

n is 0;

$R^1$ represents a group of formula -A-$R^8$
in which A represents a $C_1$ or $C_2$ alkylene group and $R^8$ represents a phenyl group, a naphthyl group or a nitrogen-containing aromatic heterocyclic group,
an alkylcarbamoyl group in which the alkyl part is $C_1$-$C_4$ alkyl, a dialkylcarbamoyl group in which each alkyl part is $C_1$-$C_4$ alkyl or a group of formula -NHCOR$^{11}$,
in which $R^{11}$ represents a $C_1$-$C_4$ alkyl group, a $C_1$-$C_4$ alkyl group having a heterocyclic substituent or a nitrogen-containing heterocyclic group;

$R^2$ represents a group of formula -B-$R^{12}$, in which B represents a $C_1$ or $C_2$ alkylene group and $R^{12}$ represents a phenyl group, a naphthyl group or a nitrogen-containing aromatic heterocyclic group;

$R^3$ represents a hydrogen atom or a methyl group;

$R^4$ represents a $C_3$ or $C_4$ alkyl group, a phenylalkyl group in which the alkyl part is $C_1$ or $C_2$ alkyl and the phenyl part is unsubstituted or has at least one of substituents (d) and/or (g) and/or (h) defined in Claim 1, a $C_1$ or $C_2$ alkyl group having an aromatic heterocyclic substituent, a $C_3$ or $C_4$ alkenyl group or a $C_3$ or $C_4$ alkynyl group;

$R^5$ represents an isopropyl group or a cyclohexyl group;

$R^7$ represents a substituted $C_2$-$C_6$ alkyl group having at least one substituent selected from amino groups and protected amino groups or a substituted $C_2$-$C_6$ alkyl group having at least one substituent selected from amino groups and protected amino groups and at least one of substituents (p); and

substituents (p):hydroxy groups, carboxy groups, $C_2$-$C_5$ alkoxycarbonyl groups and carbamoyl groups.

11. A process as claimed in any one of Claims 1 to 5, wherein:

m is 1;

n is the cypher 0 or the integer 1;

$R^1$ represents a group of formula -A-$R^8$,
wherein A represents a $C_1$-$C_4$ alkylene group and $R^8$ represents an aryl group or an aromatic heterocyclic group having 5 or 6 ring atoms,
or a group of formula -NHCOR$^{11}$,
wherein $R^{11}$ represents a $C_1$-$C_4$ alkyl group a $C_7$-$C_9$ aralkyl group, a $C_1$-$C_4$ alkyl group having a heterocyclic substituent wherein the heterocyclic substituent has 5 or 6 ring atoms, an aryl group, an aromatic heterocyclic group having from 5 to 10 ring atoms, a $C_1$-$C_4$ alkoxy group or a $C_7$-$C_9$ aralkyloxy group.
provided that, when both m = 1 and n = 1, $R^1$ represents said group of formula -A-$R^8$;

$R^2$ represents a group of formula in -B-$R^{12}$, in which B represents a $C_1$-$C_4$ alkylene group, and $R^{12}$ represents an aryl group or an aromatic heterocyclic group having 5 or 6 ring atoms;

$R^3$ represents a hydrogen atom;

$R^4$ represents a hydrogen atom, a $C_1$-$C_6$ alkyl group, a $C_1$-$C_6$ alkyl group having at least one of substituents -(q), a $C_2$-$C_5$ alkenyl group, a $C_2$-$C_5$ alkenyl group having at least one halogen substituent or a $C_2$-$C_5$ alkynyl group;

$R^5$ represents an isopropyl group, a cyclopentyl group, a cyclohexyl group or a phenyl group;

$R^6$ represents a $C_1$-$C_5$ alkyl group;

$R^7$ represents a substituted $C_2$-$C_6$ alkyl group having at least one of substituents (r) or a substituted $C_2$-$C_6$ alkyl group having at least one of substituents (r) and at least one of substituents (f), defined in Claim 1;

said heterocyclic groups and heterocyclic substituents have from 1 to 3 ring nitrogen and/or oxygen and/or sulphur hetero-atoms and are unsubstituted or have at least one of substituents (d) and/or (g) and/or (h) and/or (i), defined in Claim 1;

substituents (q): hydroxy groups, $C_1$-$C_4$ alkoxy groups, $C_7$-$C_9$ aralkyloxy groups, $C_1$-$C_7$ aliphatic carboxylic acyloxy groups, $C_1$-$C_4$ alkylthio groups, $C_7$-$C_9$ aralkylthio groups, $C_1$-$C_4$ alkylsulphinyl groups, $C_1$-$C_4$ alkylsulphonyl groups, $C_1$-$C_7$ aliphatic carboxylic acylamino groups, aromatic carboxylic acylamino groups, $C_2$-$C_7$ alkoxycarbonylamino groups, $C_8$-$C_{10}$ aralkyloxycarbonylamino groups, $C_2$-$C_7$ alkoxycarbonyl groups, aryloxycarbonyl groups, $C_8$-$C_{10}$ aralkyloxycarbonyl groups, carbamoyl groups, alkylcarbamoyl groups in which the alkyl part is $C_1$-$C_4$ alkyl, dialkylcarbamoyl groups in which each alkyl part is $C_1$-$C_4$ alkyl, thiocarbamoyl groups, alkyl(thiocarbamoyl) groups in which the alkyl part is $C_1$-$C_4$ alkyl, dialkyl(thiocarbamoyl) groups in which each alkyl part is $C_1$-$C_4$, ureido groups, thioureido groups, $C_3$-$C_8$ cycloalkyl groups, $C_5$-$C_8$ cycloalkenyl groups, aryl groups, aromatic heterocyclic groups, halogen atoms, halomethyl groups, mercapto groups, amino groups, $C_1$-$C_4$ alkylamino groups, dialkylamino groups in which each alkyl part is $C_1$-$C_4$ alkyl, carboxy groups and guanidino groups;

substituents (r):amino groups, protected amino groups, $C_1$-$C_4$ aminoalkylamino groups, aminoalkylaminoalkylamino groups in which each alkyl part is $C_1$-$C_4$, guanidino groups, substituted phenyl groups having at least one of substituents (n), and sulpho groups; and

substituents (n):$C_1$-$C_4$ aminoalkyl groups and di-substituted $C_1$-$C_4$ alkoxy groups, in which one substituent is the hydroxy group and the other is selected from amino groups, $C_1$-$C_4$ alkylamino groups, dialkylamino groups in which each alkyl part is $C_1$-$C_4$ and heterocyclic groups.

12. A process according to any one of Claims 1 to 5, wherein the reagents and reaction conditions are so selected as to prepare:

$N^{\alpha}$-[N-nicotinoyl-3-(1-naphthyl)-alanyl-leucyl-statyl]-lysinamide

$N^{\epsilon}$-[N-nicotinoyl-3-(1-naphthyl)-alanyl-leucyl-statyl]-lysinol

N -(m-aminomethylbenzyl)-[N-nicotinoyl-3-(1-naphthyl)-alanyl-leucyl-statin]amide

$N^{\epsilon}$-[N-nicotinoyl-3-(1-naphthyl)-alanyl-3-(4-thiazolyl)-alanyl-statyl]-lysinol

$N^{\epsilon}$-[N-picolinyl-3-(1-naphthyl)-alanyl-3-(5-imidazolyl)-alanyl-statyl]-lysinol

$N^{\epsilon}$-[N-picolinyl-3-(1-naphthyl)-alanyl-leucyl-statyl]-lysinol

$N^{\alpha}$-[N-bis(1-naphthylmethyl)acetyl-leucyl-statyl]-lysinol

$N^{\epsilon}$-[N-benzyloxycarbonyl-3-(1-naphthyl)-alanyl-3-(5-imidazolyl)-alanyl-statyl]-lysinate

$N^{\epsilon}$-[N-bis(1-naphthylmethyl)acetyl-leucyl-statyl]-lysine

$N^{\epsilon}$-[N-bis(1-naphthylmethyl)acetyl-3-(4-thiazolyl)-alanyl-statyl]-lysinol

$N^{\epsilon}${4-[N-nicotinoyl-3-(1-naphthyl)-alanyl-3-(4-thiazolyl)-alanylamino]-5-cyclohexyl-3-hydroxypentanoyl}-lysinamide

N-(m-aminomethylbenzyl)-4-[N-nicotinoyl-3-(1-naphthyl)-alanyl-3-(4-thiazolyl)-alanylamino]-5-cyclohexyl-3-hydroxypentanamide

$N^{\alpha}$-[N-nicotinoyl-3-(1-naphthyl)-alanyl-3-(5-imidazolyl)-N-methyl-alanyl-statyl]-lysinamide

$N^{\epsilon}$-[N-nicotinoyl-3-(1-naphthyl)-alanyl-3-(4-thiazolyl)-N-methyl-alanyl-statyl]-lysinamide

$N^{\epsilon}$-{4-[N-nicotinoyl-3-(1-naphthyl)-alanyl-3-(4-thiazolyl)-N-methyl-alanylamino]-5-cyclohexyl-3-hydroxy pentanoyl}-lysinamide

$N^{\epsilon}$-[N-bis(1-naphthylmethyl)acetyl-3-(5-imidazolyl)-alanyl-statyl-leucyl]-lysinol

or a pharmaceutically acceptable salt thereof.

13. The use for the manufacture of a medicament for the treatment of angiotensin induced hypertension of a compound of formula (I):

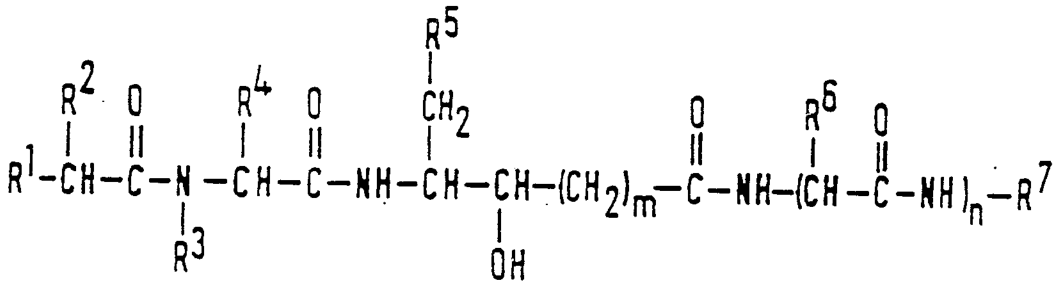 (I)

(in which $R^1$-$R^7$, m and n, are as defined in any one of Claims 1 and 6 to 11) or a pharmaceutically acceptable salt thereof.

14. The use according to Claim 13, in which said compound is:

$N^{\alpha}$-[N-nicotinoyl-3-(1-naphthyl)-alanyl-leucyl-statyl]-lysinamide

$N^{\epsilon}$-[N-nicotinoyl-3-(1-naphthyl)-alanyl-leucyl-statyl]-lysinol

N-(m-aminomethylbenzyl)-[N-nicotinoyl-3-(1-naphthyl)-alanyl-leucyl-statin]amide

$N^{\epsilon}$-[N-nicotinoyl-3-(1-naphthyl)-alanyl-3-(4-thiazolyl)-alanyl-statyl]-lysinol

$N^{\epsilon}$-[N-picolinyl-3-(1-naphthyl)-alanyl-3-(5-imidazolyl)-alanyl-statyl]-lysinol

$N^{\epsilon}$-[N-picolinyl-3-(1-naphthyl)-alanyl-leucyl-statyl]-lysinol

$N^{\alpha}$-[N-bis(1-naphthylmethyl)acetyl-leucyl-statyl]-lysinol

$N^{\epsilon}$-[N-benzyloxycarbonyl-3-(1-naphthyl)-alanyl-3-(5-imidazolyl)-alanyl-statyl]-lysinate

$N^{\epsilon}$-[N-bis(1-naphthylmethyl)acetyl-leucyl-statyl]-lysine

$N^{\epsilon}$-[N-bis(1-naphthylmethyl)acetyl-3-(4-thiazolyl)-alanyl-statyl]-lysinol

$N^{\epsilon}$-{4-[N-nicotinoyl-3-(1-naphthyl)-alanyl-3-(4-thiazolyl)-alanylamino]-5-cyclohexyl-3-hydroxy pentanoyl}-lysinamide

N-(m -aminomethylbenzyl)-4-[N-nicotinoyl-3-(1-naphthyl)-alanyl-3-(4-thiazolyl)-alanylamino]-5-cyclohexyl-3-hydroxypentanamide

$N^{\alpha}$-[N-nicotinoyl-3-(1-naphthyl)-alanyl-3-(5-imidazolyl)-N-methyl-alanyl-statyl]-lysinamide

$N^{\epsilon}$-[N-nicotinoyl-3-(1-naphthyl)-alanyl-3-(4-thiazolyl)-N-methyl-alanyl-statyl]-lysinamide

$N^{\epsilon}$-{4-[N-nicotinoyl-3-(1-naphthyl)-alanyl-3-(4-thiazolyl)-N-methyl-alanylamino]-5-cyclohexyl-3-hydroxypentanoyl}-lysinamide

$N^{\epsilon}$-[N-bis(1-naphthylmethyl)acetyl-3-(5-imidazolyl)-alanyl-statyl-leucyl]-lysinol

or a pharmaceutically acceptable salt thereof.

15. The use according to Claim 13 or Claim 14, in which said medicament is formulated for oral administration.

16. The use according to Claim 13 or Claim 14, in which said medicament is formulated for parenteral administration.